(19) Europäisches Patentamt
European Patent Office
Office européen des brevets

(11) **EP 1 265 990 B1**

(12) # EUROPEAN PATENT SPECIFICATION

(45) Date of publication and mention of the grant of the patent:
**08.03.2006 Bulletin 2006/10**

(21) Application number: **01920387.6**

(22) Date of filing: **15.03.2001**

(51) Int Cl.:
*C12N 9/02* (2006.01) *C07K 14/435* (2006.01)
*C12N 15/12* (2006.01) *C12N 15/10* (2006.01)
*C12N 15/11* (2006.01) *C12N 15/66* (2006.01)
*A61K 49/00* (2006.01) *A01H 5/00* (2006.01)
*A01K 67/027* (2006.01) *A61K 38/17* (2006.01)
*F41C 3/00* (2006.01) *F21S 10/00* (2006.01)
*A23L 1/00* (2006.01) *C12G 1/00* (2006.01)
*C07K 16/18* (2006.01) *C12N 5/10* (2006.01)

(86) International application number:
**PCT/US2001/008277**

(87) International publication number:
**WO 2001/068824 (20.09.2001 Gazette 2001/38)**

(54) **RENILLA RENIFORMIS FLUORESCENT PROTEINS, NUCLEIC ACIDS ENCODING THE FLUORESCENT PROTEINS AND THE USE THEREOF IN DIAGNOSTICS, HIGH THROUGHPUT SCREENING AND NOVELTY ITEMS**

LUCIFERASE, FLUORESZENZPROTEINE, FÜR LUCIFERASE UND FLUORESZENZPROTEINE KODIEREND NUKLEINSAÜRE UND IHRE VERWENDUNG IN DER DIAGNOSE, HOCH-DURCHSATZ SCREENINGSVERFAHREN

PROTEINES FLUORESCENTES ISSUES DE i RENILLA RENIFORMIS /i , ACIDES NUCLEIQUES CODANT CES PROTEINES FLUORESCENTES ET LEUR UTILISATION DANS LE DIAGNOSTIC, LE CRIBLAGE A HAUT RENDEMENT ET AVEC DE NOUVEAUX ARTICLES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.03.2000 US 189691 P**

(43) Date of publication of application:
**18.12.2002 Bulletin 2002/51**

(73) Proprietors:
- **Prolume, Ltd.**
  **Pittsburgh, PA 15236 (US)**
- **Bryan, Bruce J.**
  **Beverly Hills, CA 90210 (US)**

(72) Inventors:
- **BRYAN, Bruce, J.**
  **Beverly Hills, CA 90210 (US)**
- **SZENT-GYORGYI, Christopher**
  **Pittsburgh, PA 15237 (US)**
- **SZCZEPANIAK, William**
  **Pittsburgh, PA 15206 (US)**

(74) Representative: **Baldock, Sharon Claire et al**
**BOULT WADE TENNANT,**
**Verulam Gardens**
**70 Gray's Inn Road**
**London WC1X 8BT (GB)**

(56) References cited:
**WO-A-01/32688** **WO-A-92/15673**
**WO-A-98/14605** **WO-A-98/26277**
**WO-A-99/49019** **US-A- 5 876 995**

- **WARD W W ET AL: "AN ENERGY TRANSFER PROTEIN IN COELENTERATE BIO LUMINESCENCE CHARACTERIZATION OF THE RENILLA-RENIFORMIS GREEN FLUORESCENT PROTEIN" JOURNAL OF BIOLOGICAL CHEMISTRY, vol. 254, no. 3, 1979, pages 781-788, XP002935261 EN ISSN: 0021-9258**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- FRATAMICO P M ET AL: "Construction and characterization of Escherichia coli O157:H7 strains expressing firefly luciferase and green fluorescent protein and their use in survival studies" JOURNAL OF FOOD PROTECTION, DES MOINES, IO, US, vol. 60, no. 10, 1997, pages 1167-1173, XP002118033 ISSN: 0362-028X
- XU Y ET AL: "A bioluminiscence resonance energy transfer (BRET) system: application to interacting circadian clock proteins" PROCEEDINGS OF THE NATIONAL ACADEMY OF SCIENCES OF USA, NATIONAL ACADEMY OF SCIENCE. WASHINGTON, US, vol. 96, no. 1, 5 January 1999 (1999-01-05), pages 151-156, XP002118035 ISSN: 0027-8424
- HEIM R ET AL: "ENGINEERING GREEN FLUORESCENT PROTEIN FOR IMPROVED BRIGHTNESS, LONGER WAVELENGTHS AND FLUROESCENCE RESONANCE ENERGY TRANSFER" CURRENT BIOLOGY, CURRENT SCIENCE,, GB, vol. 6, no. 2, 1 February 1996 (1996-02-01), pages 178-182, XP000676582 ISSN: 0960-9822
- EHRIG T ET AL: "GREEN-FLUORESCENT PROTEIN MUTANTS WITH ALTERED FLUORENCE EXCITATIONSPECTRA" FEBS LETTERS, ELSEVIER SCIENCE PUBLISHERS, AMSTERDAM, NL, vol. 367, 1995, pages 163-166, XP000579119 ISSN: 0014-5793

## Description

### RELATED APPLICATIONS

Benefit of priority is claimed to U.S. provisional application Serial No. 60/189,691, filed March 15, 2000, to Bryan and Prolume, LTD, entitled "RENILLA RENIFORMIS FLUORESCENT PROTEINS, NUCLEIC ACIDS ENCODING THE FLUORESCENT PROTEINS AND THE USE THEREOF IN DIAGNOSTICS, HIGH THROUGHPUT SCREENING AND NOVELTY ITEMS" is claimed.

This application is related to allowed U.S. application Serial No. 09/277,716, filed March 26, 1999, to Bruce Bryan and Christopher Szent-Gyorgyi, entitled "LUCIFERASES, FLUORESCENT PROTEINS, NUCLEIC ACIDS ENCODING THE LUCIFERASES AND FLUORESCENT PROTEINS AND THE USE THEREOF IN DIAGNOSTICS, HIGH THROUGHPUT SCREENING AND NOVELTY ITEMS." This application is related to International PCT application No. WO 99/49019 to Bruce Bryan and Prolume, LTD., entitled "LUCIFERASES, FLUORESCENT PROTEINS, NUCLEIC ACIDS ENCODING THE LUCIFERASES AND FLUORESCENT PROTEINS AND THE USE THEREOF IN DIAGNOSTICS, HIGH THROUGHPUT SCREENING AND NOVELTY ITEMS."

This application is also related to subject matter in U.S. application Serial No. 08/757,046, filed November 25, 1996, to Bruce Bryan entitled "BIOLUMINESCENT NOVELTY ITEMS", now U.S. Patent No. 5,876,995, issued March 2, 1999, and in U.S. application Serial No. 08/597,274, filed February 6, 1996, to Bruce Bryan, entitled "BIOLUMINESCENT NOVELTY ITEMS". This application is also related to U.S. application Serial No. 08/908,909, filed August 8, 1997, to Bruce Bryan entitled "DETECTION AND VISUALIZATION OF NEOPLASTIC TISSUE AND OTHER TISSUES". The application is also related to U.S. application Serial No. 08/990,103, filed December 12, 1997, to Bruce Bryan entitled "APPARATUS AND METHODS FOR DETECTING AND IDENTIFYING INFECTIOUS AGENTS".

Where permitted, the subject matter of each of the above noted U.S. applications and patents herein incorporated by reference in its entirety.

### FIELD OF INVENTION

Provided herein are isolated and purified nucleic acids and encoded fluorescent proteins from *Renilla reniformis* and uses thereof.

### BACKGROUND OF THE INVENTION

Luminescence is a phenomenon in which energy is specifically channeled to a molecule to produce an excited state. Return to a lower energy state is accompanied by release of a photon (hy). Luminescence includes fluorescence, phosphorescence, chemiluminescence and bioluminescence. Bioluminescence is the process by which living organisms emit light that is visible to other organisms. Luminescence may be represented as follows:

$$A + B \rightarrow X^{*} + Y$$

$$X^{*} \rightarrow X + h\nu,$$

where X* is an electronically excited molecule and hy represents light emission upon return of X* to a lower energy state. Where the luminescence is bioluminescence, creation of the excited state derives from an enzyme catalyzed reaction. The color of the emitted light in a bioluminescent (or chemiluminescent or other luminescent) reaction is characteristic of the excited molecule, and is independent from its source of excitation and temperature.

An essential condition for bioluminescence is the use of molecular oxygen, either bound or free in the presence of a luciferase. Luciferases, are oxygenases, that act on a substrate, luciferin, in the presence of molecular oxygen and transform the substrate to an excited state. Upon return to a lower energy level, energy is released in the form of light (for reviews see, *e.g.,* McElroy *et al.* (1966) in *Molecular Architecture in Cell Physiology,* Hayashi *et al.,* eds., Prentice-Hall, Inc., Englewood Cliffs, NJ, pp. 63-80; Ward *et al.,* Chapter 7 in *Chemi-and Bioluminescence,* Burr, ed., Marcel Dekker, Inc. NY, pp.321-358; Hastings, J. W. in (1995) *Cell Physiology:Source Book,* N. Sperelakis (ed.), Academic Press, pp 665-681; *Luminescence, Narcosis and Life in the Deep Sea,* Johnson, Vantage Press, NY, see, esp. pp. 50-56).

Though rare overall, bioluminescence is more common in marine organisms than in terrestrial organisms. Bioluminescence has developed from as many as thirty evolutionarily distinct origins and, thus, is manifested in a variety of ways so that the biochemical and physiological mechanisms responsible for bioluminescence in different organisms

are distinct. Bioluminescent species span many genera and include microscopic organisms, such as bacteria (primarily marine bacteria including *Vibrio* species), fungi, algae and dinoflagellates, to marine organisms, including arthropods, mollusks, echinoderms, and chordates, and terrestrial organism including annelid worms and insects.

## Assays employing bioluminescence

During the past twenty years, high-sensitivity biochemical assays used in research and in medicine have increasingly employed luminescence and fluorescence rather than radioisotopes. This change has been driven partly by the increasing expense of radioisotope disposal and partly by the need to find more rapid and convenient assay methods. More recently, the need to perform biochemical assays *in situ* in living cells and whole animals has driven researchers toward protein-based luminescence and fluorescence. The uses of firefly luciferase for ATP assays, aequorin and obelin as calcium reporters, *Vargula* luciferase as a neurophysiological indicator, and the *Aequorea* green fluorescent protein as a protein tracer and pH indicator show the potential of bioluminescence-based methods in research laboratories.

Bioluminescence is also beginning to directly impact medicine and biotechnology; for example, *Aequorea* green fluorescent protein (GFP) is employed to mark cells in murine model systems and as a reporter in high throughput drug screening. *Renilla* luciferase is under development for use in diagnostic platforms.

## Bioluminescence generating systems

Bioluminescence, as well as other types of chemiluminescence, is used for quantitative determinations of specific substances in biology and medicine. For example, luciferase genes have been cloned and exploited as reporter genes in numerous assays, for many purposes. Since the different luciferase systems have different specific requirements, they may be used to detect and quantify a variety of substances. The majority of commercial bioluminescence applications are based on firefly (*Photinus pyralis*) luciferase. One of the first and still widely used assays involves the use of firefly luciferase to detect the presence of ATP. It is also used to detect and quantify other substrates or co-factors in the reaction. Any reaction that produces or utilizes NAD(H), NADP(H) or long chain aldehyde, either directly or indirectly, can be coupled to the light-emitting reaction of bacterial luciferase.

Another luciferase system that has been used commercially for analytical purposes is the *Aequorin* system. The purified jellyfish photoprotein, aequorin, is used to detect and quantify intracellular $Ca^{2+}$ and its changes under various experimental conditions. The *Aequorin* photoprotein is relatively small (~20kDa), nontoxic, and can be injected into cells in quantities adequate to detect calcium over a large concentration range ($3 \times 10^{-7}$ to $10^{-4}$ M).

Because of their analytical utility, luciferases and substrates have been studied and well-characterized and are commercially available (*e.g.*, firefly luciferase is available from Sigma, St. Louis, MO, and Boehringer Mannheim Biochemicals,Indianapolis, IN; recombinantly produced firefly luciferase and other reagents based on this gene or for use with this protein are available from Promega Corporation, Madison, WI; the aequorin photoprotein luciferase from jellyfish and luciferase from *Renilla* are commercially available from Sealite Sciences, Bogart, GA; coelenterazine, the naturally-occurring substrate for these luciferases, is available from Molecular Probes, Eugene, OR). These luciferases and related reagents are used as reagents for diagnostics, quality control, environmental testing and other such analyses.

Because of the utility of luciferases as reagents in analytical systems and the potential for use in high throughput screening systems, there is a need to identify and isolated a variety of luciferases that have improved or different spectral properties compared to those presently available. For all these reasons, it would be advantageous to have luciferases from a variety of species, such as *Gaussia* and various *Renilla* species available.

## Fluorescent Proteins

Reporter genes, when co-transfected into recipient cells with a gene of interest, provide a means to detect transfection and other events. Among reporter genes are those that encode fluorescent proteins. The bioluminescence generating systems described herein are among those used as reporter genes. To increase the sensitivity bioluminescence generating systems have been combined with fluorescent compounds and proteins, such as naturally fluorescent phycobiliproteins. Also of interest are the fluorescent proteins that are present in a variety of marine invertebrates, such as the green and blue fluorescent proteins, particularly the green fluorescent protein (GFP) of *Aequorea victoria.*

The green fluorescent proteins (GFP) constitute a class of chromoproteins found only among certain bioluminescent coelenterates. These accessory proteins are fluorescent and function as the ultimate bioluminescence emitter in these organisms by accepting energy from enzyme-bound, excited-state oxyluciferin (*e.g.*, see Ward *et al.* (1979) *J. Biol. Chem. 254*:781-788; Ward *et al.* (1978) *Photochem. Photobiol. 27*:389-396; Ward *et al.* (1982) *Biochemistry 21*: 4535-4540).

The best characterized GFPs are those isolated from the jellyfish species *Aequorea,* particularly *Aequorea*

*victoria* (*A. victoria)* and *Aequorea forskålea* (Ward *et al*. (1982) *Biochemistry 21*:4535-4540; Prendergast *et al.* (1978) *Biochemistry 17*:3448-3453). Purified *A. victoria* GFP is a monomeric protein of about 27 Kda that absorbs blue light with excitation wavelength maximum of 395 nm, with a minor peak at 470 nm, and emits green fluorescence with an emission wavelength of about 510 nm and a minor peak near 540 nm (Ward *et al.* (1979) *Photochem. Photobiol. Rev 4*:1-57). This GFP has certain limitations. The excitation maximum of the wildtype GFP is not within the range of wavelengths of standard fluorescein detection optics.

The detection of green fluorescence does not require any exogenous substrates or co-factors. Instead, the high level of fluorescence results from the intrinsic chromophore of the protein. The chromophore includes modified amino acid residues within the polypeptide chain. For example, the fluorescent chromophore of *A. victoria* GFP is encoded by the hexapeptide sequence, FSYGVQ, encompassing amino acid residues 64-69. The chromophore is formed by the intramolecular cyclization of the polypeptide backbone at residues Ser65 and Gly67 and the oxidation of the $\alpha$-$\beta$ bond of residue Tyr66 (*e.g.*, see Cody *et al.* (1993) *Biochemistry 32*:1212-1218; Shimomura (1978) *FEBS Letters 104*:220-222; Ward *et al*. (1989) *Photochem. Photobiol. 49*:62S). The emission spectrum of the isolated chromophore and the denatured protein at neutral Ph do not match the spectrum of the native protein, suggesting that chromophore formation occurs post-transiationally *(e.g.,* see Cody *et al.* (1993) *Biochemistry 32*:1212-1218).

In addition, the crystal structure of purified *A. victoria* GFP has been determined *(e.g.,* see Ormö (1996) *Science 273*:1392-1395). The predominant structural features of the protein are an 11-stranded $\beta$ barrel that forms a nearly perfect cylinder wrapping around a single central $\alpha$-helix, which contains the modified *p*-hydroxybenzylideneimadaxolidinone chromophore. The chromophore is centrally located within the barrel structure and is completely shielded from exposure to bulk solvent.

DNA encoding an isotype of *A. victoria* GFP has been isolated and its nucleotide sequence has been determined (*e.g.*, see Prasher (1992) *Gene 111*:229-233). The *A. victoria* CDNA contains a 714 nucleotide open reading frame that encodes a 238 amino acid polypeptide of a calculated M, of 26,888 Da. Recombinantly expressed *A. victoria* GFPs retain their ability to fluoresce *in vivo* in a wide variety organisms, including bacteria *(e.g.,* see Chalfie *et al.* (1994) *Science 263*:802-805; Miller *et al.* (1997) *Gene 191*:149-153), yeast and fungi (Fey *et al.* (1995) *Gene 165*:127-130; Straight *et al*. (1996) *Curr. Biol. 6*:1599-1608; Cormack *et al*. (1997) *Microbiology 143*:303-311), *Drosophila* (*e.g.*, see Wang *et al*. (1994) *Nature 369*:400-403; Plautz (1996) *Gene 173*:83-87), plants (Heinlein *et al*. (1995); Casper *et al*. (1996) *Gene 173*:69-73), fish (Amsterdam *et al*. (1995)), and mammals (Ikawa *et al*. (1995). *Aequorea* GFP vectors and isolated *Aequorea* GFP proteins have been used as markers for measuring gene expression, cell migration and localization, microtubule formation and assembly of functional ion channels (*e.g.*, see Terry *et al.* (1995) *Biochem. Biophys. Res. Commun. 217*:21-27; Kain *et al.* (1995) *Biotechniques 19*:650-655).The *A. victoria* GFP, however, is not ideal for use in analytical and diagnostic processes. Consequently GFP mutants have been selected with the hope of identifying mutants that have single excitation spectral peaks shifted to the red.

In fact a stated purpose in constructing such mutants has been to attempt to make the *A. victoria* GFP more like the GFP from *Renilla,* but which has properties that make it far more ideal for use as an analytical tool. For many practical applications, the spectrum of Renilla GFP is be preferable to that of the *Aequorea* GFP, because wavelength discrimination between different fluorophores and detection of resonance energy transfer are easier if the component spectra are tall and narrow rather than low and broad (see, U.S. Patent No. 5,625,048). Furthermore, the longer wavelength excitation peak (475 nm) of *Renilla* GFP is almost ideal for fluorescein filter sets and is resistant to photobleaching, but has lower amplitude than the shorter wavelength peak at 395 nm, which is more susceptible to photobleaching (Chalfie *et al.* (1994) *Science 263*:802-805).

There exists a phylogenetically diverse and largely unexplored repertoire of bioluminescent proteins that are a reservoir for future development. For these reasons, it would be desirable to have a variety of new luciferases and fluorescent proteins, particularly, *Renilla reniformis* GFP available rather than use muteins of *A. victoria* GFP. Published International PCT application No. WO 99/49019 (see, also, allowed U.S. application Serial No. 09/277,716) provides a variety of GFPs including those from *Renilla* species. It remains desirable to have a variety of GFPs and luciferases available in order to optimize systems for particular applications and to improve upon existing methods. Therefore, it is an object herein to provide isolated nucleic acid molecules encoding *Renilla reniformis* GFP and the protein encoded thereby. It is also an object herein to provide bioluminescence generating systems that include the luciferases, luciferins, and also include *Renilla reniformis* GFP.

## SUMMARY OF THE INVENTION

Isolated nucleic acid molecules that encode *Renilla reniformis* fluorescent proteins are provided. Nucleic acid probes and primers derived therefrom are also provided. Functionally equivalent nucleic acids, such as those that hybridize under conditions of high stringency to the disclosed molecules and those that have high sequence identity, are also contemplated. Nucleic acid molecules and the encoded proteins are set forth in SEQ ID Nos. 23-27, an exemplary mutein is set forth in SEQ ID No. 33. Also contemplated are nucleic acid molecules that encode the protein set forth in

SEQ ID No. 27.

Host cells, including bacterial, yeast and mammalian host cells, and plasmids for expression of the nucleic acids encoding the *Renilla reniformis* green fluorescent protein (GFP), are also provided. Combinations of luciferases and the *Renilla reniformis* GFP are also provided.

The genes can be modified by substitution of codons optimized for expression in selected host cells or hosts, such as humans and other mammals, or can be mutagenized to alter the emission properties. Mutations that alter spectral properties are also contemplated.

Such mutations may be identified by substituting each codon with one encoding another amino acid, such as alanine, and determining the effect on the spectral properties of the resulting protein. Particular regions of interest are those in which corresponding the sites mutated in other GFPs, such Aequora to produce proteins with altered spectral properties are altered.

The *Renilla reniformis* GFP may be used in combination with nucleic acids encoding luciferases, such as those known to those of skill in the art and those that are described in copending allowed U.S. application Serial No. 09/277,716 (see, also, Published International PCT application No. WO 99/49019).

Compositions containing the *Renilla reniformis* GFP or the *Renilla reniformis* GFP and luciferase combination are provided. The compositions can take any of a number of forms, depending on the intended method of use therefor. In certain embodiments, for example, the compositions contain a *Gaussia* luciferase, *Gaussia* luciferase peptide or *Gaussia* luciferase fusion protein, formulated for use in luminescent novelty items, immunoassays, donors in FET (fluorescent energy transfer) assays, FRET (fluorescent resonance energy transfer) assays, HTRF (homogeneous time-resolved fluorescence) assays or used in conjunction with multi-well assay devices containing integrated photodetectors, such as those described herein.

The bioluminescence-generating system includes, in addition to the luciferase a *Renilla reniformis* GFP or mutated form thereof. These compositions can be used in a variety of methods and systems, such as included in conjunction with diagnostic systems for the *in vivo* detection of neoplastic tissues and other tissues, such as those methods described herein.

Combinations of the *Renilla reniformis* GFP with an articles of manufacture to produce novelty items are provided. These novelty items are designed for entertainment, recreation and amusement, and include, but are not limited to: toys, particularly squirt guns, toy cigarettes, toy "Halloween" eggs, footbags and board/card games; finger paints and other paints, slimy play material; textiles, particularly clothing, such as shirts, hats and sports gear suits, threads and yarns; bubbles in bubble making toys and other toys that produce bubbles; balloons; figurines; personal items, such as cosmetics, bath powders, body lotions, gels, powders and creams, nail polishes, make-up, toothpastes and other dentifrices, soaps, body paints, and bubble bath; items such as inks, paper; foods, such as gelatins, icings and frostings; fish food containing luciferins and transgenic fish, particularly transgenic fish that express a luciferase; plant food containing a luciferin or luciferase, preferably a luciferin for use with transgenic plants that express luciferase; and beverages, such as beer, wine, champagne, soft drinks, and ice cubes and ice in other configurations; fountains, including liquid "fireworks" and other such jets or sprays or aerosols of compositions that are solutions, mixtures, suspensions, powders, pastes, particles or other suitable form. The combinations optionally include a bioluminescence generating system. The bioluminescence generating systems can be provided as two compositions: a first composition containing a luciferase and a second composition containing one or more additional components of a bioluminescence generating system.

Any article of manufacture that can be combined with a bioluminescence-generating system as provided herein and thereby provide entertainment, recreation and/or amusement, including use of the items for recreation or to attract attention, such as for advertising goods and/or services that are associated with a logo or trademark is contemplated herein. Such uses may be in addition to or in conjunction with or in place of the ordinary or normal use of such items. As a result of the combination, the items glow or produce, such as in the case of squirt guns and fountains, a glowing fluid or spray of liquid or particles. The novelty in the novelty item derives from its bioluminescence.

## GFPS

Isolated nucleic acids that encode GFP from *Renilla reniformis* are provided herein. Also provided are isolated and purified nucleic acids that encode a component of the bioluminescence generating system and a the green fluorescent protein (GFP) (see SEQ ID Nos. 23-27). In particular, nucleic acid molecules that encode *Renilla reniformis* green fluorescent protein (GFPs) and nucleic acid probes and primers derived therefrom are provided. Nucleic acid molecules encoding *Renilla reniformis* GFP are provided (see SEQ ID Nos. 23-26).

Nucleic acid probes and primers containing 14, 16, 30, 100 or more contiguous nucleotides from any of SEQ ID Nos. 23-26. Nucleic acid probes can be labeled, which if needed, for detection, containing at least about 14, preferably at least about 16, or, if desired, 20 or 30 or more, contiguous nucleotides of sequence of nucleotides encoding the *Renilla reniformis* GFP.

Methods using the probes for the isolation and cloning of GFP-encoding DNA in *Renilla reniformis* are also provided. Vectors containing DNA encoding the *Renilla reniformis* GFP are provided. In particular, expression vectors that contain DNA encoding a *Renilla reniformis* or in operational association with a promoter element that allows for the constitutive or inducible expression of *Renilla reniformis.*

The vectors are capable of expressing the *Renilla reniformis* GFP in a wide variety of host cells. Vectors for producing chimeric *Renilla reniformis* GFP/luciferase fusion proteins and/or polycistronic mRNA containing a promoter element and a multiple cloning site located upstream or downstream of DNA encoding *Renilla reniformis* GFP are also provided.

Recombinant cells containing heterologous nucleic acid encoding a *Renilla reniformis* GFP are also provided. Purified *Renilla reniformis* GFP peptides and compositions containing the *Renilla* GFPs and GFP peptides alone or in combination with at least one component of a bioluminescence-generating system, such as a *Renilla* luciferase, are provided. The *Renilla* GFP and GFP peptide compositions can be used, for example, to provide fluorescent illumination of novelty items or used in methods of detecting and visualizing neoplastic tissue and other tissues, detecting infectious agents using immunoassays, such homogenous immunoassays and *in vitro* fluorescent-based screening assays using multi-well assay devices, or provided in kits for carrying out any of the above-described methods. In particular, these proteins may be used in FP (fluorescence polarization) assays, FET (fluorescent energy transfer) assays, FRET (fluorescent resonance energy transfer) assays and HTRF (homogeneous time-resolved fluorescence) assays and also in the BRET assays and sensors provided herein.

Non-radioactive energy transfer reactions, such as FET or FRET, FP and HTRF assays, are homogeneous luminescence assays based on energy transfer are carried out between a donor luminescent label and an acceptor label (see, *e.g.,* Cardullo *et al.* (1988) *Proc. Natl. Acad. Sci. U.S.A. 85*:8790-8794; Peerce *et al.* (1986) *Proc. Natl. Acad. Sci. U.S.A. 83*:8092-8096; U.S. Patent No. 4,777,128; U.S. Patent No. 5,162,508; U.S. Patent No. 4,927,923; U.S. Patent No. 5,279,943; and International PCT Application No. WO 92/01225). Non-radioactive energy transfer reactions using GFPs have been developed (see, International PCT application Nos. WO 98/02571 and WO 97/28261). Non-radioactive energy transfer reactions using GFPs and luciferases, such as a luciferase and its cognate GFP (or multimers thereof), such as in a fusion protein, are contemplated herein.

Nucleic acids that exhibit substantial sequence identity with the nucleic acids provided herein are also contemplated. These are nucleic acids that can be produced by substituting codons that encode conservative amino acids and also nucleic acids that exhibit at least about 80%, preferably 90 or 95% sequence identity. Sequence identity refers to identity as determined using standard programs with default gap penalties and other defaults as provided by the manufacturer thereof.

The nucleic acids provide an opportunity to produce luciferases and GFPs, which have advantageous application in all areas in which luciferase/luciferins and GFPs have application. The nucleic acids can be used to obtain and produce GFPs and GFPs from other, particularly *Renilla* species using the probes described herein that correspond to conserved regions. These GFPs have advantageous application in all areas in which GFPs and/or luciferase/luciferins have application. For example, The GFP's provide a means to amplify the output signal of bioluminescence generating systems. *Renilla* GFP has a single excitation absorbance peak in blue light (and around 498 nm) and a predominantly single emission peak around 510 nm (with a small shoulder near 540). This spectra provides a means for it to absorb blue light and efficiently convert it to green light. This results in an amplification of the output. When used in conjunction with a bioluminescence generating system that yields blue light, such as *Aequorea* or *Renilla* or *Vargula (Cypridina),* the output signal for any application, including diagnostic applications, is amplified. In addition, this green light can serve as an energy donor in fluorescence-based assays, such as fluorescence polarization assays, FET (fluorescent energy transfer) assays, FRET (fluorescent resonance energy transfer) assays and HTRF (homogeneous time-resolved fluorescence) assays. Particular assays, herein referred to as BRET (bioluminescence resonance energy transfer assays in which energy is transferred from a bioluminescence reaction of a luciferase to a fluorescent protein), are provided.

Non-radioactive energy transfer reactions, such as FET or FRET, FP and HTRF assays, are homogeneous luminescence assays based on energy transfer that are carried out between a donor luminescent label and an acceptor label (see, *e.g.,* Cardullo *et al.* (1988) *Proc. Natl. Acad. Sci. U.S.A. 85*:8790-8794; Peerce *et al.* (1986) *Proc. Natl. Acad. Sci. U.S.A. 83*:8092-8096; U.S. Patent No. 4,777,128; U.S. Patent No. 5,162,508; U.S. Patent No. 4,927,923; U.S. Patent No. 5,279,943; and International PCT Application No. WO 92/01225). Non-radioactive energy transfer reactions using GFPs have been developed (see, International PCT application Nos. WO 98/02571 and WO 97/28261).

Mutagenesis of the GFPs is contemplated herein, particularly mutagenesis that results in modified GFPs that have red-shifted excitation and emission spectra. The resulting systems have higher output compared to the unmutagenized forms. These GFPs may be selected by random mutagenesis and selection for GFPs with altered spectra or by selected mutagenesis of the chromophore region of the GFP.

The DNA may be introduced as a linear DNA molecule (fragment) or may be included in an expression vector for stable or transient expression of the encoding DNA. In certain embodiments, the cells contain DNA or RNA encoding a *Renilla* GFP also express the recombinant *Renilla* GFP or polypeptide. It is preferred that the cells are selected to

express functional GFPs that retain the ability to fluorescence and that are not toxic to the host cell. In some embodiments, cells may also include heterologous nucleic acid encoding a component of a bioluminescence-generating system, preferably a photoprotein or luciferase. In preferred embodiments, the nucleic acid encoding the bioluminescence-generating system component is isolated from the species *Aequorea, Vargula, Pleuromamma, Ptilosarcus* or *Renilla.* In more preferred embodiments, the bioluminescence-generating system component is a *Renilla reniformis* luciferase or *mulleri* including the amino acid sequence set forth in SEQ ID No. 18 or the *Pleuromamma* luciferase set forth in SEQ ID No. 28, or the *Gaussia* luciferase set forth in SEQ ID No. 19.

The GFPs provided herein may be used in combination with any suitable bioluminescence generating system, but is preferably used in combination with a *Renilla* or *Aequorea, Pleuromamma* or *Gaussia* luciferase.

Purified *Renilla* GFPs, particularly purified *Renilla reniformis* GFP peptides are provided. Presently preferred *Renilla* GFP for use in the compositions herein is *Renilla reniformis* GFP including the sequence of amino acids set forth above and in the Sequence Listing.

Fusions of the nucleic acid, particularly DNA, encoding *Renilla* GFP with DNA encoding a luciferase are also provided herein.

The cells that express functional luciferase and/or GFP, which may be used alone or in conjunction with a bioluminescence-generating system, in cell-based assays and screening methods, such as those described herein.

Presently preferred host cells for expressing GFP and luciferase are bacteria, yeasts, fungi, plant cells, insect cells and animal cells.

The luciferases and GFPs or cells that express them also may be used in methods of screening for bacterial contamination and methods of screening for metal contaminants. To screen for bacterial contamination, bacterial cells that express the luciferase and/or GFP are put in autoclaves or in other areas in which testing is contemplated. After treatment or use of the area, the area is tested for the presence of glowing bacteria. Presence of such bacteria is indicative of a failure to eradicate other bacteria. Screening for heavy metals and other environmental contaminants can also be performed with cells that contain the nucleic acids provided herein, if expression is linked to a system that is dependent upon the particular heavy metal or contaminant.

The systems and cells provided herein can be used for high throughout screening protocols, intracellular assays, medical diagnostic assays, environmental testing, such as tracing bacteria in water supplies, in conjunction with enzymes for detecting heavy metals, in spores for testing autoclaves in hospital, foods and industrial autoclaves. Non-pathogenic bacteria containing the systems can be included in feed to animals to detect bacterial contamination in animal products and in meats.

Compositions containing a *Renilla* GFP are provided. The compositions can take any of a number of forms, depending on the intended method of use therefor. In certain embodiments, for example, the compositions contain a *Renilla* GFP or GFP peptide, preferably *Renilla mulleri* GFP or *Renilla reniformis* GFP peptide, formulated for use in luminescent novelty items, immunoassays, FET (fluorescent energy transfer) assays, FRET (fluorescent resonance energy transfer) assays, HTRF (homogeneous time-resolved fluorescence) assays or used in conjunction with multi-well assay devices containing integrated photodetectors, such as those described herein. In other instances, the GFPs are used in beverages, foods or cosmetics.

Compositions that contain a *Renilla reniformis* GFP or GFP peptide and at least one component of a bioluminescence-generating system, preferably a luciferase, luciferin or a luciferase and a luciferin, are provided. In preferred embodiments, the luciferase/luciferin bioluminescence- generating system is selected from those isolated from: an insect system, a coelenterate system, a ctenophore system, a bacterial system, a mollusk system, a crustacea system, a fish system, an annelid system, and an earthworm system. Bioluminescence-generating systems include those isolated from *Renilla, Aequorea,* and *Vargula, Gaussia* and *Pleuromamma.*

Combinations containing a first composition containing a *Renilla reniformis* GFP or *Ptilosarcus* GFP or mixtures thereof and a second composition containing a bioluminescence- generating system for use with inanimate articles of manufacture to produce novelty items are provided. These novelty items, which are articles of manufacture, are designed for entertainment, recreation and amusement, and include, but are not limited to: toys, particularly squirt guns, toy cigarettes, toy "Halloween" eggs, footbags and board/card games; finger paints and other paints, slimy play material; textiles, particularly clothing, such as shirts, hats and sports gear suits, threads and yarns; bubbles in bubble making toys and other toys that produce bubbles; balloons; figurines; personal items, such as bath powders, body lotions, gels, powders and creams, nail polishes, cosmetics including make-up, toothpastes and other dentifrices, soaps, cosmetics, body paints, and bubble bath, bubbles made from non-detergent sources, particularly proteins such as albumin and other non-toxic proteins; in fishing lures and glowing transgenic worms, particularly crosslinked polyacrylamide containing a fluorescent protein and/or components of a bioluminescence generating system, which glow upon contact with water; items such as inks, paper; foods, such as gelatins, icings and frostings; fish food containing luciferins and transgenic animals, such as transgenic fish, worms, monkeys, rodents, ungulates, ovine, ruminants and others not human, that express a luciferase and/or *Renilla reniformis* GFP; transgenic worms that express *Renilla reniformis* GFP and are used as lures; plant food containing a luciferin or luciferase, preferably a luciferin for use with transgenic plants that express

luciferase and *Renilla reniformis* GFP, transgenic plants that express *Renilla reniformis* GFP, particularly ornamental plants, such as orchids, roses, and other plants with decorative flowers; transgenic plants and animals in which the *Renilla reniformis* GFP is a marker for tracking introduction of other genes; and beverages, such as beer, wine, champagne, soft drinks, milk and ice cubes and ice in other configurations containing *Renilla reniformis* GFP; fountains, including liquid "fireworks" and other such jets or sprays or aerosols of compositions that are solutions, mixtures, suspensions, powders, pastes, particles or other suitable form.

Any article of manufacture that can be combined with a bioluminescence-generating system and *Renilla reniformis* GFP or with just a *Renilla reniformis* GFP, as provided herein, that thereby provide entertainment, recreation and/or amusement, including use of the items for recreation or to attract attention, such as for advertising goods and/or services that are associated with a logo or trademark is contemplated herein. Such uses may be in addition to or in conjunction with or in place of the ordinary or normal use of such items. As a result of the combination, the items glow or produce, such as in the case of squirt guns and fountains, a glowing fluid or spray of liquid or particles.

Methods for diagnosis and visualization of tissues *in vivo* or *in situ* using compositions containing a *Renilla reniformis* GFP and/or a *Renilla reniformis or mulleri* luciferase or others of the luciferases and/or GFPs provided herein are provided. For example, the *Renilla reniformis* GFP protein can be used in conjunction with diagnostic systems that rely on bioluminescence for visualizing tissues *in situ.* The systems are particularly useful for visualizing and detecting neoplastic tissue and specialty tissue, such as during non-invasive and invasive procedures. The systems include compositions containing conjugates that include a tissue specific, particularly a tumor-specific, targeting agent linked to a targeted agent, a *Renilla reniformis* GFP, a luciferase or luciferin. The systems also include a second composition that contains the remaining components of a bioluminescence generating reaction and/or the *Renilla reniformis* GFP. In some embodiments, all components, except for activators, which are provided *in situ* or are present in the body or tissue, are included in a single composition.

Methods for diagnosis and visualization of tissues *in vivo* or *in situ* using compositions containing a *Gaussia* luciferase are provided. For example, the *Gaussia* luciferase or *Gaussia* luciferase peptide can be used in conjunction with diagnostic systems that rely on bioluminescence for visualizing tissues *in situ.* The systems are particularly useful for visualizing and detecting neoplastic tissue and specialty tissue, such as during non-invasive and invasive procedures. The systems include compositions containing conjugates that include a tissue specific, particularly a tumor-specific, targeting agent linked to a targeted agent, a *Gaussia* luciferase, a GFP or luciferin. The systems also include a second composition that contains the remaining components of a bioluminescence generating reaction and/or the *Gaussia* luciferase. In some embodiments, all components, except for activators, which are provided *in situ* or are present in the body or tissue, are included in a single composition.

In particular, the diagnostic systems include two compositions. A first composition that contains conjugates that, in preferred embodiments, include antibodies directed against tumor antigens conjugated to a component of the bioluminescence generating reaction, a luciferase or luciferin, preferably a luciferase are provided. In certain embodiments, conjugates containing tumor-specific targeting agents are linked to luciferases or luciferins. In other embodiments, tumor-specific targeting agents are linked to microcarriers that are coupled with, preferably more than one of the bioluminescence generating components, preferably more than one luciferase molecule.

The second composition contains the remaining components of a bioluminescence generating system, typically the luciferin or luciferase substrate. In some embodiments, these components, particularly the luciferin are linked to a protein, such as a serum albumin, or other protein carrier. The carrier and time release formulations, permit systemically administered components to travel to the targeted tissue without interaction with blood cell components, such as hemoglobin that deactivates the luciferin or luciferase.

Methods for diagnosing diseases, particularly infectious diseases, using chip methodology (see, *e.g.,* copending U.S. application Serial No. 08/990,103) a luciferase/luciferin bioluminescence-generating system and a *Renilla reniformis* GFP are provided. In particular, the chip includes an integrated photodetector that detects the photons emitted by the bioluminescence-generating system, particularly using luciferase encoded by the nucleic acids provided herein and/or *Renilla reniformis* GFP.

In one embodiment, the chip is made using an integrated circuit with an array, such as an X-Y array, of photodetectors. The surface of circuit is treated to render it inert to conditions of the diagnostic assays for which the chip is intended, and is adapted, such as by derivatization for linking molecules, such as antibodies. A selected antibody or panel of antibodies, such as an antibody specific for a bacterial antigen, is affixed to the surface of the chip above each photodetector. After contacting the chip with a test sample, the chip is contacted with a second antibody linked to a *Renilla* GFP, a chimeric antibody-*Renilla* GFP fusion protein or an antibody linked to a component of a bioluminescence generating system, such as a luciferase or luciferin, that are specific for the antigen. The remaining components of the bioluminescence generating reaction are added, and, if any of the antibodies linked to a component of a bioluminescence generating system are present on the chip, light will be generated and detected by the adjacent photodetector. The photodetector is operatively linked to a computer, which is programmed with information identifying the linked antibodies, records the event, and thereby identifies antigens present in the test sample.

Methods for generating chimeric GFP fusion proteins are provided. The methods include linking DNA encoding a gene of interest, or portion thereof, to DNA encoding a GFP coding region in the same translational reading frame. The encoded-protein of interest may be linked in-frame to the amino- or carboxyl-terminus of the GFP. The DNA encoding the chimeric protein is then linked in operable association with a promoter element of a suitable expression vector. Alternatively, the promoter element can be obtained directly from the targeted gene of interest and the promoter-containing fragment linked upstream of the GFP coding sequence to produce chimeric GFP proteins or two produce polycistronic mRNAs that encode the *Renilla reniformis* GFP and a luciferase, preferably a *Renilla* luciferase, more preferably *Renilla reniformis* luciferase.

Methods for identifying compounds using recombinant cells that express heterologous DNA encoding a *Renilla reniformis* GFP under the control of a promoter element of a gene of interest are provided. The recombinant cells can be used to identify compounds or ligands that modulate the level of transcription from the promoter of interest by measuring *Renilla reniformis* GFP-mediated fluorescence. Recombinant cells expressing the chimeric *Renilla reniformis* GFP or polycistronic mRNA encoding *Renilla reniformis* and a luciferase, may also be used for monitoring gene expression or protein trafficking, or determining the cellular localization of the target protein by identifying localized regions of GFP-mediated fluorescence within the recombinant cell.

Other assays using the GFPs and/or luciferases are contemplated herein. Any assay or diagnostic method known used by those of skill in the art that employ *Aequora* GFPs and/or other luciferases are contemplated herein.

Kits containing the GFPs for use in the methods, including those described herein, are provided. In one embodiment, the kits containing an article of manufacture and appropriate reagents for generating bioluminescence are provided. The kits containing such soap compositions, with preferably a moderate Ph (between 5 and 8) and bioluminescence generating reagents, including luciferase and luciferin and the GFP are provided herein. These kits, for example, can be used with a bubble-blowing or producing toy. These kits can also include a reloading or charging cartridge or can be used in connection with a food.

In another embodiment, the kits are used for detecting and visualizing neoplastic tissue and other tissues and include a first composition that contains the GFP and at least one component of a bioluminescence generating system, and a second that contains the activating composition, which contains the remaining components of the bioluminescence generating system and any necessary activating agents.

Thus, these kits will typically include two compositions, a first composition containing the GFP formulated for systemic administration (or in some embodiments local or topical application), and a second composition containing the components or remaining components of a bioluminescence generating system, formulated for systemic, topical or local administration depending upon the application. Instructions for administration will be included.

In other embodiments, the kits are used for detecting and identifying diseases, particularly infectious diseases, using multi-well assay devices and include a multi-well assay device containing a plurality of wells, each having an integrated photodetector, to which an antibody or panel of antibodies specific for one or more infectious agents are attached, and composition containing a secondary antibody, such as an antibody specific for the infectious agent that is linked to a *Renilla reniformis* GFP protein, a chimeric antibody-*Renilla reniformis)* GFP fusion protein or $F(Ab)_2$ antibody fragment-*Renilla reniformis* GFP fusion protein. A second composition containing a bioluminescence generating system that emits a wavelength of light within the excitation range of the *Renilla mulleri* GFP, such as species of *Renilla* or *Aequorea,* for exciting the *Renilla reniformis,* which produces light that is detected by the photodetector of the device to indicate the presence of the agent.

As noted above, fusions of nucleic acid encoding the luciferases and or GFPs provided herein with other luciferases and GFPs are provided. Of particular interest are fusions that encode pairs of luciferases and GFPs, such as a *Renilla* luciferase and a *Renilla* GFP (or a homodimer or other multiple of a *Renilla* GFP). The luciferase and GFP bind and in the presence of a luciferin will produced fluorescence that is red shifted compared to the luciferase in the absence of the GFP. This fusion or fusions in which the GFP and luciferase are linked via a target, such as a peptide, can be used as a tool to assess anything that interacts with the linker.

Muteins of the GFPs and luciferases are provided. Of particular interest are muteins, such as temperature sensitive muteins, of the GFP and luciferases that alter their interaction, such as mutations in the *Renilla* luciferase and *Renilla* GFP that alters their interaction at a critical temperature.

Antibodies, polyclonal and monoclonal antibodies that specifically bind to any of the proteins encoded by the nucleic acids provided herein are also provided. These antibodies, monoclonal or polyclonal, can be prepared employing standard techniques, known to those of skill in the art. In particular, immunoglobulins or antibodies obtained from the serum of an animal immunized with a substantially pure preparation of a luciferase or GFP provided herein or an or epitope-containing fragment thereof are provided. Monoclonal antibodies are also provided. The immunoglobulins that are produced have, among other properties, the ability to specifically and preferentially bind to and/or cause the immunoprecipitation of a GFP or luciferase, particularly a *Renilla* or *Ptilosarcus* GFP or a *Pleuromamma, Gaussia* or *Renilla mulleri* luciferase, that may be present in a biological sample or a solution derived from such a biological sample.

## DESCRIPTION OF THE FIGURES

FIGURE 1 depicts phylogenetic relationships among the anthozoan GFPs.

FIGURE 2 illustrates the underlying principle of Bioluminescent Resonance Energy Transfer (BRET) and its use as sensor: A) in isolation, a luciferase, preferably an anthozoan luciferase, emits blue light from the coelenterazine-derived chromophore; B) in isolation, a GFP, preferably an anthozoan GFP that binds to the luciferase, that is excited with blue-green light emits green light from its integral peptide based fluorophore; C) when the luciferase and GFP associate as a complex *in vivo* or *in vitro,* the luciferase non-radiatively transfers its reaction energy to the GFP flurophore, which then emits the green light; D) any molecular interaction that disrupts the luciferase-GFP complex can be quantitatively monitored by observing the spectral shift from green to blue light.

FIGURE 3 illustrates exemplary BRET sensor architecture.

FIGURE 4 depicts the substitution of altered fluorophores into the background of *Ptilosarcus, Renilla mulleri* and *Renilla reniformis* GFPs (the underlined regions corresponds to amino acids 56-75 of SEQ ID No. 27 *Renilla reniformis* GFP; amino acids 59-78 of SEQ ID No. 16 *Renilla mulleri* GFP; and amino acids 59-78 of SEQ ID No. 32 for *Ptilosarcus* GFP).

FIGURE 5 depicts the three anthozoan fluorescent protein for which a crystal structure exists, another available commercially from Clontech as dsRed (from *Discosoma striata;* also known as drFP583, as in this alignment); a dark gray background depicts amino acid conservation, and a light gray background depicts shared physicochemical properties.

FIGURE 6 compares the sequences of a variety of GFPs, identifying sites for mutation to reduce multimerization; abbreviations are as follows: *Amemonia majona* is amFP486; *Zoanthus sp.* zFP506 and zFP538; *Discosoma sp.* "red" is drFP583; *Clavularia* sp. is cFP484; and the GFP from the anthozoal *A. sulcata* is designated FP595.

## DETAILED DESCRIPTION OF THE INVENTION

A. DEFINITIONS

B. Fluorescent Proteins

- 1. Green and blue fluorescent proteins
- *2. Renilla reniformis* GFP

C. BIOLUMINESCENCE GENERATING SYSTEMS AND COMPONENTS

- 1. General description
  - a. Luciferases
  - b. Luciferins
  - c. Activators
  - d. Reactions
- 2. The *Renilla* system
- 3. Ctenophore systems
- 4. The aequorin system
  - a. Aequorin and related photoproteins
  - b. Luciferin
- 5. Crustacean, particularly *Cyrpidina* systems
  - a. *Vargula* luciferase
    - (1) Purification from *Cypridina*

(2) Preparation by Recombinant Methods

b. *Vargula* luciferin
c. Reaction

6. Insect bioluminescent systems including fireflies, click beetles, and other insect system

a. Luciferase
b. Luciferin
c. Reaction

7. Other systems

a. Bacterial systems

(1) Luciferases
(2) Luciferins
(3) Reactions

b. Dinoflagellate bioluminescence generating systems

D. ISOLATION AND IDENTIFICATION OF NUCLEIC ACIDS ENCODING LUCIFERASES AND GFPs

1. Isolation of specimens of the genus *Renilla*
2. Preparation of *Renilla* cDNA expression libraries

a. RNA isolation and cDNA synthesis
b. Construction of cDNA expression libraries

3. Cloning of *Renilla reniformis* Green Fluorescent Protein
4. Isolation and identification of DNA encoding *Renilla mulleri* GFP
5. Isolation and identification of DNA encoding *Renilla mulleri* luciferase

E. RECOMBINANT EXPRESSION OF PROTEINS

1. DNA encoding *Renilla* proteins
2. DNA constructs for recombinant production of *Renilla reniformis* and other proteins
3. Host organisms for recombinant production of *Renilla* proteins
4. Methods for recombinant production of *Renilla* proteins
5. Recombinant cells expressing heterologous nucleic acid encoding luciferases and GFPs

F. COMPOSITIONS AND CONJUGATES

1. *Renilla* GFP compositions
2. *Renilla* luciferase compositions
3. Conjugates

a. Linkers
b. Targeting Agents
c. Anti-tumor Antigen Antibodies
d. Preparation of the conjugates

4. Formulation of the compositions for use in the diagnostic systems

a. The first composition: formulation of the conjugates
b. The second composition
c. Practice of the reactions in combination with targeting agents

G. COMBINATIONS
H. Exemplary uses of *Renilla reniformis* GFPs and encoding nucleic acid molecules

1. Methods for diagnosis of neoplasms and other tissues
2. Methods of diagnosing diseases
3. Methods for generating *Renilla mulleri* luciferase, *Pleuromamma* luciferase and *Gaussia* luciferase fusion proteins with *Renilla reniformis* GFP
4. Cell-based assays for identifying compounds

I. KITS
J. Muteins

1. Mutation of GFP surfaces to disrupt multimerization
2. Use of advantageous GFP surfaces with substituted fluorophores

K. Transgenic plants and animals
L. Bioluminescence Resonance Energy Transfer (BRET) System

1. Design of sensors based on BRET
2. BRET Sensor Architectures
3. Advantages of BRET sensors

**A. DEFINITIONS**

Unless defined otherwise, all technical and scientific terms used herein have the same meaning as is commonly understood by one of skill in the art to which this invention belongs. All patents, applications and publications of referred to throughout the disclosure are incorporated by reference in their entirety.

As used herein, chemiluminescence refers to a chemical reaction in which energy is specifically channeled to a molecule causing it to become electronically excited and subsequently to release a photon thereby emitting visible light. Temperature does not contribute to this channeled energy. Thus, chemiluminescence involves the direct conversion of chemical energy to light energy.

As used herein, luminescence refers to the detectable EM radiation, generally, UV, IR or visible EM radiation that is produced when the excited product of an exergic chemical process reverts to its ground state with the emission of light. Chemiluminescence is luminescence that results from a chemical reaction. Bioluminescence is chemiluminescence that results from a chemical reaction using biological molecules (or synthetic versions or analogs thereof) as substrates and/or enzymes.

As used herein, bioluminescence, which is a type of chemiluminescence, refers to the emission of light by biological molecules, particularly proteins. The essential condition for bioluminescence is molecular oxygen, either bound or free in the presence of an oxygenase, a luciferase, which acts on a substrate, a luciferin. Bioluminescence is generated by an enzyme or other protein (luciferase) that is an oxygenase that acts on a substrate luciferin (a bioluminescence substrate) in the presence of molecular oxygen and transforms the substrate to an excited state, which upon return to a lower energy level releases the energy in the form of light.

As used herein, the substrates and enzymes for producing bioluminescence are generically referred to as luciferin and luciferase, respectively. When reference is made to a particular species thereof, for clarity, each generic term is used with the name of the organism from which it derives, for example, bacterial luciferin or firefly luciferase.

As used herein, luciferase refers to oxygenases that catalyze a light emitting reaction. For instance, bacterial luciferases catalyze the oxidation of flavin mononucleotide (FMN) and aliphatic aldehydes, which reaction produces light. Another class of luciferases, found among marine arthropods, catalyzes the oxidation of *Cypridina* (*Vargula*) luciferin, and another class of luciferases catalyzes the oxidation of *Coleoptera* luciferin.

Thus, luciferase refers to an enzyme or photoprotein that catalyzes a bioluminescent reaction (a reaction that produces bioluminescence). The luciferases, such as firefly and *Gaussia* and *Renilla* luciferases, that are enzymes which act catalytically and are unchanged during the bioluminescence generating reaction. The luciferase photoproteins, such as the aequorin photoprotein to which luciferin is non-covalently bound, are changed, such as by release of the luciferin, during bioluminescence generating reaction. The luciferase is a protein that occurs naturally in an organism or a variant or mutant thereof, such as a variant produced by mutagenesis that has one or more properties, such as thermal stability, that differ from the naturally-occurring protein. Luciferases and modified mutant or variant forms thereof are well known. For purposes herein, reference to luciferase refers to either the photoproteins or luciferases.

Thus, reference, for example, to "*Gaussia* luciferase" means an enzyme isolated from member of the genus

*Gaussia* or an equivalent molecule obtained from any other source, such as from another related copepod, or that has been prepared synthetically. It is intended to encompass *Gaussia* luciferases with conservative amino acid substitutions that do not substantially alter activity. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, *e.g.,* Watson *et al. Molecular Biology of the Gene,* 4th Edition, 1987, The Bejacmin/Cummings Pub. co., p.224).

"*Renilla* GFP" refers to GFPs from the genus *Renilla* and to mutants or variants thereof. It is intended to encompass *Renilla* GFPs with conservative amino acid substitutions that do not substantially alter activity and physical properties, such as the emission spectra and ability to shift the spectral output of bioluminescence generating systems.

Such substitutions are preferably made in accordance with those set forth in TABLE 1 as follows:

**TABLE 1**

| Original residue | Conservative substitution |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gin |
| lie (I) | Leu; Val |
| Leu (L) | Ile; Val |
| Lys (K) | Arg; Gin; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions are also permissible and may be determined empirically or in accord with known conservative substitutions.

The luciferases and luciferin and activators thereof are referred to as bioluminescence generating reagents or components. Typically, a subset of these reagents will be provided or combined with an article of manufacture. Bioluminescence will be produced upon contacting the combination with the remaining reagents. Thus, as used herein, the component luciferases, luciferins, and other factors, such as $O_2$, $Mg^{2+}$, $Ca^{2+}$ are also referred to as bioluminescence generating reagents (or agents or components).

As used herein, a *Renilla reniformis* green fluorescent protein (GFP) refers to a fluorescent protein that is encoded by a sequence of nucleotides that encodes the protein of SEQ ID No. 27 or to a green fluorescent protein from *Renilla reniformis* having at least 80%,90% or 95% or greater sequence identity thereto; or that is encoded by a sequence of nucleotides that hybridizes under high stringency along its full length to the coding portion of the sequence of nucleotides set forth in any of SEQ ID Nos. 23-25. A *Renilla reniformis* GFP is protein that is fluorescent and is produced in a *Renilla reniformis.*

As used herein, bioluminescence substrate refers to the compound that is oxidized in the presence of a luciferase, and any necessary activators, and generates light. These substrates are referred to as luciferins herein, are substrates that undergo oxidation in a bioluminescence reaction. These bioluminescence substrates include any luciferin or analog thereof or any synthetic compound with which a luciferase interacts to generate light. Preferred substrates are those that are oxidized in the presence of a luciferase or protein in a light-generating reaction. Bioluminescence substrates, thus, include those compounds that those of skill in the art recognize as luciferins. Luciferins, for example, include firefly luciferin, *Cypridina* (also known as *Vargula)* luciferin (coelenterazine), bacterial luciferin, as well as synthetic analogs of these substrates or other compounds that are oxidized in the presence of a luciferase in a reaction the produces bioluminescence.

As used herein, capable of conversion into a bioluminescence substrate means susceptible to chemical reaction, such as oxidation or reduction, that yields a bioluminescence substrate. For example, the luminescence producing reaction of bioluminescent bacteria involves the reduction of a flavin mononucleotide group (FMN) to reduced flavin mononucleotide ($FMNH_2$) by a flavin reductase enzyme. The reduced flavin mononucleotide (substrate) then reacts with oxygen (an activator) and bacterial luciferase to form an intermediate peroxy flavin that undergoes further reaction, in the presence of a long-chain aldehyde, to generate light. With respect to this reaction, the reduced flavin and the long chain aldehyde are substrates.

As used herein, a bioluminescence generating system refers to the set of reagents required to conduct a bioluminescent reaction. Thus, the specific luciferase, luciferin and other substrates, solvents and other reagents that may be required to complete a bioluminescent reaction form a bioluminescence system. Thus a bioluminescence generating system refers to any set of reagents that, under appropriate reaction conditions, yield bioluminescence. Appropriate reaction conditions refers to the conditions necessary for a bioluminescence reaction to occur, such as pH, salt concentrations and temperature. In general, bioluminescence systems include a bioluminescence substrate, luciferin, a luciferase, which includes enzymes luciferases and photoproteins, and one or more activators. A specific bioluminescence system may be identified by reference to the specific organism from which the luciferase derives; for example, the *Vargula* (also called *Cypridina)* bioluminescence system (or Vargula system) includes a *Vargula* luciferase, such as a luciferase isolated from the ostracod, *Vargula* or produced using recombinant means or modifications of these luciferases. This system would also include the particular activators necessary to complete the bioluminescence reaction, such as oxygen and a substrate with which the luciferase reacts in the presence of the oxygen to produce light.

The luciferases provided herein may be incorporated into bioluminescence generating systems and used, as appropriate, with the GFPs provided herein or with other GFPs. Similarly, the GFPs provided herein may be used with known bioluminescence generating systems.

As used herein, the amino acids, which occur in the various amino acid sequences appearing herein, are identified according to their well-known, three-letter or one-letter abbreviations. The nucleotides, which occur in the various DNA molecules, are designated with the standard single-letter designations used routinely in the art.

As used herein, a fluorescent protein refers to a protein that possesses the ability to fluoresce (*i.e.*, to absorb energy at one wavelength and emit it at another wavelength). These proteins can be used as a fluorescent label or marker and in any applications in which such labels would be used, such as immunoassays, CRET, FRET, and FET assays, and in the assays designated herein as BRET assays. For example, a green fluorescent protein refers to a polypeptide that has a peak in the emission spectrum at about 510 nm.

As used herein, the term BRET (Bioluminescence Resonance Energy Transfer) refers to non-radiative luciferase-to-FP energy transfer. It differs from (Fluorescence Resonance Energy Transfer), which refers to energy transfer between chemical fluors.

As used herein, a BRET system refers the combination of a FP, in this case *Renilla reniformis* GFP and a luciferase for resonance energy transfer. BRET refers to any method in which the luciferase is used to generate the light upon reaction with a luciferin which is then non-radiatively transferred to a FP. The energy is transferred to a FP, particularly a GFP, which focuses and shifts the energy and emits it at a different wavelength. In preferred embodiments, the BRET system includes a bioluminescence generating system and a *Renilla reniformis* GFP. The bioluminescence generating system is preferably a *Renilla* system. Hence, the preferred pair is a *Renilla* luciferase and a *Renilla* GFP, which specifically interact. Alterations in the binding will be reflected in changes in the emission spectra of light produced by the luciferase. As a result the pair can function as a sensor of external events.

As used herein, a biosensor (or sensor) refers to a BRET system for use to detect alterations in the environment *in vitro* or *in vivo* in which the BRET system is used.

As used herein, modulator with reference to a BRET system refers to a molecule or molecules that undergo a conformation change in response to interaction with another molecule thereby affecting the proximity and/or orientation of the GFP and luciferase in the BRET system. Modulators include, but are not limited to, a protease site, a second messenger binding site, an ion binding molecule, a receptor, an oligomer, an enzyme substrate, a ligand, or other such binding molecule. If the GFP and luciferase are each linked to the modulator, changes in conformation alter the spacial relationship between the GFP and luciferase. The modulator can be a single entity covalently attached to one or both of the luciferase and GFP; it can be two separate entities each linked to either the luciferase or GFP. The modulator(s), GFP and luciferase can be a single fusion protein, or a fusion protein of at least two of the entities. The components can be chemically linked, such as through thiol or disulfide linkages, using linkers as provided herein. The GFP and luciferase can be linked directly or via linker, which can be a chemical linkage.

As used herein, "not strictly catalytically" means that the photoprotein acts as a catalyst to promote the oxidation of the substrate, but it is changed in the reaction, since the bound substrate is oxidized and bound molecular oxygen is used in the reaction. Such photoproteins are regenerated by addition of the substrate and molecular oxygen under appropriate conditions known to those of skill in this art.

As used herein, "nucleic acid" refers to a polynucleotide containing at least two covalently linked nucleotide or

nucleotide analog subunits. A nucleic acid can be a deoxyribonucleic acid (DNA), a ribonucleic acid (RNA), or an analog of DNA or RNA. Nucleotide analogs are commercially available and methods of preparing polynucleotides containing such nucleotide analogs are known (Lin *et al*. (1994) *Nucl. Acids Res. 22*:5220-5234; Jellinek *et al.* (1995) *Biochemistry 34*:11363-11372; Pagratis *et al*. (1997) *Nature Biotechnol. 15*:68-73). The nucleic acid can be single-stranded, double-stranded, or a mixture thereof. For purposes herein, unless specified otherwise, the nucleic acid is double-stranded, or it is apparent from the context.

As used herein, a second messenger includes, but are not limited to, cAMP, cGMP, inositol phosphates, such as IP2 and IP3, NO (nitric oxide), $Ca^{2+}$, ceramide; DAG and arachidonic acid.

Hence, the term "nucleic acid" refers to single-stranded and/or double-stranded polynucleotides, such as deoxyribonucleic acid (DNA) and ribonucleic acid (RNA), as well as analogs or derivatives of either RNA or DNA. Also included in the term "nucleic acid" are analogs of nucleic acids such as peptide nucleic acid (PNA), phosphorothioate DNA, and other such analogs and derivatives.

As used herein, the term "nucleic acid molecule" and "nucleic acid fragment" are used interchangeably.

As used herein, DNA is meant to include all types and sizes of DNA molecules including cDNA, plasmids and DNA including modified nucleotides and nucleotide analogs.

As used herein, nucleotides include nucleoside mono-, di-, and triphosphates. Nucleotides also include modified nucleotides, such as, but are not limited to, phosphorothioate nucleotides and deazapurine nucleotides and other nucleotide analogs.

As used herein, a nucleic acid probe is single-stranded DNA or RNA that has a sequence of nucleotides that includes at least 14 contiguous bases, preferably at least 16 contiguous bases, typically about 30, that are the same as (or the complement of) any 14 or more contiguous bases set forth in any of SEQ ID No. and herein. Among the preferred regions from which to construct probes include 5' and/or 3' coding sequences, sequences predicted to encode regions that are conserved among *Renilla* species. Probes from regions conserved among *Renilla* species GFPs are for isolating GFP-encoding nucleic acid from *Renilla* libraries.

In preferred embodiments, the nucleic acid probes are degenerate probes of at least 14 nucleotides, preferably 16 to 30 nucleotides, are provided.

In preferred embodiments, the nucleic acid probes are degenerate probes of at least 14 nucleotides, preferably 16 to 30 nucleotides, that are based on amino acids of *Renilla reniformis* set forth in above.

As used herein, vector (or plasmid) refers to discrete elements that are used to introduce heterologous DNA into cells for either expression or replication thereof. Selection and use of such vehicles are well within the skill of the artisan. An expression vector includes vectors capable of expressing DNA operatively linked with regulatory sequences, such as promoter regions, that are capable of effecting expression of such DNA molecules. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome. Presently preferred plasmids for expression of *Gaussia* luciferase, *Renilla* GFP and luciferase are those that are expressed in bacteria and yeast, such as those described herein.

As used herein, a promoter region or promoter element refers to a segment of DNA or RNA that controls transcription of the DNA or RNA to which it is operatively linked. The promoter region includes specific sequences that are sufficient for RNA polymerase recognition, binding and transcription initiation. This portion of the promoter region is referred to as the promoter. In addition, the promoter region includes sequences that modulate this recognition, binding and transcription initiation activity of RNA polymerase. These sequences may be cis acting or may be responsive to *trans* acting factors. Promoters, depending upon the nature of the regulation, may be constitutive or regulated. Exemplary promoters contemplated for use in prokaryotes include the bacteriophage T7 and T3 promoters, and the like.

As used herein, operatively linked or operationally associated refers to the functional relationship of DNA with regulatory and effector sequences of nucleotides, such as promoters, enhancers, transcriptional and translational stop sites, and other signal sequences. For example, operative linkage of DNA to a promoter refers to the physical and functional relationship between the DNA and the promoter such that the transcription of such DNA is initiated from the promoter by an RNA polymerase that specifically recognizes, binds to and transcribes the DNA. In order to optimize expression and/or *in vitro* transcription, it may be necessary to remove, add or alter 5' untranslated portions of the clones to eliminate extra, potential inappropriate alternative translation initiation (i.e., start) codons or other sequences that may interfere with or reduce expression, either at the level of transcription or translation. Alternatively, consensus ribosome binding sites (see, *e.g.,* Kozak (1991) *J. Biol. Chem. 266*:19867-19870) can be inserted immediately 5' of the start codon and may enhance expression. The desirability of (or need for) such modification may be empirically determined.

As used herein, to target a targeted agent, such as a luciferase, means to direct it to a cell that expresses a selected receptor or other cell surface protein by linking the agent to a such agent. Upon binding to or interaction with the receptor or cell surface protein the targeted agent, can be reacted with an appropriate substrate and activating

agents, whereby bioluminescent light is produced and the tumorous tissue or cells distinguished from non-tumorous tissue.

As used herein, an effective amount of a compound for treating a particular disease is an amount that is sufficient to ameliorate, or in some manner reduce the symptoms associated with the disease. Such amount may be administered as a single dosage or may be administered according to a regimen, whereby it is effective. The amount may cure the disease but, typically, is administered in order to ameliorate the symptoms of the disease. Repeated administration may be required to achieve the desired amelioration of symptoms.

As used herein, an effective amount of a conjugate for diagnosing a disease is an amount that will result in a detectable tissue. The tissues are detected by visualization either without aid from a detector more sensitive than the human eye, or with the use of a light source to excite any fluorescent products.

As used herein, visualizable means detectable by eye, particularly during surgery under normal surgical conditions, or, if necessary, slightly dimmed light.

As used herein, pharmaceutically acceptable salts, esters or other derivatives of the conjugates include any salts, esters or derivatives that may be readily prepared by those of skill in this art using known methods for such derivatization and that produce compounds that may be administered to animals or humans without substantial toxic effects and that either are pharmaceutically active or are prodrugs.

As used herein, treatment means any manner in which the symptoms of a conditions, disorder or disease are ameliorated or otherwise beneficially altered. Treatment also encompasses any pharmaceutical use of the compositions herein.

As used herein, amelioration of the symptoms of a particular disorder by administration of a particular pharmaceutical composition refers to any lessening, whether permanent or temporary, lasting or transient that can be attributed to or associated with administration of the composition.

As used herein, substantially pure means sufficiently homogeneous to appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers or isomers. In such instances, further purification might increase the specific activity of the compound.

As used herein, a prodrug is a compound that, upon *in vivo* administration, is metabolized or otherwise converted to the biologically, pharmaceutically or therapeutically active form of the compound. To produce a prodrug, the pharmaceutically active compound is modified such that the active compound will be regenerated by metabolic processes. The prodrug may be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo*, those of skill in this art, once a pharmaceutically active compound is known, can design prodrugs of the compound (see, *e.g.*, Nogrady (1985) *Medicinal Chemistry A Biochemical Approach,* Oxford University Press, New York, pages 388-392).

As used herein, biological activity refers to the *in vivo* activities of a compound or physiological responses that result upon *in vivo* administration of a compound, composition or other mixture. Biological activity, thus, encompasses therapeutic effects and pharmaceutical activity of such compounds, compositions and mixtures. Biological activities may be observed in *in vitro* systems designed to test or use such activities. Thus, for purposes herein the biological activity of a luciferase is its oxygenase activity whereby, upon oxidation of a substrate, light is produced.

As used herein, targeting agent refers to an agent that specifically or preferentially targets a linked targeted agent, a luciferin or luciferase, to a neoplastic cell or tissue.

As used herein, tumor antigen refers to a cell surface protein expressed or located on the surface of tumor cells.

As used herein, neoplastic cells include any type of transformed or altered cell that exhibits characteristics typical of transformed cells, such as a lack of contact inhibition and the acquisition of tumor-specific antigens. Such cells include, but are not limited to leukemic cells and cells derived from a tumor.

As used herein, neoplastic disease is any disease in which neoplastic cells are present in the individual afflicted with the disease. Such diseases include, any disease characterized as cancer.

As used herein, metastatic tumors refers to tumors that are not localized in one site.

As used herein, specialty tissue refers to non-tumorous tissue for which information regarding location is desired. Such tissues include, for example, endometriotic tissue, ectopic pregnancies, tissues associated with certain disorders and myopathies or pathologies.

As used herein, a receptor refers to a molecule that has an affinity for a given ligand. Receptors may be naturally-occurring or synthetic molecules. Receptors may also be referred to in the art as anti-ligands. As used herein, the receptor and anti-ligand are interchangeable. Receptors can be used in their unaltered state or as aggregates with

other species. Receptors may be attached, covalently or noncovalently, or in physical contact with, to a binding member, either directly or indirectly via a specific binding substance or linker. Examples of receptors, include, but are not limited to: antibodies, cell membrane receptors surface receptors and internalizing receptors, monoclonal antibodies and antisera reactive with specific antigenic determinants (such as on viruses, cells, or other materials), drugs, polynucleotides, nucleic acids, peptides, cofactors, lectins, sugars, polysaccharides, cells, cellular membranes, and organelles.

Examples of receptors and applications using such receptors, include but are not restricted to:

a) enzymes: specific transport proteins or enzymes essential to survival of microorganisms, which could serve as targets for antibiotic (ligand) selection;

b) antibodies: identification of a ligand-binding site on the antibody molecule that combines with the epitope of an antigen of interest may be investigated; determination of a sequence that mimics an antigenic epitope may lead to the development of vaccines of which the immunogen is based on one or more of such sequences or lead to the development of related diagnostic agents or compounds useful in therapeutic treatments such as for auto-immune diseases

c) nucleic acids: identification of ligand, such as protein or RNA, binding sites;

d) catalytic polypeptides: polymers, preferably polypeptides, that are capable of promoting a chemical reaction involving the conversion of one or more reactants to one or more products; such polypeptides generally include a binding site specific for at least one reactant or reaction intermediate and an active functionality proximate to the binding site, in which the functionality is capable of chemically modifying the bound reactant (see, *e.g.*, U.S. Patent No. 5,215,899);

e) hormone receptors: determination of the ligands that bind with high affinity to a receptor is useful in the development of hormone replacement therapies; for example, identification of ligands that bind to such receptors may lead to the development of drugs to control blood pressure; and

f) opiate receptors: determination of ligands that bind to the opiate receptors in the brain is useful in the development of less-addictive replacements for morphine and related drugs.

As used herein, antibody includes antibody fragments, such as Fab fragments, which are composed of a light chain and the variable region of a heavy chain.

As used herein, an antibody conjugate refers to a conjugate in which the targeting agent is an antibody.

As used herein, antibody activation refers to the process whereby activated antibodies are produced. Antibodies are activated upon reaction with a linker, such as heterobifunctional reagent.

As used herein, a surgical viewing refers to any procedure in which an opening is made in the body of an animal. Such procedures include traditional surgeries and diagnostic procedures, such as laparoscopies and arthroscopic procedures.

As used herein, humanized antibodies refer to antibodies that are modified to include "human" sequences of amino acids so that administration to a human will not provoke an immune response. Methods for preparation of such antibodies are known. For example, the hybridoma that expresses the monoclonal antibody is altered by recombinant DNA techniques to express an antibody in which the amino acid composition of the non-variable regions is based on human antibodies. Computer programs have been designed to identify such regions.

As used herein, ATP, AMP, NAD+ and NADH refer to adenosine triphosphate, adenosine monophosphate, nicotinamide adenine dinucleotide (oxidized form) and nicotinamide adenine dinucleotide (reduced form), respectively.

As used herein, production by recombinant means by using recombinant DNA methods means the use of the well known methods of molecular biology for expressing proteins encoded by cloned DNA.

As used herein, substantially identical to a product means sufficiently similar so that the property of interest is sufficiently unchanged so that the substantially identical product can be used in place of the product.

As used herein equivalent, when referring to two sequences of nucleic acids means that the two sequences in question encode the same sequence of amino acids or equivalent proteins. When "equivalent" is used in referring to two proteins or peptides, it means that the two proteins or peptides have substantially the same amino acid sequence with only conservative amino acid substitutions (see, *e.g.,* Table 1, above) that do not substantially alter the activity or function of the protein or peptide. When "equivalent" refers to a property, the property does not need to be present to the same extent (e.g., two peptides can exhibit different rates of the same type of enzymatic activity), but the activities are preferably substantially the same. "Complementary," when referring to two nucleotide sequences, means that the two sequences of nucleotides are capable of hybridizing, preferably with less than 25%, more preferably with less than 15%, even more preferably with less than 5%, most preferably with no mismatches between opposed nucleotides. Preferably the two molecules will hybridize under conditions of high stringency.

As used herein: stringency of hybridization in determining percentage mismatch is as follows:

1) high stringency: 0.1 x SSPE, 0.1% SDS, 65°C

2) medium stringency: 0.2 x SSPE, 0.1% SDS, 50°C
3) low stringency: 1.0 x SSPE, 0.1% SDS, 50°C

It is understood that equivalent stringencies may be achieved using alternative buffers, salts and temperatures.

The term "substantially" identical or homologous or similar varies with the context as understood by those skilled in the relevant art and generally means at least 70%, preferably means at least 80%, more preferably at least 90%, and most preferably at least 95% identity. The terms "homology" and "identity" are often used interchangeably. In general, sequences are aligned so that the highest order match is obtained (see, *e.g.*: *Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data, Part I,* Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991; Carillo *et al.* (1988) *SIAM J Applied Math 48*:1073). By sequence identity, the number of conserved amino acids are determined by standard alignment algorithms programs, and are used with default gap penalties established by each supplier. Substantially homologous nucleic acid molecules would hybridize typically at moderate stringency or at high stringency all along the length of the nucleic acid of interest. Also contemplated are nucleic acid molecules that contain degenerate codons in place of codons in the hybridizing nucleic acid molecule.

Whether any two nucleic acid molecules have nucleotide sequences that are at least 80%, 85%, 90%, 95%, 96%, 97%, 98% or 99% "identical" can be determined using known computer algorithms such as the "FAST A" program, using for example, the default parameters as in Pearson *et al.* (1988) *Proc. Natl. Acad. Sci. USA 85*:2444 (other programs include the GCG program package (Devereux, J., *et al., Nucleic Acids Research 12(I)*:387 (1984)), BLASTP, BLASTN, FASTA (Atschul, S.F., *et al., J Molec Biol 215*:403 (1990); Guide to Huge Computers, Martin J. Bishop, ed., Academic Press, San Diego, 1994, and Carillo *et al.* (1988) *SIAM J Applied Math 48*:1073). For example, the BLAST function of the National Center for Biotechnology Information database may be used to determine identity. Other commercially or publicly available programs include, DNAStar "MegAlign" program (Madison, WI) and the University of Wisconsin Genetics Computer Group (UWG) "Gap" program (Madison WI)). Percent homology or identity of proteins and/or nucleic acid moleucles may be determined, for example, by comparing sequence information using a GAP computer program (*e.g.,* Needleman *et al.* (1970) *J. Mol. Biol.* 48:443, as revised by Smith and Waterman ((1981) *Adv. Appl. Math.* 2:482). Briefly, the GAP program defines similarity as the number of aligned symbols (i.e., nucleotides or amino acids) which are similar, divided by the total number of symbols in the shorter of the two sequences. Default parameters for the GAP program may include: (1) a unary comparison matrix (containing a value of 1 for identities and 0 for non-identities) and the weighted comparison matrix of Gribskov *et al.* (1986) *Nucl. Acids Res.* 14:6745, as described by Schwartz and Dayhoff, eds., *ATLAS OF PROTEIN SEQUENCE AND STRUCTURE,* National Biomedical Research Foundation, pp. 353-358 (1979); (2) a penalty of 3.0 for each gap and an additional 0.10 penalty for each symbol in each gap; and (3) no penalty for end gaps.

Therefore, as used herein, the term "identity" represents a comparison between a test and a reference polypeptide or polynucleotide. For example, a test polypeptide may be defined as any polypeptide that is 90% or more identical to a reference polypeptide. As used herein, the term at least "90% identical to" refers to percent identities from 90 to 99.99 relative to the reference polypeptides. Identity at a level of 90% or more is indicative of the fact that, assuming for exemplification purposes a test and reference polynucleotide length of 100 amino acids are compared. No more than 10% (i.e., 10 out of 100) amino acids in the test polypeptide differs from that of the reference polypeptides. Similar comparisons may be made between a test and reference polynucleotides. Such differences may be represented as point mutations randomly distributed over the entire length of an amino acid sequence or they may be clustered in one or more locations of varying length up to the maximum allowable, e.g. 10/100 amino acid difference (approximately 90% identity). Differences are defined as nucleic acid or amino acid substitutions, or deletions. At level of homologies or identities above about 85-90%, the result should be independent of the program and gap parameters set; such high levels of identity readily can be assess, often without relying on software.

As used herein, primer refers to an oligonucleotide containing two or more deoxyribonucleotides or ribonucleotides, preferably more than three, from which synthesis of a primer extension product can be initiated. Experimental conditions conducive to synthesis include the presence of nucleoside triphosphates and an agent for polymerization and extension, such as DNA polymerase, and a suitable buffer, temperature and pH.

As used herein, a composition refers to any mixture. It may be a solution, a suspension, liquid, powder, a paste, aqueous, non-aqueous or any combination thereof.

As used herein, a combination refers to any association between two or among more items.

As used herein, fluid refers to any composition that can flow. Fluids thus encompass compositions that are in the form of semi-solids, pastes, solutions, aqueous mixtures, gels, lotions, creams and other such compositions.

Examples of receptors and applications using such receptors, include but are not restricted to:

a) enzymes: specific transport proteins or enzymes essential to survival of microorganisms, which could serve as targets for antibiotic (ligand) selection;

b) antibodies: identification of a ligand-binding site on the antibody molecule that combines with the epitope of an antigen of interest may be investigated; determination of a sequence that mimics an antigenic epitope may lead to the development of vaccines of which the immunogen is based on one or more of such sequences or lead to the development of related diagnostic agents or compounds useful in therapeutic treatments such as for auto-immune diseases

c) nucleic acids: identification of ligand, such as protein or RNA, binding sites;

d) catalytic polypeptides: polymers, preferably polypeptides, that are capable of promoting a chemical reaction involving the conversion of one or more reactants to one or more products; such polypeptides generally include a binding site specific for at least one reactant or reaction intermediate and an active functionality proximate to the binding site, in which the functionality is capable of chemically modifying the bound reactant (see, *e.g.*, U.S. Patent No. 5,215,899);

e) hormone receptors: determination of the ligands that bind with high affinity to a receptor is useful in the development of hormone replacement therapies; for example, identification of ligands that bind to such receptors may lead to the development of drugs to control blood pressure; and

f) opiate receptors: determination of ligands that bind to the opiate receptors in the brain is useful in the development of less-addictive replacements for morphine and related drugs.

As used herein, complementary refers to the topological compatibility or matching together of interacting surfaces of a ligand molecule and its receptor. Thus, the receptor and its ligand can be described as complementary, and furthermore, the contact surface characteristics are complementary to each other.

As used herein, a ligand-receptor pair or complex formed when two macromolecules have combined through molecular recognition to form a complex.

As used herein, a substrate refers to any matrix that is used either directly or following suitable derivatization, as a solid support for chemical synthesis, assays and other such processes. Preferred substrates herein, are silicon substrates or siliconized substrates that are derivitized on the surface intended for linkage of anti-ligands and ligands and other macromolecules, including the fluorescent proteins, phycobiliproteins and other emission shifters.

As used herein, a matrix refers to any solid or semisolid or insoluble support on which the molecule of interest, typically a biological molecule, macromolecule, organic molecule or biospecific ligand is linked or contacted. Typically a matrix is a substrate material having a rigid or semi-rigid surface. In many embodiments, at least one surface of the substrate will be substantially flat, although in some embodiments it may be desirable to physically separate synthesis regions for different polymers with, for example, wells, raised regions, etched trenches, or other such topology. Matrix materials include any materials that are used as affinity matrices or supports for chemical and biological molecule syntheses and analyses, such as, but are not limited to: polystyrene, polyearbonate, polypropylene, nylon, glass, dextran, chitin, sand, pumice, polytetrafluoroethylene, agarose, polysaccharides, dendrimers, buckyballs, polyacrylamide, Kieselguhr-polyacrylamide non-covalent composite, polystyrene-polyacrylamide covalent composite, polystyrene-PEG (polyethyleneglycol) composite, silicon, rubber, and other materials used as supports for solid phase syntheses, affinity separations and purifications, hybridization reactions, immunoassays and other such applications.

As used herein, the attachment layer refers the surface of the chip device to which molecules are linked. Typically, the chip is a semiconductor device, which is coated on a least a portion of the surface to render it suitable for linking molecules and inert to any reactions to which the device is exposed. Molecules are linked either directly or indirectly to the surface, linkage may be effected by absorption or adsorption, through covalent bonds, ionic interactions or any other interaction. Where necessary the attachment layer is adapted, such as by derivatization for linking the molecules.

## B. FLUORESCENT PROTEINS

The GFP from *Aequorea* and that of the sea pansy *Renilla reniformis* share the same chromophore, yet Aequorea GFP has two absorbance peaks at 395 and 475 nm, whereas Renilla GFP has only a single absorbance peak at 498 nm, with about 5.5 fold greater monomer extinction coefficient than the major 395 nm peak of the Aequorea protein (Ward, W. W. in Biohtminescence and Chemiluminescence (eds. DeLuca, M. A. & McElroy, W. D.) 235-242 (Academic Press, New York, 1981)). The spectra of the isolated chromophore and denatured protein at neutral pH do not match the spectra of either native protein (Cody, C. W. *et al.* (1993) *Biochemistry 32*:1212-1218).

### 1. Green and blue fluorescent proteins

As described herein, blue light is produced using the *Renilla* luciferase or the *Aequorea* photoprotein in the

presence of $Ca^{2+}$ and the coelenterazine luciferin or analog thereof. This light can be converted into a green light if a green fluorescent protein (GFP) is added to the reaction. Green fluorescent proteins, which have been purified (see, *e.g.,* Prasher *et al.* (1992) *Gene 111*:229-233) and also cloned (see, *e.g.*, International PCT Application No. WO 95/07463, which is based on U.S. application Serial No. 08/119,678 and U.S. application Serial No. 08/192,274, which are herein incorporated by reference), are used by cnidarians as energy-transfer acceptors. GFPs fluoresce *in vivo* upon receiving energy from a luciferase-oxyluciferein excited-state complex or a $Ca^{2+}$-activated photoprotein. The chromophore is modified amino acid residues within the polypeptide. The best characterized GFPs are those of *Aequorea* and *Renilla* (see, *e.g.*, Prasher *et al.* (1992) *Gene 111*:229-233; Hart, *et al.* (1979) *Biochemistry 18*:2204-2210). For example, a green fluorescent protein (GFP) from *Aequorea victoria* contains 238 amino acids, absorbs blue light and emits green light. Thus, inclusion of this protein in a composition containing the aequorin photoprotein charged with coelenterazine and oxygen, can, in the presence of calcium, result in the production of green light. Thus, it is contemplated that GFPs may be included in the bioluminescence generating reactions that employ the aequorin or-*Renilla* luciferases or other suitable luciferase in order to enhance or alter color of the resulting bioluminescence.

## 2. *Renilla reniformis* GFP

Purified *Renilla reniformis* GFP and muteins thereof are provided. Presently preferred *Renilla* GFP for use in the compositions herein is *Renilla reniformis* GFP having the sequence of amino acids set forth in SEQ ID No. 27. The *Renilla* GFP and GFP peptides can be isolated from natural sources or isolated from a prokaryotic or eukaryotic cell transfected with nucleic acid that encodes the *Renilla* GFP and/or GFP peptides, such as those encoded by the sequences of nucleotides set forth in SEQ ID Nos. 23-25.

The encoding nucleic acid molecules are provided. Preferred are those that encode the protein having the sequence of amino acids (SEQ ID No. 27):

mdlaklglkevmptkinleglvgdhafsmegvgegnilegtqevkisvtkgaplpfafdivsvafsygn
raytgypeeisdyflqsfpegftyerniryqdggtaivksdisledgkfivnvdfkakdlrrmgpvmqqd
ivgmqpsyesmytnvtsvigeciiafklqtgkhftyhmrtvykskkpvetmplyhfiqhrlvktnvdta
sgyvvqhetaiaahstikkiegslp,

and is preferably the sequence set forth in SEQ ID No. 26.

In particular, nucleic acid molecules encoding a *Renilla reniformis* GFP having any of the following sequences are provided (see SEQ ID Nos. 23-25):

Renilla renformis GFP Clone-1

GGCACGAGGGTTTCCTGACACAATAAAAACCTTTCAAATTGTTTCTC
TGTAGCAGTAAGTATGGATCTCGCAAAACTTGGTTTGAAGGAAGTG
ATGCCTACTAAAATCAACTTAGAAGGACTGGTTGGCGACCACGCTT
TCTCAATGGAAGGAGTTGGCGAAGGCAACATATTGGAAGGAACTCA
AGAGGTGAAGATATCGGTAACAAAAGGCGCACCACTCCCATTCGC
ATTTGATATCGTATCTGTGGCTTTTTCATATGGGAACAGAGCTTA
TACCGGTTACCCAGAAGAAATTTCCGACTACTTCCTCCAGTCGTT
TCCAGAAGGCTTTACTTACGAGAGAAACATTCGTTATCAAGATGG
AGGAACTGCAATTGTTAAATCTGATATAAGCTTGGAAGATGGTAA
ATTCATAGTGAATGTAGACTTCAAAGCGAAGGATCTACGTCGCAT
GGGACCAGTCATGCAGCAAGACATCGTGGGTATGCAGCCATCGTA
TGAGTCAATGTACACCAATGTCACTTCAGTTATAGGGGAATGTAT
AATAGCATTCAAACTTCAAACTGGCAAGCATTTCACTTACCACAT
GAGGACAGTTTACAAATCAAAGAAGCCAGTGGAAACTATGCCATTG
TATCATTTCATCCAGCATCGCCTCGTTAAGACCAATGTGGACACAG
CCAGTGGTTACGTTGTGCAACACGAGACAGCAATTGCAGCGCATTC
TACAATCAAAAAAATTGAAGGCTCTTTACCATAGATACCTGTACACA

ATTATTCTATGCACGTAGCATTTTTTTGGAAATATAAGTGGTATTGT
TCAATAAAATATTAAATATAAAAAAAAAAAAAAAAAAAAAAA;

Renilla renformis GFP Clone-2

GGCACGAGGCTGACACAATAAAAAACCTTTCAAATTGTTTCTCTGTAGCAGG
AAGTATGGATCTCGCAAAACTTGGTTTGAAGGAAGTGATGCCTACTAAAATC
AACTTAGAAGGACTGGTTGGCGACCACGCTTTCTCAATGGAAGGAGTTGGCG
AAGGCAACATATTGGAAGGAACTCAAGAGGTGAAGATATCGGTAACAAAAGG
CGCACCACTCCCATTCGCATTTGATATCGTATCTGTTGCTTTCTCATATGGG
AACAGAGCTTATACTGGTTACCCAGAAGAAATTTCCGACTACTTCCTCCAGT
CGTTTCCAGAAGGCTTTACTTACGAGAGAAACATTCGTTATCAAGATGGAGG
AACTGCAATTGTTAAATCTGATATAAGCTTGGAAGATGGTAAATTCATAGTG
AATGTAGACTTCAAAGCGAAGGATCTACGTCGCATGGGACCAGTCATGCAGC
AAGACATCGTGGGTATGCAGCCATCGTATGAGTCAATGTACACCAATGTCAC
TTCAGTTATAGGGGAATGTATAATAGCATTCAAACTTCAAACTGGCAAACAT
TTCACTTACCACATGAGGACAGTTTACAAATCAAAGAAGCCAGTGGAAACTA
TGCCATTGTATCATTTCATCCAGCATCGCCTCGTTAAGACCAATGTGGACAC
AGCCAGTGGTTACGTTGTGCAACACGAGACAGCAATTGCAGCGCATTCTACA
ATCAAAAAAATTGAAGGCTCTTTACCATAGATATCTATACACAATTATTCTA
TGCACGTAGCATTTTTTTGGAAATATAAGTGGTATTGTTCAATAAAATATTA
AATATAAAAAAAAAAAAAAAAAAAAAAA; and

Renilla renformis GFP Clone-3

GGCACGAGGGTTTCCTGACACAATAAAAACCTTTCAAATTGTTTCTCTGTAG
CAGTAAGTATGGATCTCGCAAAACTTGGTTTGAAGGAAGTGATGCCTACTA
AAATCAACTTAGAAGGACTGGTTGGCGACCACGCTTTCTCAATGGAAGGAGTT
GGCGAAGGCAACATATTGGAAGGAACTCAAGAGGTGAAGATATCGGTAACAA
AAGGCGCACCACTCCCATTCGCATTTGATATCGTATCTGTGGCTTTTTCATAT
GGGAACAGAGCTTATACCGGTTACCCAGAAGAAATTTCCGACTACTTCCTCCA
GTCGTTTCCAGAAGGCTTTACTTACGAGAGAAACATTCGTTATCAAGATGGAG
GAACTGCAATTGTTAAATCTGATATAAGCTTGGAAGATGGTAAATTCATAGT
GAATGTAGACTTCAAAGCGAAGGATCTACGTCGCATGGGACCAGTCATGCAG
CAAGACATCGTGGGTATGCAGCCATCGTATGAGTCAATGTACACCAATGTCAC
TTCAGTTATAGGGGAATGTATAATAGCATTCAAACTTCAAACTGGCAAGCATT
TCACTTACCACATGAGGACAGTTTACAAATCAAAGAAGCCAGTGGAAACTATG
CCATTGTATCATTTCATCCAGCATCGCCTCGTTAAGACCAATGTGGACACAGC
CAGTGGTTACGTTGTGCAACACGAGACAGCAATTGCAGCGCATTCTACAATCA
AAAAAATTGAAGGCTCTTTACCATAGATACCTGTACACAATTATTCTATGCAC
GTAGCATTTTTTTGGAAATATAAGTGGTATTGTTCAATAAAATATTAAATATAT
GCTTTTGCAAAAAAAAAAAAAAAAAAAAA

are provided.

An exemplary mutein is set forth in SEQ ID No. 33, and humanized codon are set forth in SEQ ID No. 26.

Also contemplated are the coding portion of the sequence of nucleotides that hybridize under moderate or high stringency to the sequence of nucleotides set forth above, particularly when using probes provided herein, are provided.

Probes derived from this nucleic acid that can be used in methods provided herein to isolated GFPs from any *Renilla reniformis* species. In an exemplary embodiment, nucleic acid encoding *Renilla reniformis* GFP is provided. This nucleic acid encodes the sequence of amino acids set forth above.

GFPs, including the *Renilla reniformis* protein provided herein, are activated by blue light to emit green light and thus may be used in the absence of luciferase and in conjunction with an external light source with novelty items (see U.S. Patent Nos. 5,876,995, 6,152,358 and 6,113,886) and in conjunction with bioluminescence generating system for novelty items (see U.S. Patent Nos. 5,876,995, 6,152,358 and 6,113,886), for tumor diagnosis (see, allowed copending U.S. application Serial No. 08/908,909) and in biochips (see, U.S. application Serial No. 08/990,103, which is published as International PCT application No. WO 98/26277).

*Renilla reniformis* GFP is intended for use in any of the novelty items and combinations, such as the foods, including beverages, greeting cards, and toys, including bubble making toys, particularly bubble-making compositions or mixtures. Also of particular interest are the use of these proteins in cosmetics, particularly face paints or make-up, hair colorants or hair conditioners, mousses or other such products and skin creams. Such systems are particularly of interest because no luciferase is needed to activate the photoprotein and because the proteins are non-toxic and safe to apply to the skin, hair, eyes and to ingest. These fluorescent proteins may also be used in addition to bioluminescence generating systems to enhance or create an array of different colors. Transgenic animals and plants that express the *Renilla reniformis* GFP-encoding nucleic acid are also provided. Such animals and plants, include transgenic fish, transgenic worms for use, for example, as lures for fishing; transgenic animals, such as monkeys and rodents for research in which a marker gene is used, and transgenic animals as novelty items and to produce glowing foods, such as ham, eggs, chicken, and other meats; transgenic plants in which the *Renilla reniformis* is a marker, and also transgenic plants that are novelty items, particularly ornamental plants, such as glowing orchids, roses and other flowering plants.

The *Renilla reniformis* GFP may be used alone or in combination with bioluminescence generating systems to produce an array of colors. They may be used in combinations such that the color of, for example, a beverage changes over time, or includes layers of different colors. The cloning and expression of *Renilla reniformis* GFP and uses thereof are described below.

## C. BIOLUMINESCENCE GENERATING SYSTEMS AND COMPONENTS

The following is a description of bioluminescence generating systems and the components thereof. The *Renilla reniformis* GFP provided herein can be used alone for a variety of applications, and with any compatible bioluminescence generating systems.

A bioluminescence-generating system refers to the components that are necessary and sufficient to generate bioluminescence. These include a luciferase, luciferin and any necessary co-factors or conditions. Virtually any bioluminescent system known to those of skill in the art will be amenable to use in the apparatus, systems, combinations and methods provided herein. Factors for consideration in selecting a bioluminescent-generating system, include, but are not limited to: the targeting agent used in combination with the bioluminescence; the medium in which the reaction is run; stability of the components, such as temperature or pH sensitivity; shelf life of the components; sustainability of the light emission, whether constant or intermittent; availability of components; desired light intensity; color of the light; and other such factors. Such bioluminescence generating systems are known (see those described in U.S. Patent Nos. 5,876,995, 6,152,358 and 6,113,886).

### 1. General description

In general, bioluminescence refers to an energy-yielding chemical reaction in which a specific chemical substrate, a luciferin, undergoes oxidation, catalyzed by an enzyme, a luciferase. Bioluminescent reactions are easily maintained, requiring only replenishment of exhausted luciferin or other substrate or cofactor or other protein, in order to continue or revive the reaction. Bioluminescence generating reactions are well-known to those of skill in this art and any such reaction may be adapted for use in combination with articles of manufacture as described herein.

There are numerous organisms and sources of bioluminescence generating systems, and some representative genera and species that exhibit bioluminescence are set forth in the following table (reproduced in part from Hastings in (1995) *Cell Physiology:Source Book,* N. Sperelakis (ed.), Academic Press, pp 665-681):

**TABLE 2 Representative luminous organism**

| Type of Organism | Representative genera |
|---|---|
| Bacteria | Photobacterium<br>Vibrio |

Table continued

| Type of Organism | Representative genera |
|---|---|
| | Xenorhabdus |
| Mushrooms | Panus, Armillaria<br>Pleurotus |
| Dinofiagellates | Gonyaulax<br>Pyrocystis<br>Noctiluca |
| Cnidaria (coelenterates)<br>Jellyfish<br>Hydroid<br>Sea Pansy | <br>Aequorea<br>Obelia<br>Renilla |
| Ctenophores | Mnemiopsis<br>Beroe |
| Annelids<br>Earthworms<br>Marine polychaetes<br>Syllid fireworm | <br>Diplocardia<br>Chaetopterus, Phyxotrix<br>Odontosyllis |
| Molluscs<br>Limpet<br>Clam<br>Squid | <br>Latia<br>Pholas<br>Heteroteuthis<br>Heterocarpus |
| Crustacea<br>Ostracod | <br>Vargula (Cypridina) |
| Shrimp (euphausids)<br><br><br><br>Decapod<br>Copepods | Meganyctiphanes<br>Acanthophyra<br>Oplophorus<br>Gnathophausia<br>Sergestes |
| Insects<br>Coleopterids (beetles)<br>Firefly<br>Click beetles<br>Railroad worm<br>Diptera (flies) | <br><br>Photinus, Photuris<br>Pyrophorus<br>Phengodes, Phrixothrix<br>Arachnocampa |
| Echinoderms<br>Brittle stars<br>Sea cucumbers | <br>Ophiopsila<br>Laetmogone |
| Anthozoans | Renilla |
| Chordates<br>Tunicates | <br>Pyrosoma |
| Fish<br>Cartilaginous<br>Bony<br>Ponyfish<br>Flashlight fish | <br>Squalus<br><br>Leiognathus<br>Photoblepharon |

Table continued

| Type of Organism | Representative genera |
|---|---|
| Angler fish | Cryptopsaras |
| Midshipman | Porichthys |
| Lantern fish | Benia |
| Shiny loosejaw | Aristostomias |
| Hatchet fish | Agyropelecus |
| and other fish | Pachystomias |
| | Malacosteus |
| Midwater fish | Cyclothone |
| | Neoscopelus |
| | Tarletonbeania |

Other bioluminescent organisms contemplated for use herein are *Gonadostomias, Gaussia* (copepods), *Watensia, Halisturia;* Vampire squid, *Glyphus,* Mycotophids (fish), *Vinciguerria, Howella, Florenciella, Chaudiodus, Melanocostus* and Sea Pens.

It is understood that a bioluminescence generating system may be isolated from natural sources, such as those in the above Table, or may be produced synthetically. In addition, for uses herein, the components need only be sufficiently pure so that mixture thereof, under appropriate reaction conditions, produces a glow so that cells and tissues can be visualized during a surgical procedure.

Thus, in some embodiments, a crude extract or merely grinding up the organism may be adequate. Generally, however, substantially pure components are used. Also, components may be synthetic components that are not isolated from natural sources. DNA encoding luciferases is available (see, *e.g.,* SEQ ID Nos. 1-13) and has been modified (see, e.g., SEQ ID Nos. 3 and 10-13) and synthetic and alternative substrates have been devised. The DNA listed herein is only representative of the DNA encoding luciferases that is available.

Any bioluminescence generating system, whether synthetic or isolated form natural sources, such as those set forth in Table 2, elsewhere herein or known to those of skill in the art, is intended for use in the combinations, systems and methods provided herein. Chemiluminescence systems per se, which do not rely on oxygenases (luciferases) are not encompassed herein.

**(a) Luciferases**

Luciferases refer to any compound that, in the presence of any necessary activators, catalyze the oxidation of a bioluminescence substrate (luciferin) in the presence of molecular oxygen, whether free or bound, from a lower energy state to a higher energy state such that the substrate, upon return to the lower energy state, emits light. For purposes herein, luciferase is broadly used to encompass enzymes that act catalytically to generate light by oxidation of a substrate and also photoproteins, such as aequorin, that act, though not strictly catalytically (since such proteins are exhausted in the reaction), in conjunction with a substrate in the presence of oxygen to generate light. These luciferases, including photoproteins; such as aequorin, are herein also included among the luciferases. These reagents include the naturally-occurring luciferases (including photoproteins), proteins produced by recombinant DNA, and mutated or modified variants thereof that retain the ability to generate light in the presence of an appropriate substrate, co-factors and activators or any other such protein that acts as a catalyst to oxidize a substrate, whereby light is produced.

Generically, the protein that catalyzes or initiates the bioluminescent reaction is referred to as a luciferase, and the oxidizable substrate is referred to as a luciferin. The oxidized reaction product is termed oxyluciferin, and certain luciferin precursors are termed etioluciferin. Thus, for purposes herein bioluminescence encompasses light produced by reactions that are catalyzed by (in the case of luciferases that act enzymatically) or initiated by (in the case of the photoproteins, such as aequorin, that are not regenerated in the reaction) a biological protein or analog, derivative or mutant thereof.

For clarity herein, these catalytic proteins are referred to as luciferases and include enzymes such as the luciferases that catalyze the oxidation of luciferin, emitting light and releasing oxyluciferin. Also included among luciferases are photoproteins, which catalyze the oxidation of luciferin to emit light but are changed in the reaction and must be reconstituted to be used again. The luciferases may be naturally occurring or may be modified, such as by genetic engineering to improve or alter certain properties. As long as the resulting molecule retains the ability to catalyze the bioluminescent reaction, it is encompassed herein.

Any protein that has luciferase activity (a protein that catalyzes oxidation of a substrate in the presence of molecular oxygen to produce light as defined herein) may be used herein. The preferred luciferases are those that are

described herein or that have minor sequence variations. Such minor sequence variations include, but are not limited to, minor allelic or species variations and insertions or deletions of residues, particularly cysteine residues. Suitable conservative substitutions of amino acids are known to those of skill in this art and may be made generally without altering the biological activity of the resulting molecule. Such substitutions are preferably made in accordance with those set forth in TABLE 1 as described above.

The luciferases may be obtained commercially, isolated from natural sources, expressed in host cells using DNA encoding the luciferase, or obtained in any manner known to those of skill in the art. For purposes herein, crude extracts obtained by grinding up selected source organisms may suffice. Since large quantities of the luciferase may be desired, isolation of the luciferase from host cells is preferred. DNA for such purposes is widely available as are modified forms thereof.

Examples of luciferases include, but are not limited to, those isolated from the ctenophores *Mnemiopsis* (mnemiopsin) and *Beroe ovata* (berovin), those isolated from the coelenterates *Aequorea* (aequorin), *Obelia* (obelin), *Pelagia,* the *Renilla* luciferase, the luciferases isolated from the mollusca *Pholas* (pholasin), the luciferases isolated from fish, such as *Aristostomias, Pachystomias* and *Porichthys* and from the ostracods, such as *Cypridina* (also referred to as *Vargula*). Preferred luciferases for use herein are the Aequorin protein, *Renilla* luciferase and *Cypridina* (also called *Vargula*) luciferase (see, *e.g.*, SEQ ID Nos. 1, 2, and 4-13). Also, preferred are luciferases which react to produce red and/or near infrared light. These include luciferases found in species of *Aristostomias,* such as *A. scintillans, Pachystomias, Malacosteus,* such as *M. niger.*

### (b) Luciferins

The substrates for the reaction or for inclusion in the conjugates include any molecule(s) with which the luciferase reacts to produce light. Such molecules include the naturally-occurring substrates, modified forms thereof, and synthetic substrates (see, *e.g.*, U.S. Patent Nos. 5,374,534 and 5,098,828). Exemplary luciferins include those described herein, as well as derivatives thereof, analogs thereof, synthetic substrates, such as dioxetanes (see, e.g., U.S. Patent Nos. 5,004,565 and 5,455,357), and other compounds that are oxidized by a luciferase in a light-producing reaction (see, *e.g.,* U.S. Patent Nos. 5,374,534, 5,098,828 and 4,950,588). Such substrates also may be identified empirically by selecting compounds that are oxidized in bioluminescent reactions.

### (c) Activators

The bioluminescent generating systems also require additional components discussed herein and known to those of skill in the art. All bioluminescent reactions require molecular oxygen in the form of dissolved or bound oxygen. Thus, molecular oxygen, dissolved in water or in air or bound to a photoprotein, is the activator for bioluminescence reactions. Depending upon the form of the components, other activators include, but are not limited to, ATP (for firefly luciferase), flavin reductase (bacterial systems) for regenerating $FMNH_2$ from FMN, and $Ca^{2+}$ or other suitable metal ion (aequorin).

Most of the systems provided herein will generate light when the luciferase and luciferin are mixed and exposed to air or water. The systems that use photoproteins that have bound oxygen, such as aequorin, however, will require exposure to $Ca^{2+}$ (or other suitable metal ion), which can be provided in the form of an aqueous composition of a calcium salt. In these instances, addition of a $Ca^{2+}$ (or other suitable metal ion) to a mixture of luciferase (aequorin) and luciferin (such as coelenterazine) will result in generation of light. The *Renilla* system and other Anthozoa systems also require $Ca^{2+}$ (or other suitable metal ion).

If crude preparations are used, such as ground up *Cypridina* (shrimp) or ground fireflies, it may be necessary to add only water. In instances in which fireflies (or a firefly or beetle luciferase) are used the reaction may only require addition ATP. The precise components will be apparent, in light of the disclosure herein, to those of skill in this art or may be readily determined empirically.

It is also understood that these mixtures will also contain any additional salts or buffers or ions that are necessary for each reaction to proceed. Since these reactions are well-characterized, those of skill in the art will be able to determine precise proportions and requisite components. Selection of components will depend upon the apparatus, article of manufacture and luciferase. Various embodiments are described and exemplified herein; in view of such description, other embodiments will be apparent.

### (d) Reactions

In all embodiments, all but one component, either the luciferase or luciferin, of a bioluminescence generating system will be mixed or packaged with or otherwise combined. Since the result to be achieved is the production of light visible to the naked eye for qualitative, not quantitative, diagnostic purposes, the precise proportions and amounts of

components of the bioluminescence reaction need not be stringently determined or met. They must be sufficient to produce light. Generally, an amount of luciferin and luciferase sufficient to generate a visible glow is used; this amount can be readily determined empirically and is dependent upon the selected system and selected application. Where quantitative measurements are required, more precision may be required.

For purposes herein, such amount is preferably at least the concentrations and proportions used for analytical purposes by those of skill in the such arts. Higher concentrations may be used if the glow is not sufficiently bright. Alternatively, a microcarrier coupled to more than one luciferase molecule linked to a targeting agent may be utilized to increase signal output. Also because the conditions in which the reactions are used are not laboratory conditions and the components are subject to storage, higher concentration may be used to overcome any loss of activity. Typically, the amounts are 1 mg, preferably 10 mg and more preferably 100 mg, of a luciferase per liter of reaction mixture or 1 mg, preferably 10 mg, more preferably 100 mg. Compositions may contain at least about 0.01 mg/l, and typically 0.1 mg/l, 1 mg/l, 10 mg/l or more of each component on the item. The amount of luciferin is also between about 0.01 and 100 mg/l, preferably between 0.1 and 10 mg/l, additional luciferin can be added to many of the reactions to continue the reaction. In embodiments in which the luciferase acts catalytically and does not need to be regenerated, lower amounts of luciferase can be used. In those in which it is changed during the reaction, it also can be replenished; typically higher concentrations will be selected. Ranges of concentration per liter (or the amount of coating on substrate the results from contacting with such composition) of each component on the order of 0.1 to 20 mg, preferably 0.1 to 10 mg, more preferably between about 1 and 10 mg of each component will be sufficient. When preparing coated substrates, as described herein, greater amounts of coating compositions containing higher concentrations of the luciferase or luciferin may be used.

Thus, for example, in presence of calcium, 5 mg of luciferin, such as coelenterazine, in one liter of water will glow brightly for at least about 10 to 20 minutes, depending on the temperature of the water, when about 10 mgs of luciferase, such as aequorin photoprotein luciferase or luciferase from *Renilla*, is added thereto. Increasing the concentration of luciferase, for example, to 100 mg/l, provides a particularly brilliant display of light.

It is understood, that concentrations and amounts to be used depend upon the selected bioluminescence generating system but these may be readily determined empirically. Proportions, particularly those used when commencing an empirical determination, are generally those used for analytical purposes, and amounts or concentrations are at least those used for analytical purposes, but the amounts can be increased, particularly if a sustained and brighter glow is desired.

For purposes herein, *Renilla reniformis* GFP is added to the reaction in order to shift the spectrum of the generated light.

### 2. The *Renilla* system

*Renilla,* also known as soft coral sea pansies, are members of the class of coelenterates Anthozoa, which includes other bioluminescent genera, such as *Cavarnularia, Ptilosarcus, Stylatula, Acanthoptilum,* and *Parazoanthus.* Bioluminescent members of the Anthozoa genera contain luciferases and luciferins that are similar in structure (see, *e.g.*, Cormier *et al.* (1973) *J. Cell. Physiol. 81*:291-298; see, also Ward *et al.* (1975) *Proc. Natl. Acad. Sci. U.S.A. 72*: 2530-2534). The luciferases and luciferins from each of these anthozoans crossreact with one another and produce a characteristic blue luminescence.

*Renilla* luciferase and the other coelenterate and ctenophore luciferases, such as the aequorin photoprotein, use imidazopyrazine substrates, particularly the substrates generically called coelenterazine (see, formulae (I) and (II) of Section B.1.b, above). Other genera that have luciferases that use a coelenterazine include: squid, such as *Chiroteuthis, Eucleoteuthis*, *Onychoteuthis, Watasenia,* cuttlefish, *Sepiolina;* shrimp, such as *Oplophorus, Acanthophyra, Sergestes,* and *Gnathophausia;* deep-sea fish, such as *Argyropelecus, Yarella, Diaphus, Gonadostomias* and *Neoscopelus.*

*Renilla* luciferase does not, however, have bound oxygen, and thus requires dissolved oxygen in order to produce light in the presence of a suitable luciferin substrate. Since *Renilla* luciferase acts as a true enzyme (*i.e.*, it does not have to be reconstituted for further use) the resulting luminescence can be long-lasting in the presence of saturating levels of luciferin. Also, *Renilla* luciferase is relatively stable to heat.

*Renilla* luciferases, DNA encoding *Renilla reniformis* luciferase, and use of the *Renilla reniformis* DNA to produce recombinant luciferase, as well as DNA encoding luciferase from other coelenterates, are well known and available (see, *e.g.,* SEQ ID No. 1, U.S. Patent Nos. 5,418,155 and 5,292,658; see, also, Prasher *et al.* (1985) *Biochem. Biophys. Res. Commun. 126*:1259-1268; Cormier (1981) "*Renilla* and *Aequorea* bioluminescence" in *Bioluminescence and Chemiluminescence,* pp. 225-233; Charbonneau *et al.* (1979) *J. Biol. Chem. 254*:769-780; Ward *et al.* (1979) *J. Biol. Chem. 254*:781-788; Lorenz *et al.* (1981) *Proc. Natl. Acad. Sci. U.S.A. 88*: 4438-4442; Hori *et al.* (1977) *Proc. Natl. Acad. Sci. U.S.A. 74*:4285-4287; Hori *et al.* (1975) *Biochemistry 14*:2371-2376; Hori *et al.* (1977) *Proc. Natl. Acad. Sci. U.S.A. 74*:4285-4287; Inouye *et al.* (1975) *Jap. Soc. Chem. Lett.*141-144; and Matthews *et al.* (1979) *Biochemistry 16*: 85-91). The DNA encoding *Renilla reniformis* luciferase and host cells containing such DNA provide a convenient means

for producing large quantities of *Renilla reniformis* enzyme, such as in those known to those of skill in the art (see, *e.g.,* U.S. Patent Nos. 5,418,155 and 5,292,658, which describe recombinant production of *Renilla reniformis* luciferase).

When used herein, the *Renilla* luciferase can be packaged in lyophilized form, encapsulated in a vehicle, either by itself or in combination with the luciferin substrate. Prior to use the mixture is contacted with an aqueous composition, preferably a phosphate buffered saline pH 7-8; dissolved $O_2$ will activate the reaction. Final concentrations of luciferase in the glowing mixture will be on the order of 0.01 to 1 mg/l or more. Concentrations of luciferin will be at least about $10^{-8}$ M, but 1 to 100 or more orders of magnitude higher to produce a long lasting bioluminescence.

In certain embodiments herein, about 1 to 10 mg, or preferably 2-5 mg, more preferably about 3 mg of coelenterazine will be used with about 100 mg of *Renilla* luciferase. The precise amounts, of course can be determined empirically, and, also will depend to some extent on the ultimate concentration and application. In particular, about addition of about 0.25 ml of a crude extract from the bacteria that express *Renilla* to 100 ml of a suitable assay buffer and about 0.005 μg was sufficient to produce a visible and lasting glow (see, U.S. Patent Nos. 5,418,155 and 5,292,658, which describe recombinant production of *Renilla reniformis* luciferase).

Lyophilized mixtures, and compositions containing the *Renilla* luciferase are also provided. The luciferase or mixtures of the luciferase and luciferin may also be encapsulated into a suitable delivery vehicle, such as a liposome, glass particle, capillary tube, drug delivery vehicle, gelatin, time release coating or other such vehicle. The luciferase may also be linked to a substrate, such as biocompatible materials.

**3. Ctenophore systems**

Ctenophores, such as *Mnemiopsis* (mnemiopsin) and *Beroe ovata* (berovin), and coelenterates, such as *Aequorea* (aequorin), *Obelia* (obelin) and *Pelagia,* produce bioluminescent light using similar chemistries (see, *e.g.,* Stephenson *et al.* (1981) *Biochimica et Biophysica Acta 678*:65-75; Hart *et al.* (1979) *Biochemistry 18*:2204-2210; International PCT Application No. WO 94/18342, which is based on U.S. application Serial No. 08/017,116, U.S. Patent No. 5,486,455 and other references and patents cited herein). The *Aequorin* and *Renilla* systems are representative and are described in detail herein as exemplary and as among the presently preferred systems. The *Aequorin* and *Renilla* systems can use the same luciferin and produce light using the same chemistry, but each luciferase is different. The *Aequorin* luciferase aequorin, as well as, for example, the luciferases mnemiopsin and berovin, is a photoprotein that includes bound oxygen and bound luciferin, requires $Ca^{2+}$ (or other suitable metal ion) to trigger the reaction, and must be regenerated for repeated use; whereas, the *Renilla* luciferase acts as a true enzyme because it is unchanged during the reaction and it requires dissolved molecular oxygen.

**4. The aequorin system**

The aequorin system is well known (see, *e.g.*, Tsuji *et al.* (1986) "Site-specific mutagenesis of the calcium-binding photoprotein aequorin," *Proc. Natl. Acad. Sci. USA 83*:8107-8111; Prasher *et al.* (1985) "Cloning and Expression of the cDNA Coding for Aequorin, a Bioluminescent Calcium-Binding Protein," *Biochemical and Biophysical Research Communications 126*:1259-1268; Prasher *et al.* (1986) *Methods in Enzymology 133*:288-297; Prasher, *et al.* (1987) "Sequence Comparisons of cDNAs Encoding for Aequorin Isotypes," *Biochemistry 26*:1326-1332; Charbonneau *et al.* (1985) "Amino Acid Sequence of the Calcium-Dependent Photoprotein Aequorin," *Biochemistry 24*:6762-6771; Shimomura *et al.* (1981) "Resistivity to denaturation of the apoprotein of aequorin and reconstitution of the luminescent photoprotein from the partially denatured apoprotein," *Biochem. J. 199*:825-828; Inouye *et al.* (1989) *J. Biochem. 105*: 473-477; Inouye *et al.* (1986) "Expression of Apoaequorin Complementary DNA in Escherichia coli," *Biochemistry 25*: 8425-8429; Inouye *et al.* (1985) "Cloning and sequence analysis of cDNA for the luminescent protein aequorin," *Proc. Natl. Acad. Sci. USA 82*:3154-3158; Prendergast, *et al.* (1978) "Chemical and Physical Properties of Aequorin and the Green Fluorescent Protein Isolated from *Aequorea forskalea" J. Am. Chem. Soc. 17*:3448-3453; European Patent Application 0 540 064 A1; European Patent Application 0 226 979 A2, European Patent Application 0 245 093 A1 and European Patent Application 0 245 093 B1; U.S. Patent No. 5,093,240; U.S. Patent No. 5,360,728; U.S. Patent No. 5,139,937; U.S. Patent No. 5,422,266; U.S. Patent No. 5,023,181; U.S. Patent No. 5,162,227; and SEQ ID Nos. 5-13, which set forth DNA encoding the apoprotein; and a form, described in U.S. Patent No. 5,162,227, European Patent Application 0 540 064 A1 and Sealite Sciences Technical Report No. 3 (1994), is commercially available from Sealite, Sciences, Bogart, GA as AQUALITE®).

This system is among the preferred systems for use herein. As will be evident, since the aequorin photoprotein includes noncovalently bound luciferin and molecular oxygen, it is suitable for storage in this form as a lyophilized powder or encapsulated into a selected delivery vehicle. The system can be encapsulated into pellets, such as liposomes or other delivery vehicles. When used, the vehicles are contacted with a composition, even tap water, that contains $Ca^{2+}$ (or other suitable metal ion), to produce a mixture that glows.

### a. Aequorin and related photoproteins

The photoprotein, aequorin, isolated from the jellyfish, *Aequorea,* emits light upon the addition of $Ca^{2+}$ (or other suitable metal ion). The aequorin photoprotein, which includes bound luciferin and bound oxygen that is released by $Ca^{2+}$, does not require dissolved oxygen. Luminescence is triggered by calcium, which releases oxygen and the luciferin substrate producing apoaqueorin.

The bioluminescence photoprotein aequorin is isolated from a number of species of the jellyfish *Aequorea.* It is a 22 kilodalton (kD) molecular weight peptide complex (see, *e.g.*, Shimomura *et al.* (1962) *J. Cellular and Comp. Physiol. 59*:233-238; Shimomura *et al.* (1969) *Biochemistry 8*:3991-3997; Kohama *et al.* (1971) *Biochemistry 10*: 4149-4152; and Shimomura *et al.* (1972) *Biochemistry 11*:1602-1608). The native protein contains oxygen and a heterocyclic compound coelenterazine, a luciferin, (see, below) noncovalently bound thereto. The protein contains three calcium binding sites. Upon addition of trace amounts $Ca^{2+}$ (or other suitable metal ion, such as strontium) to the photoprotein, it undergoes a conformational change that catalyzes the oxidation of the bound coelenterazine using the protein-bound oxygen. Energy from this oxidation is released as a flash of blue light, centered at 469 nm. Concentrations of calcium ions as low as $10^{-6}$ M are sufficient to trigger the oxidation reaction.

Naturally-occurring apoaequorin is not a single compound but rather is a mixture of microheterogeneous molecular species. *Aequoria* jellyfish extracts contain as many as twelve distinct variants of the protein (see, *e.g.*, Prasher *et al.* (187) *Biochemistry 26*:1326-1332; Blinks *et al.* (1975) *Fed. Proc. 34*:474). DNA encoding numerous forms has been isolated (see, *e.g.*, SEQ ID Nos. 5-9 and 13).

The photoprotein can be reconstituted (see, *e.g.*, U.S. Patent No. 5,023,181) by combining the apoprotein, such as a protein recombinantly produced in *E. coli,* with a coelenterazine, such as a synthetic coelenterazine, in the presence of oxygen and a reducing agent (see, *e.g.,* Shimomura *et al.* (1975) *Nature 256*:236-238; Shimomura *et al.* (1981) *Biochemistry J. 199*:825-828), such as 2-mercaptoethanol, and also EDTA or EGTA (concentrations between about 5 to about 100 mM or higher for applications herein) tie up any $Ca^{2+}$ to prevent triggering the oxidation reaction until desired. DNA encoding a modified form of the apoprotein that does not require 2-mercaptoethanol for reconstitution is also available (see, *e.g.*, U.S. Patent No. U.S. Patent No. 5,093,240). The reconstituted photoprotein is also commercially available (sold, e.g., under the trademark AQUALITE®, which is described in U.S. Patent No. 5,162,227).

The light reaction is triggered by adding $Ca^{2+}$ at a concentration sufficient to overcome the effects of the chelator and achieve the $10^{-6}$ M concentration. Because such low concentrations of $Ca^{2+}$ can trigger the reaction, for use in the methods herein, higher concentrations of chelator may be included in the compositions of photoprotein. Accordingly, higher concentrations of added $Ca^{2+}$ in the form of a calcium salt will be required. Precise amounts may be empirically determined. For use herein, it may be sufficient to merely add water to the photoprotein, which is provided in the form of a concentrated composition or in lyophilized or powdered form. Thus, for purposes herein, addition of small quantities of $Ca^{2+}$, such as those present in phosphate buffered saline (PBS) or other suitable buffers or the moisture on the tissue to which the compositions are contacted, should trigger the bioluminescence reaction.

Numerous isoforms of the aequorin apoprotein been identified isolated. DNA encoding these proteins has been cloned, and the proteins and modified forms thereof have been produced using suitable host cells (see, *e.g.*, U.S. Patent Nos. 5,162,227, 5,360,728, 5,093,240; see, also, Prasher *et al.* (1985) *Biophys. Biochem. Res. Commun. 126*: 1259-1268; Inouye *et al.* (1986) *Biochemistry 25*:8425-8429). U.S. Patent No. 5,093,240; U.S. Patent No. 5,360,728; U.S. Patent No. 5,139,937; U.S. Patent No. 5,288,623; U.S. Patent No. 5,422,266, U.S. Patent No. 5,162,227 and SEQ ID Nos. 5-13, which set forth DNA encoding the apoprotein; and a form is commercially available form Sealite, Sciences, Bogart, GA as AQUALITE®). DNA encoding apoaequorin or variants thereof is useful for recombinant production of high quantities of the apoprotein. The photoprotein is reconstituted upon addition of the luciferin, coelenterazine, preferably a sulfated derivative thereof, or an analog thereof, and molecular oxygen (see, *e.g.*, U.S. Patent No. 5,023,181). The apoprotein and other constituents of the photoprotein and bioluminescence generating reaction can be mixed under appropriate conditions to regenerate the photoprotein and concomitantly have the photoprotein produce light. Reconstitution requires the presence of a reducing agent, such as mercaptoethanol, except for modified forms, discussed below, that are designed so that a reducing agent is not required (see, *e.g.*, U.S. Patent No. 5,093,240).

For use herein, it is preferred aequorin is produced using DNA, such as that set forth in SEQ ID Nos. 5-13 and known to those of skill in the art or modified forms thereof. The DNA encoding aequorin is expressed in a host cell, such as *E. coli*, isolated and reconstituted to produce the photoprotein (see, *e.g.,* U.S. Patent Nos. 5,418,155, 5,292,658, 5,360,728, 5,422,266, 5,162,227).

Of interest herein, are forms of the apoprotein that have been modified so that the bioluminescent activity is greater than unmodified apoaequorin (see, *e.g.*, U.S. Patent No. 5,360,728, SEQ ID Nos. 10-12). Modified forms that exhibit greater bioluminescent activity than unmodified apoaequorin include proteins including sequences set forth in SEQ ID Nos. 10-12, in which aspartate 124 is changed to serine, glutamate 135 is changed to serine, and glycine 129 is changed to alanine, respectively. Other modified forms with increased bioluminescence are also available.

For use in certain embodiments herein, the apoprotein and other components of the aequorin bioluminescence

generating system are packaged or provided as a mixture, which, when desired is subjected to conditions under which the photoprotein reconstitutes from the apoprotein, luciferin and oxygen (see, *e.g.*, U.S. Patent No. 5,023,181; and U.S. Patent No. 5,093,240). Particularly preferred are forms of the apoprotein that do not require a reducing agent, such as 2-mercaptoethanol, for reconstitution. These forms, described, for example in U.S. Patent No. 5,093,240 (see, also Tsuji *et al.* (1986) *Proc. Natl. Acad. Sci. U.S.A. 83*:8107-8111), are modified by replacement of one or more, preferably all three cysteine residues with, for example serine. Replacement may be effected by modification of the DNA encoding the aequorin apoprotein, such as that set forth in SEQ ID No. 5, and replacing the cysteine codons with serine.

The photoproteins and luciferases from related species, such as *Obelia* are also contemplated for use herein. DNA encoding the $Ca^{2+}$-activated photoprotein obelin from the hydroid polyp *Obelia longissima* is known and available (see, *e.g.*, Illarionov *et al.* (1995) *Gene 153*:273-274; and Bondar *et al.* (1995) *Biochim. Biophys. Acta 1231*:29-32). This photoprotein can also be activated by $Mn^{2+}$ (see, *e.g.*, Vysotski *et al.* (1995) *Arch. Bioch. Biophys. 316*:92-93, Vysotski *et al.* (1993) *J. Biolumin. Chemilumin. 8*:301-305).

In general for use herein, the components of the bioluminescence are packaged or provided so that there is insufficient metal ions to trigger the reaction. When used, the trace amounts of triggering metal ion, particularly $Ca^{2+}$ is contacted with the other components. For a more sustained glow, aequorin can be continuously reconstituted or can be added or can be provided in high excess.

**b. Luciferin**

The aequorin luciferin is coelenterazine and analogs therein, which include molecules including the structure (formula (I)):

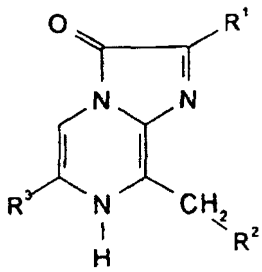

in which $R_1$ is $CH_2C_6H_5$ or $CH_3$; $R_2$ is $C_6H_5$, and $R_3$ is *p*-$C_6H_4OH$ or $CH_3$ or other such analogs that have activity. Preferred coelenterazine has the structure in which $R^1$ is *p*-$CH_2C_6H_4OH$, $R_2$ is $C_6H_5$, and $R_3$ is *p*-$C_6H_4OH$, which can be prepared by known methods (see, *e.g.*, Inouye *et al.* (1975) *Jap. Chem. Soc., Chemistry Lttrs.* pp 141-144; and Hart *et al.* (1979) *Biochemistry 18*:2204-2210). Among the preferred analogs, are those that are modified, whereby the spectral frequency of the resulting light is shifted to another frequency.

The preferred coelenterazine has the structure (formula (II)):

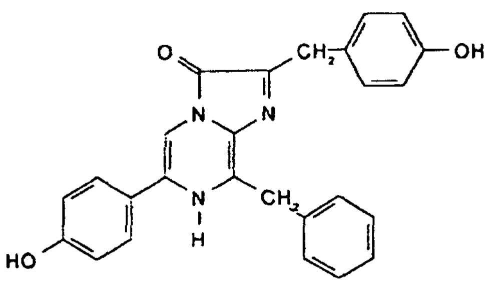

and sulfated derivatives thereof.

Another coelentratrazine has formula (V):

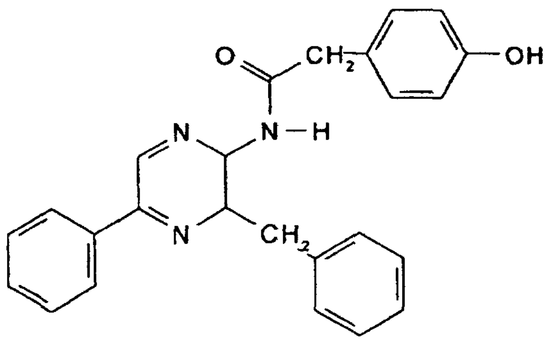

(see, Hart *et al.* (1979) *Biochemistry 18*:2204-2210). Using this derivative in the presence of luciferase all of the light is in the ultraviolet with a peak at 390 nm. Upon addition of GFP, all light emitted is now in the visible range with a peak at 509 nm accompanied by an about 200-fold increase in the amount of light emitted. Viewed with a cut-off filter of 470 nm, in the light yield in the absence of GFP would be about zero, and would be detectable in the presence of GFP. This provides the basis for an immunoassay described in the EXAMPLES.

The reaction of coelenterazine when bound to the aequorin photoprotein with bound oxygen and in the presence of $Ca^{2+}$ can represented as follows:

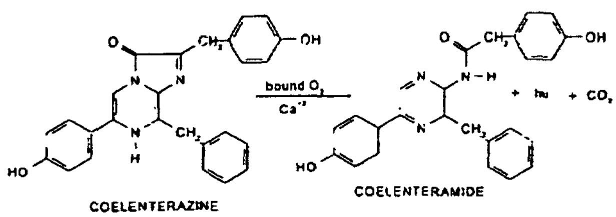


COELENTERAZINE

COELENTERAMIDE

The photoprotein aequorin (which contains apoaequorin bound to a coelenterate luciferin molecule) and *Renilla* luciferase, discussed below, can use the same coelenterate luciferin. The aequorin photoprotein catalyses the oxidation of coelenterate luciferin (coelenterazine) to oxyluciferin (coelenteramide) with the concomitant production of blue light ($lambda_{max}$ = 469 nm).

Importantly, the sulfate derivative of the coelenterate luciferin (lauryl-luciferin) is particularly stable in water, and thus may be used in a coelenteratelike bioluminescent system. In this system, adenosine diphosphate (ADP) and a sulpha-kinase are used to convert the coelenterazine to the sulphated form. Sulfatase is then used to reconvert the lauryl-luciferin to the native coelenterazine. Thus, the more stable lauryl-luciferin is used in the item to be illuminated and the luciferase combined with the sulfatase are added to the luciferin mixture when illumination is desired.

Thus, the bioluminescent system of *Aequorea* is particularly suitable for use in the methods herein. The particular amounts and the manner in which the components are provided depends upon the type of neoplasia or specialty tissue to be visualized. This system can be provided in lyophilized form, that will glow upon addition of $Ca^{2+}$. It can be encapsulated, linked to microcarriers, such as microbeads, or in as a compositions, such as a solution or suspension, preferably in the presence of sufficient chelating agent to prevent triggering the reaction. The concentration of the aequorin photoprotein will vary and can be determined empirically. Typically concentrations of at least 0.1 mg/l, more preferably at least 1 mg/l and higher, will be selected. In certain embodiments, 1-10 mg luciferin/100 mg of luciferase will be used in selected volumes and at the desired concentrations will be used.

### 5. Crustacean, particularly *Cyrpidina* systems

The ostracods, such as *Vargula serratta, hilgendorfii* and *noctiluca* are small marine crustaceans, sometimes called sea fireflies. These sea fireflies are found in the waters off the coast of Japan and emit light by squirting luciferin and luciferase into the water, where the reaction, which produces a bright blue luminous cloud, occurs. The reaction involves only luciferin, luciferase and molecular oxygen, and, thus, is very suitable for application herein.

The systems, such as the *Vargula* bioluminescent systems, are particularly preferred herein because the components are stable at room temperature if dried and powdered and will continue to react even if contaminated. Further, the bioluminescent reaction requires only the luciferin/luciferase components in concentrations as low as 1:40

parts per billion to 1:100 parts per billion, water and molecular oxygen to proceed. An exhausted system can renewed by addition of luciferin.

**a. *Vargula* luciferase**

The *Vargula* luciferase is water soluble and is among those preferred for use in the methods herein. *Vargula* luciferase is a 555-amino acid polypeptide that has been produced by isolation from *Vargula* and also using recombinant technology by expressing the DNA in suitable bacterial and mammalian host cells (see, *e.g.,* Thompson *et al.* (1989) *Proc. Natl. Acad. Sci. U.S.A. 86*:6567-6571; Inouye *et al.* (1992) *Proc. Natl. Acad. Sci. U.S.A. 89*:9584-9587; Johnson *et al.* (1978) *Methods in Enzymology LVII*:331-349; Tsuji *et al.* (1978) *Methods Enzymol. 57*:364-72; Tsuji (1974) *Biochemistry* 13:5204-5209; Japanese Patent Application No. JP 3-30678 Osaka; and European Patent Application No. EP 0 387 355 A1).

**(1) Purification from *Cypridina***

Methods for purification of *Vargula (Cypridina)* luciferase are well known. For example, crude extracts containing the active can be readily prepared by grinding up or crushing the *Vargula* shrimp. In other embodiments, a preparation of *Cypridina hilgendorfi* luciferase can be prepared by immersing stored frozen *C. hilgendorfi* in distilled water containing, 0.5-5.0 M salt, preferably 0.5-2.0 M sodium or potassium chloride, ammonium sulfate, at 0-30° C, preferably 0-10° C, for 1-48 hr, preferably 10-24 hr, for extraction followed by hydrophobic chromatography and then ion exchange or affinity chromatography (TORAY IND INC, Japanese patent application JP 4258288, published September 14, 1993; see, also, Tsuji *et al.* (1978) *Methods Enzymol. 57*:364-72 for other methods).

**(2) Preparation by Recombinant Methods**

The luciferase is preferably produced by expression of cloned DNA encoding the luciferase (European Patent Application No. 0 387 355 A1; International PCT Application No. WO 95/001542; see, also SEQ ID No. 5, which sets forth the sequence from Japanese Patent Application No. JP 3-30678 and Thompson *et al.* (1989) *Proc. Natl. Acad. Sci. U. S.A. 86*:6567-6571) DNA encoding the luciferase or variants thereof is introduced into *E. coli* using appropriate vectors and isolated using standard methods.

**b. *Vargula* luciferin**

The natural luciferin is a substituted imidazopyrazine nucleus, such a compound of formula (III):

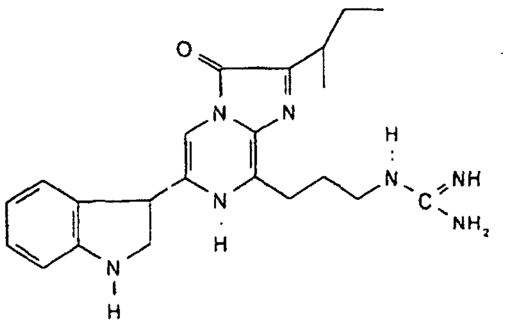

The luciferin can be isolated from ground dried *Vargula* by heating the extract, which destroys the luciferase but leaves the luciferin intact (see, *e.g.*, U.S. Patent No. 4,853,327).

Analogs thereof and other compounds that react with the luciferase in a light producing reaction also may be used.

Other bioluminescent organisms that have luciferases that can react with the *Vargula* luciferin include, the genera *Apogon, Parapriacanthus* and *Porichthys.*

**c. Reaction**

The luciferin upon reaction with oxygen forms a dioxetanone intermediate (which includes a cyclic peroxide similar to the firefly cyclic peroxide molecule intermediate). In the final step of the bioluminescent reaction, the peroxide

breaks down to form $CO_2$ and an excited carbonyl. The excited molecule then emits a blue to blue-green light.

The optimum pH for the reaction is about 7. For purposes herein, any pH at which the reaction occurs may be used. The concentrations of reagents are those normally used for analytical reactions or higher (see, *e.g.*, Thompson *et al*. (1990) *Gene 96*:257-262). Typically concentrations of the luciferase between 0.1 and 10 mg/l, preferably 0.5 to 2.5 mg/l will be used. Similar concentrations or higher concentrations of the luciferin may be used.

**6. Insect bioluminescent systems including fireflies, click beetles, and other insect system**

The biochemistry of firefly bioluminescence was the first bioluminescent system to be characterized (see, *e.g.*, Wienhausen *et al.* (1985) *Photochemistry and Photobiology 42*:609-611; McElroy *et al.* (1966) in *Molecular Architecture in cell Physiology,* Hayashi *et al.,* eds. Prentice Hall, Inc., Englewood Cliffs, NJ, pp. 63-80) and it is commercially available (*e.g.,* from Promega Corporation, Madison, WI, see, *e.g.,* Leach *et al.* (1986) *Methods in Enzymology 133*:51-70, esp. Table 1). Luciferases from different species of fireflies are antigenically similar. These species include members of the genera *Photinus, Photurins* and *Luciola.* Further, the bioluminescent reaction produces more light at 30°C than at 20°C, the luciferase is stabilized by small quantities of bovine albumin serum, and the reaction can be buffered by tricine.

**a. Luciferase**

DNA clones encoding luciferases from various insects and the use to produce the encoded luciferase is well known. For example, DNA clones that encode luciferase from *Photinus pyralis, Luciola cruciata* (see, *e.g.,* de Wet *et al*. (1985) *Proc. Natl. Acad. Sci. U.S.A. 82*:7870-7873; de We *et al.* (1986) *Methods in Enzymology 133:3;* U.S. Patent No. 4,968,613, see, also SEQ ID No. 3) are available. The DNA has also been expressed in *Saccharomyces* (see, *e.g.*, Japanese Application No. JP 63317079, published December 26, 1988, KIKKOMAN CORP) and in tobacco.

In addition to the wild-type luciferase modified insect luciferases have been prepared. For example, heat stable luciferase mutants, DNA-encoding the mutants, vectors and transformed cells for producing the luciferases are available. A protein with 60% amino acid sequence homology with luciferases from *Photinus pyralis, Luciola mingrelica, L. cruciata* or *L. lateralis* and having luciferase activity is available (see, *e.g.*, International PCT Application No. WO 95/25798). It is more stable above 30° C than naturally-occurring insect luciferases and may also be produced at 37° C or above, with higher yield.

Modified luciferases that generate light at different wavelengths (compared with native luciferase), and thus, may be selected for their color-producing characteristics. For example, synthetic mutant beetle luciferase(s) and DNA encoding such luciferases that produce bioluminescence at a wavelength different from wild-type luciferase are known (Promega Corp, International PCT Application No. WO 95/18853, which is based on U.S. application Serial No. 08/177,081). The mutant beetle luciferase has an amino acid sequence differing from that of the corresponding wild-type *Luciola cruciata* (see, *e.g.,* U.S. Patent Nos. 5,182,202, 5,219,737, 5,352,598, see, also SEQ ID No.3) by a substitution (s) at one or two positions. The mutant luciferase produces a bioluminescence with a wavelength of peak intensity that differs by at least 1 nm from that produced by wild-type luciferases.

Other mutant luciferases can be produced. Mutant luciferases with the amino acid sequence of wild-type luciferase, but with at least one mutation in which valine is replaced by isoleucine at the amino acid number 233, valine by isoleucine at 239, serine by asparagine at 286, glycine by serine at 326, histidine by tyrosine at 433 or proline by serine at 452 are known (see, e.g., U.S. Patent Nos. 5,219,737, and 5,330,906). The luciferases are produced by expressing DNA-encoding each mutant luciferase in *E. coli* and isolating the protein. These luciferases produce light with colors that differ from wild-type. The mutant luciferases catalyze luciferin to produce red (1609 nm and 612 nm), orange (1595 and 607 nm) or green (1558 nm) light. The other physical and chemical properties of mutant luciferase are substantially identical to native wild type-luciferase. The mutant luciferase has the amino acid sequence of *Luciola cruciata* luciferase with an alteration selected from Ser 286 replaced by Asn, Gly 326 replaced by Ser, His 433 replaced by Tyr or Pro 452 replaced by Ser. Thermostable luciferases are also available (see, *e.g.*, U.S. Patent No. 5,229,285; see, also International PCT Application No. WO 95/25798, which provides *Photinus* luciferase in which the glutamate at position 354 is replaced lysine and *Luciola* luciferase in which the glutamate at 356 is replaced with lysine).

These mutant luciferases as well as the wild type luciferases can be used in combination with the GFPs provided herein particularly in instances when a variety of colors are desired or when stability at higher temperatures is desired.

**b. Luciferin**

The firefly luciferin is a benzothiazole:

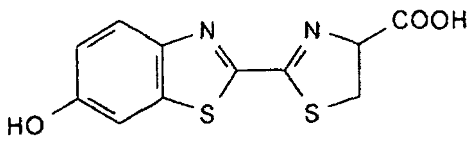

Analogs of this luciferin and synthetic firefly luciferins are also known to those of skill in art (see, *e.g.*, U.S. Patent No. 5,374,534 and 5,098,828). These include compounds of formula (IV) (see, U.S. Patent No. 5,098,828):

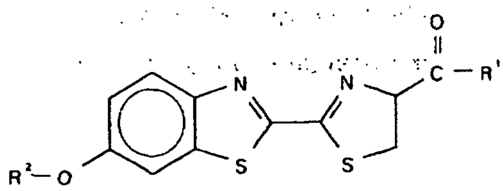

in which:

$R^1$ is hydroxy, amino, linear or branched $C_1$-$C_{20}$ alkoxy, $C_2$-$C_{20}$ alkyenyloxy, an L-amino acid radical bond via the α-amino group, an oligopeptide radical with up to ten L-amino acid units linked via the α-amino group of the terminal unit;
$R^2$ is hydrogen, $H_2PO_3$, $HSO_3$, unsubstituted or phenyl substituted linear or branched $C_1$-$C_{20}$ alkyl or $C_2$-$C_{20}$alkenyl, aryl containing 6 to 18 carbon atoms, or $R^3$-C(O)-; and
$R^3$ is an unsubstituted or phenyl substituted linear or branched $C_1$-$C_{20}$ alkyl or $C_2$-$C_{20}$alkenyl, aryl containing 6 to 18 carbon atoms, a nucleotide radical with 1 to 3 phosphate groups, or a glycosidically attached mono- or disaccharide, except when formula (IV) is a D-luciferin or D-luciferin methyl ester.

Modified luciferins that have been modified to produce light of shifted frequencies are known to those of skill in the art.

**c. Reaction**

The reaction catalyzed by firefly luciferases and related insect luciferases requires ATP, $Mg^{2+}$ as well as molecular oxygen. Luciferin must be added exogenously. Firefly luciferase catalyzes the firefly luciferin activation and the subsequent steps leading to the excited product. The luciferin reacts with ATP to form a luciferyl adenylate intermediate. This intermediate then reacts with oxygen to form a cyclic luciferyl peroxy species, similar to that of the coelenterate intermediate cyclic peroxide, which breaks down to yield $CO_2$ and an excited state of the carbonyl product. The excited molecule then emits a yellow light; the color, however, is a function of pH. As the pH is lowered the color of the bioluminescence changes from yellow-green to red.

Different species of fireflies emit different colors of bioluminescence so that the color of the reaction will be dependent upon the species from which the luciferase is obtained. Additionally, the reaction is optimized at pH 7.8.

Addition of ATP and luciferin to a reaction that is exhausted produces additional light emission. Thus, the system, once established, is relatively easily maintained. Therefore, it is highly suitable for use herein in embodiments in which a sustained glow is desired.

**7. Other systems**

Numerous other systems are known and have been described in detail for example in U.S. Patent Nos. 5,876,995, 6,152,358 and 6,113,886).

**a. Bacterial systems**

Luminous bacteria typically emit a continuous light, usually blue-green. When strongly expressed, a single bacterium may emit $10^4$ to $10^5$ photons per second. Bacterial bioluminescence systems include, among others, those systems found in the bioluminescent species of the genera *Photobacterium, Vibrio* and *Xenorhabdus.* These systems

are well known and well characterized (see, *e.g.,* Baldwin *et al.* (1984) *Biochemistry* 23:3663-3667; Nicoli *et al.* (1974) *J. Biol. Chem. 249*:2393-2396; Welches *et al.* (1981) *Biochemistry 20*:512-517; Engebrecht *et al.* (1986) *Methods in Enzymology 133*:83-99; Frackman *et al.* (1990) *J. of Bacteriology 172*:5767-5773; Miyamoto *et al.* (1986) *Methods in Enzymology 133*:70; U.S. Patent No. 4,581,335).

**(1) Luciferases**

Bacterial luciferase, as exemplified by luciferase derived from *Vibrio harveyi* (EC 1.14.14.3, alkanol reduced-FMN-oxygen oxidoreductase 1-hydroxylating, luminescing), is a mixed function oxidase, formed by the association of two different protein subunits α and β. The α-subunit has an apparent molecular weight of approximately 42,000 kD and the β-subunit has an apparent molecular weight of approximately 37,000 kD (see, *e.g.*, Cohn *et al.* (1989) *Proc. Natl. Acad. Sci. U.S.A. 90*:102-123). These subunits associate to form a 2-chain complex luciferase enzyme, which catalyzes the light emitting reaction of bioluminescent bacteria, such as *Vibrio harveyi* (U.S. Patent No. 4,581,335; Belas *et al.* (1982) *Science 218*:791-793), *Vibrio fischeri* (Engebrecht *et al.* (1983) *Cell 32*:773-781; Engebrecht *et al.* (1984) *Proc. Natl. Acad. Sci. U.S.A. 81*:4154-4158) and other marine bacteria.

Bacterial luciferase genes have been cloned (see, *e.g.*, U.S. Patent No. 5,221,623; U.S. Patent No. 4,581,335; European Patent Application No. EP 386 691 A). Plasmids for expression of bacterial luciferase, such as *Vibrio harveyi,* include pFIT001 (NRRL B-18080), pPALE001 (NRRL B-18082) and pMR19 (NRRL B-18081)) are known. For example the sequence of the entire *lux* regulon from *Vibiro fisheri* has been determined (Baldwin *et al.* (1984), *Biochemistry 23*: 3663-3667; Baldwin *et al.* (1981) *Biochem. 20*:512-517; Baldwin *et al.* (1984) *Biochem. 23*:3663-3667; see, also, *e.g.*, U.S. Patent Nos. 5,196,318, 5,221,623, and 4,581,335). This regulon includes *luxI* gene, which encodes a protein required for autoinducer synthesis (see, e.g., Engebrecht *et al.* (1984) *Proc. Natl. Acad. Sci. U.S.A. 81*:4154-4158), the *luxC, luxD,* and *luxE* genes, which encode enzymes that provide the luciferase with an aldehyde substrate, and the *luxA* and *luxB* genes, which encode the alpha and beta subunits of the luciferase.

Lux genes from other bacteria have also been cloned and are available (see, *e.g.,* Cohn *et al.* (1985) *J. Biol. Chem. 260*:6139-6146; U.S. Patent No. 5,196,524, which provides a fusion of the *luxA* and *luxB* genes from *Vibrio harveyi*). Thus, luciferase alpha and beta subunit-encoding DNA is provided and can be used to produce the luciferase. DNA encoding the α (1065 bp) and β (984 bp) subunits, DNA encoding a luciferase gene of 2124 bp, encoding the alpha and beta subunits, a recombinant vector containing DNA encoding both subunits and a transformed *E. coli* and other bacterial hosts for expression and production of the encoded luciferase are available. In addition, bacterial luciferases are commercially available.

**(2) Luciferins**

Bacterial luciferins include:

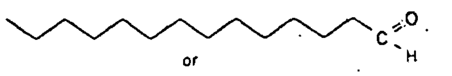 ;

or

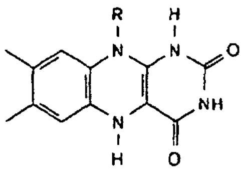

R is, for example, 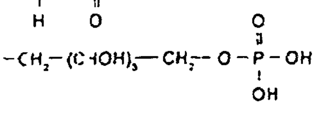

in which the tetradecanal with reduced flavin mononucleotide are considered luciferin since both are oxidized during the light emitting reaction.

**(3) Reactions**

The bacterial systems require, in addition to reduced flavin, five polypeptides to complete the bioluminescent

reaction: two subunits, α and β, of bacterial luciferin and three units of a fatty acid reductase system complex, which supplies the tetradecanal aldehyde. Examples of bacterial bioluminescent systems useful in the apparatus and methods provided herein include those derived from *Vibrio fisheri* and *Vibrio harveyi.* One advantage to this system is its ability to operate at cold temperatures; certain surgical procedures are performed by cooling the body to lower temperatures.

Bacterial luciferase catalyzes the flavin-mediated hydroxylation of a long-chain aldehyde to yield carboxylic acid and an excited flavin; the flavin decays to ground state with the concomitant emission of blue green light ($\lambda_{max}$ = 490 nm; see, *e.g.*, Legocki *et al*. (1986) *Proc. Natl. Acad. Sci. USA 81*:9080; see U.S. Patent No. 5,196,524):

$$FMNH_2 + R\text{-}CHO + O_2 \xrightarrow{\text{luciferase}} R-COOH + H_2O + h\nu\ .$$

The reaction can be initiated by contacting reduced flavin mononucleotide ($FMNH_2$) with a mixture of the bacterial luciferase, oxygen, and a long-chain aldehyde, usually n-decyl aldehyde.

DNA encoding luciferase from the fluorescent bacterium *Alteiomonas hanedai* is known (CHISSO CORP; see, also, Japanese application JP 7222590, published August 22, 1995). The reduced flavin mononucleotide ($FMNH_2$; luciferin) reacts with oxygen in the presence of bacterial luciferase to produce an intermediate peroxy flavin. This intermediate reacts with a long-chain aldehyde (tetradecanal) to form the acid and the luciferase-bound hydroxy flavin in its excited state. The excited luciferase-bound hydroxy flavin then emits light and dissociates from the luciferase as the oxidized flavin mononucleotide (FMN) and water. *In vivo* FMN is reduced again and recycled, and the aldehyde is regenerated from the acid.

Flavin reductases have been cloned (see, *e.g.*, U.S. Patent No. 5,484,723; see, SEQ ID No. 14 for a representative sequence from this patent). These as well as NAD(P)H can be included in the reaction to regenerate $FMNH_2$ for reaction with the bacterial luciferase and long chain aldehyde. The flavin reductase catalyzes the reaction of FMN, which is the luciferase reaction, into $FMNH_2$; thus, if luciferase and the reductase are included in the reaction system, it is possible to maintain the bioluminescent reaction. Namely, since the bacterial luciferase turns over many times, bioluminescence continues as long as a long chain aldehyde is present in the reaction system.

The color of light produced by bioluminescent bacteria also results from the participation of a protein blue-fluorescent protein (BFP) in the bioluminescence reaction. This protein, which is well known (see, *e.g.*, Lee *et al.* (1978) *Methods in Enzymology LVII*:226-234), may also be added to bacterial bioluminescence reactions in order to cause a shift in the color.

**b. Dinoflagellate bioluminescence generating systems**

In dinoflagellates, bioluminescence occurs in organelles termed scintillons. These organelles are outpocketings of the cytoplasm into the cell vacuole. The scintillons contain only dinoflagellate luciferase and luciferin (with its binding protein), other cytoplasmic components being somehow excluded. The dinoflagellate luciferin is a tetrapyrrole related to chlorophyll:

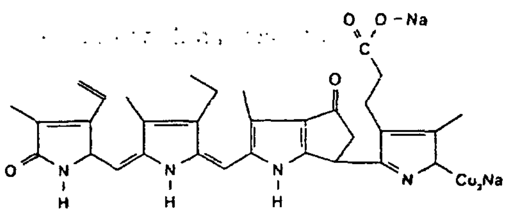

or an analog thereof.

The luciferase is a 135 kD single chain protein that is active at pH 6.5, but inactive at pH 8 (see, *e.g.,* Hastings (1981) *Bioluminescence and Chemiluminescence,* DeLuca *et al.,* eds. Academic Press, NY, pp.343-360). Luminescent activity can be obtained in extracts made at pH 8 by simply shifting the pH from 8 to 6. This occurs in soluble and particulate fractions. Within the intact scintillon, the luminescent flash occurs for ~100 msec, which is the duration of the flash *in vivo.* In solution, the kinetics are dependent on dilution, as in any enzymatic reaction. At pH 8, the luciferin is bound to a protein (luciferin binding protein) that prevents reaction of the luciferin with the luciferase. At pH 6, however, the luciferin is released and free to react with the enzyme.

## D. ISOLATION AND IDENTIFICATION OF NUCLEIC ACIDS ENCODING LUCIFERASES AND GFPs

Nucleic acid encoding bioluminescent proteins are provided. Particularly, nucleic acid encoding *Renilla reniformis* GFP is provided.

### 1. Isolation of specimens of the genus *Renilla*

Specimens of *Renilla* are readily available from the oceans of the world, including the Gulf of Mexico, Pacific Ocean and Atlantic Ocean. *Renilla* typically live on the ocean bottom at about 30 to 100 feet deep and can be easily collected by dragging. For example, specimens of *R. kollikeri* can be obtained off the coast of California or Baja, Mexico. Alternatively, live specimens of *Renilla* may be purchased from a commercial supplier *(e.g.,* Gulf Marine Incorporated, Panacea, Fla). Upon capture or receipt, the specimens are washed thoroughly and may also be dissected to enrich for light-emitting tissues. The whole organisms or dissected tissues are then snap frozen and stored in liquid nitrogen.
As described in detail in the examples below, the frozen tissues were used as a source to isolate nucleic acids encoding *Renilla mulleri* GFP and luciferase (*e.g.*, see SEQ ID No. 15 and SEQ ID No. 17, respectively).

### 2. Preparation of *Renilla* cDNA expression libraries

*Renilla* cDNA expression libraries may be prepared from intact RNA following the methods described herein or by other methods known to those of skill the art (*e.g.*, see Sambrook *et al*. (1989) *Molecular Cloning: A Laboratory Manual,* Cold Spring Harbor Laboratory Press, Cold Spring Harbor, NY.; U.S. Patent No. 5,292,658).
Typically, the preparation of cDNA libraries includes the isolation of polyadenylated RNA from the selected organism followed by single-strand DNA synthesis using reverse transcriptase, digestion of the RNA strand of the DNA/RNA hybrid and subsequent conversion of the single-stranded DNA to double stranded cDNA.

#### a. RNA isolation and cDNA synthesis

Whole *Renilla* or dissected *Renilla* tissues can be used a source of total cytoplasmic RNA for the preparation of *Renilla* cDNA. Total intact RNA can be isolated from crushed *Renilla* tissue, for example, by using a modification of methods generally known in the art (*e.g.*, see Chirgwin *et al*. (1970) *Biochemistry 18*:5294-5299). After isolating total cellular RNA, polyadenylated RNA species are then easily separated from the nonpolyadenylated species using affinity chromatography on oligodeoxythymidylate cellulose columns, (*e.g.*, as described by Aviv *et al*., (1972) *Proc. Natl. Acad. Sci. U.S.A. 69*:1408).
The purified *Renilla* polyA-mRNA is then subjected to a cDNA synthesis reaction to generate a cDNA library from total polyA-mRNA. Briefly, reverse transcriptase is used to extend an annealed polydT primer to generate an RNA/DNA duplex. The RNA strand is then digested using an RNase, *e.g.*, RNase H, and following second-strand synthesis, the cDNA molecules are blunted-ended with S1 nuclease or other appropriate nuclease. The resulting double-stranded cDNA fragments can be ligated directly into a suitable expression vector or, alternatively, oligonucleotide linkers encoding restriction endonuclease sites can be ligated to the 5'-ends of the cDNA molecules to facilitate cloning of the cDNA fragments.

#### b. Construction of cDNA expression libraries

The best characterized vectors for the construction of cDNA expression libraries are lambda vectors. Lambda-based vectors tolerate cDNA inserts of about 12 kb and provide greater ease in library screening, amplification and storage compared to standard plasmid vectors. Presently preferred vectors for the preparation of *Renilla* cDNA expression libraries are the Lambda, Uni-Zap, Lambda-Zap II or Lambda-ZAP Express/*Eco*RI/*Xho*I vectors, which are known to those of skill in the art *(e.g.,* see U.S. Pat. No. 5,128,256), and are also commercially available (Stratagene, La Jolla, CA).
Generally, the Lambda-Zap vectors combine the high efficiency of a bacteriophage lambda vector systems with the versatility of a plasmid system. Fragments cloned into these vectors can be automatically excised using a helper phage and recircularized to generate subclones in the pBK-derived phagemid. The pBK phagemid carries the neomycin-resistance gene for selection in bacteria and G418 selection in eukaryotic cells or may contain the β-lactamase resistance gene. Expression of the recombinant polypeptide is under the control of the *lac*Z promoter in bacteria and the CMV promoter in eukaryotes.
More specifically, these lambda-based vectors are composed of an initiator-terminator cassette containing the plasmid system, e.g., a pBK Bluescript derivative (Stratagene, San Diego), bracketed by the right and left arm of the bacteriophage lambda. The lambda arms allow for efficient packaging of replicated DNA whereas the excisable initiator-terminator cassette allows for easy cloning of the cDNA fragments and the generation of a plasmid library without

the need for additional subcloning.

When used herein, cDNA fragments are inserted into the multiple cloning site contained within the initiator-terminator cassette of the Lambda-Zap vector to create a set of cDNA expression vectors. The set of cDNA expression vectors is allowed to infect suitable *E. coli* cells, followed by co-infection with a filamentous helper phage. Within the cell, trans-acting proteins encoded by the helper phage, *e.g.*, the gene. II protein of M13, recognize two separate domains positioned within the lambda arms of the vector and introduce single-stranded nicks flanking the intiator-terminator cassette. Upon a subsequent round of DNA synthesis, a new DNA strand is synthesized that displaces the existing nick strand liberating the initiator-terminator cassette. The displaced strand is then circularized, packaged as filamentous phage by the helper proteins and excreted from the cell. The BK plasmid containing the cDNA is recovered by infecting an F' strain of *E. coli* and plating the infected cells on solid medium supplemented with kanamycin for the selection of pBK-containing cells.

The *Renilla* cDNA expression library can be screened using a variety of methods known to those of skill in the art. For example, identification of *Renilla* GFP may be achieved using a functional screening method employing blue light and observing colonies visually for emission of green fluorescence or by observing light emission using one or more bandpass filter.

### 3. Cloning of *Renilla reniformis* Green Fluorescent Protein

*Renilla reniformis* GFP has 233 amino acids compared GFPs from animals that contain luciferase-GFP bioluminescent systems *Renilla mulleri, Ptilosarcus* and *Aequorea Victoria.* Other such GFPs have 238 amino acids. At the amino acid level, *Renilla reniformis* is respectively 53, 51 and 19 % identical to the GFPs from these animals. The extent of identity of *Renilla reniformis* GFP to the half dozen cloned anthozoan coral GFPs, which do not contain associated luciferases, ranges from 32 to 38%. The overall identity among these GFPs is surprisingly low for a protein evolved from a common ancestor. These relationships are depicted as a phylogenetic tree (Figure 1).

Most surprising is the finding that the *Renilla reniformis* GFP is much more closely related to *Ptilosarcus* GFP (77% identity) than to *Renilla reniformis* GFP (53%). It is unclear why the sequence relatedness between these 3 GFPs does not follow traditional taxonomy. Given the sequence differences at the amino acid level, coding DNA sequences are surprisingly well conserved. *Renilla reniformis* GFP DNA is 56 and 59% identical to *Renilla mulleri* and *Ptilosarcus* GFP DNA.

Thus cloning *Renilla reniformis* GFP clone suggests why many groups may have failed in attempts to clone this gene by traditional methods. An attempt to sequence the entire protein by Edman degradation was difficult from the outset because the GFP was refractory to most attempts at specific proteolysis. Although over 80% of the protein was eventually accurately sequenced, a 30 amino acid region (110-139 of SEQ ID No. 27) had not be sequenced (as well as other regions, including amino acids 41-43, 65-71; SEQ ID No. 27). This 30 amino acid region apparently is degraded by the proteolytic methods used into very small fragments that are difficult to isolate and sequence; proper ordering of sequenced fragments was also difficult.

The cloned DNA fragments can be replicated in bacterial cells, such as *E. coli.* A preferred DNA fragment also includes a bacterial origin of replication, to ensure the maintenance of the DNA fragment from generation to generation of the bacteria. In this way, large quantities of the DNA fragment can be produced by replication in bacteria. Preferred bacterial origins of replication include, but are not limited to, the f1 ori and col E1 origins of replication. Preferred hosts contain chromosomal copies of DNA encoding T7 RNA polymerase operably linked to an inducible promoter, such as the lacUV promoter (see, U.S. Patent No. 4,952,496). Such hosts include, but are not limited to, lysogens *E. coli* strains HMS174(DE3)pLysS, BL21 (DE3)pLysS, HMS174(DE3) and BL21(DE3). Strain BL21(DE3) is preferred. The pLys strains provide low levels of T7 lysozyme, a natural inhibitor of T7 RNA polymerase.

For expression and for preparation of muteins, such as temperature sensitive muteins, eukaryotic cells, among them, yeast cells, such as *Saccharomyces* are preferred.

Nucleic acid encoding fusion proteins of the luciferases and GFPs are also provided. The resulting fusion proteins are also provided. Nucleic acids that encode luciferase and GFPs as polycistronic mRNA or under the control of separate promoters are also provided. Methods of use thereof are also provided.

The GFP cloned from *Renilla* has spectral properties that make it extremely useful. These properties include very high quantum efficiency, high molar absorbency and efficient use with universally available fluorescein filters (*e.g.*, Endo GFP filter set sold by Chroma). It is known that *Renilla reniformis* GFP is sixfold brighter than the wild-type *Aequorea* GFP on a molar basis, and three to fourfold brighter than the brightest mutant.

The *Renilla mullerei* GFP encoded by the nucleic acid clones provided herein exhibits similar functional characteristics, and the spectra appear identical with those from native *reniformis* GFP. Sequence comparison among the GFPs isolated from *Aequorea victoria, Renilla mullerei,* and *Ptilosarcus* reveal that the chromophore sequences of *R. mullerei* and *Ptilosarcus* are identical, and differ from *A. victoria.* These sequence differences point to protein sites that can be modified without affecting the essential fluorescence properties and also provide a means to identify residues

that change these properties.

### 4. Isolation and identification of DNA encoding *Renilla mulleri* GFP

Methods for identification and cloning of GPFs from *Renilla* have been described (see, published International PCT application No. WO 99/49019, and copending allowed U.S. application Serial No. 09/277,716). Nucleic acid encoding *Renilla mulleri* has been isolated. Briefly, a *R. mulleri A* Uni-Zap cDNA expression plasmid library was prepared, transformed into competent *E. coli* cells and plated onto modified L-broth plates containing carbon black to absorb background fluorescence. Transformants were sprayed with a solution containing IPTG to induce expression of the recombinant *Renilla* GFP from the heterologous cDNA. To identify GFP expressing clones, transformants were placed in blue light, preferably 470 to 490 nm light, and colonies that emitted green fluorescence were isolated and grown in pure culture.

The nucleotide sequence of the cDNA insert of a green fluorescent transformant was determined (*e.g.*, see SEQ ID No. 15). The 1,079 cDNA insert encodes a 238 amino acid polypeptide that is only 23.5 % identical to *A. victoria* GFP. The recombinant protein exhibits excitation and emission spectra similar to those reported for live *Renilla* species.

### 5. Isolation and identification of DNA encoding *Renilla mulleri* luciferase

The above-described *R. mulleri* cDNA expression library was also used to clone DNA encoding a *R. mulleri* luciferase. Single colony transformants were grown on modified L-broth plates containing carbon black and expression from the heterologous DNA was induced with IPTG, essentially as described above. After allowing time for expression, the transformants were sprayed with coelenterazine and screened for those colonies that emit blue light. Light-emitting colonies were isolated and grown in pure culture.

The nucleotide sequence of the cDNA insert contained in the light-emitting transformant was determined. The 1,217 cDNA insert encodes a 311 amino acid polypeptide. The recombinant protein exhibits excitation and emission spectra similar to those reported for live *Renilla* species.

## E. RECOMBINANT EXPRESSION OF PROTEINS

### 1. DNA encoding *Renilla* proteins

As described above, DNA encoding a *Renilla* GFP or *Renilla* luciferase can be isolated from natural sources, synthesized based on *Renilla* sequences provided herein or isolated as described herein.

In preferred embodiments, the DNA fragment encoding a *Renilla* GFP has the sequence of amino acids set forth in SEQ ID No. 27, encoded by nucleic acid, such as that set forth SEQ ID Nos. 23-26 and 27.

A DNA molecule encoding a *Renilla* luciferase has the sequence of amino acids set forth in SEQ ID No. 18. In more preferred embodiments, the DNA fragment encodes the sequence of amino acids encoded by nucleotides 31-963 of the sequence of nucleotides set forth in SEQ ID No. 17.

### 2. DNA constructs for recombinant production of *Renilla reniformis* GFP and other proteins

DNA is introduced into a plasmid for expression in a desired host. In preferred embodiments, the host is a bacterial host. The sequences of nucleotides in the plasmids that are regulatory regions, such as promoters and operators, are operationally associated with one another for transcription of the sequence of nucleotides that encode a *Renilla* GFP or luciferase. The sequence of nucleotides encoding the FGF mutein may also include DNA encoding a secretion signal, whereby the resulting peptide is a precursor of the *Renilla* GFP.

In preferred embodiments the DNA plasmids also include a transcription terminator sequence. The promoter regions and transcription terminators are each independently selected from the same or different genes.

A wide variety of multipurpose vectors suitable for the expression of heterologous proteins are known to those of skill in the art and are commercially available. Expression vectors containing inducible promoters or constitutive promoters that are linked to regulatory regions are preferred. Such promoters include, but are not limited to, the T7 phage promoter and other T7-like phage promoters, such as the T3, T5 and SP6 promoters, the *trp, lpp, tet* and *lac* promoters, such as the *lac*UV5, from *E. coli*; the SV40 promoter; the P10 or polyhedron gene promoter of baculovirus/ insect cell expression systems, retroviral long-terminal repeats and inducible promoters from other eukaryotic expression systems.

Particularly preferred vectors for recombinant expression of *Renilla mulleri* in prokaryotic organisms are *lac*- and T7 promoter-based vectors, such as the well known Bluescript vectors, which are commercially available (Stratagene, La Jolla, CA).

### 3. Host organisms for recombinant production of *Renilla* proteins

Host organisms include those organisms in which recombinant production of heterologous proteins have been carried out, such as, but not limited to, bacteria (for example, *E. coli,* yeast (for example, *Saccharomyces cerevisiae* and *Pichia pastoris*), fungi, baculovirus/insect systems, amphibian cells, mammalian cells, plant cells and insect cells. Presently preferred host organisms are strains of bacteria or yeast. Most preferred host organisms are strains of *E. coli* or *Saccharomyces cerevisiae.*

### 4. Methods for recombinant production of *Renilla* proteins

The DNA encoding a *Renilla* GFP or *Renilla mulleri* luciferase is introduced into a plasmid in operative linkage to an appropriate promoter for expression of polypeptides in a selected host organism. The DNA molecule encoding the *Renilla* GFP or luciferase may also include a protein secretion signal that functions in the selected host to direct the mature polypeptide into the periplasm or culture medium. The resulting *Renilla* GFP or luciferase can be purified by methods routinely used in the art, including methods described hereinafter in the Examples.

Methods of transforming suitable host cells, preferably bacterial cells, and more preferably *E. coli* cells, as well as methods applicable for culturing said cells containing a gene encoding a heterologous protein, are generally known in the art. See, for example, Sambrook *et al*. (1989) *Molecular Cloning: A Laboratory Manual,* Cold Spring Harbor Laboratory Press, Cold Spring Harbor, NY.

Once the *Renilla*-encoding DNA molecule has been introduced into the host cell, the desired *Renilla* GFP is produced by subjecting the host cell to conditions under which the promoter is induced, whereby the operatively linked DNA is transcribed. The cellular extracts of lysed cells containing the protein may be prepared and the resulting "clarified lysate" was employed as a source of recombinant *Renilla* GFP or *Renilla mulleri* luciferase. Alternatively, the lysate may be subjected to additional purification steps (e.g., ion exchange chromatography or immunoaffinity chromatography) to further enrich the lysate or provide a homogeneous source of the purified enzyme (see *e.g.*, U.S. Patent Nos. 5,292,658 and 5,418,155).

### 5. Recombinant cells expressing heterologous nucleic acid encoding Renilla GFP

Cells, vectors and methods are described with respect to *Renilla* The same cells, vectors and methods may be used for expressing luciferases and other GFPs from species including *Gaussia, Pleuromamma* and *Ptilosarcus.*

Recombinant cells containing heterologous nucleic acid encoding a *Renilla reniformis* GFP are provided. In preferred embodiments, the recombinant cells express the encoded *Renilla* GFP which is functional and non-toxic to the cell.

In certain embodiments, the recombinant cells may also include heterologous nucleic acid encoding a component of a bioluminescence-generating system, preferably a photoprotein or luciferase. In preferred embodiments, the nucleic acid encoding the bioluminescence-generating system component is isolated from the species *Aequorea, Vargula* or *Renilla.* In more preferred embodiments, the bioluminescence-generating system component is a *Renilla mulleri* luciferase having the amino acid sequence set forth in SEQ ID No. 18.

Recombinant host cells containing heterologous nucleic acid encoding a *Renilla mulleri* luciferase are also provided. In preferred embodiments, the heterologous nucleic acid encodes the sequence of amino acids as set forth in SEQ ID No. 18. In more preferred embodiments, the heterologous nucleic acid encodes the sequence of nucleotides set forth in SEQ ID No. 17.

Exemplary cells include bacteria (*e.g.*, *E. coli*), plant cells, cells of mammalian origin (e.g., COS cells, mouse L cells, Chinese hamster ovary (CHO) cells, human embryonic kidney (HEK) cells, African green monkey cells and other such cells known to those of skill in the art), amphibian cells (e.g., *Xenopus laevis* oöcytes), yeast cells (e.g., *Saccharomyces cerevisiae, Pichia pastoris),* and the like. Exemplary cells for expressing injected RNA transcripts include *Xenopus laevis* oöcytes. Eukaryotic cells that are preferred for transfection of DNA are known to those of skill in the art or may be empirically identified, and include HEK293 (which are available from ATCC under accession #CRL 1573); Ltk cells (which are available from ATCC under accession #CCL1.3); COS-7 cells (which are available from ATCC under accession #CRL 1651); and DG44 cells (dhfr CHO cells; see, e.g., Urlaub et al. (1986) *Cell. Molec. Genet. 12:* 555). Presently preferred cells include strains of bacteria and yeast.

The recombinant cells that contain the heterologous DNA encoding the *Renilla* GFP are produced by transfection with DNA encoding a *Renilla* GFP or luciferase or by introduction of RNA transcripts of DNA encoding a *Renilla* proteins using methods well known to those of skill in the art. The DNA may be introduced as a linear DNA fragment or may be included in an expression vector for stable or transient expression of the encoding DNA. The sequences set forth herein for *Renilla reniformis* GFP are presently preferred (see SEQ ID Nos 23-25 and 27; see, also SEQ ID No. 26, which sets forth human optimized codons).

Heterologous DNA may be maintained in the cell as an episomal element or may be integrated into chromosomal DNA of the cell. The resulting recombinant cells may then be cultured or subcultured (or passaged, in the case of mammalian cells) from such a culture or a subculture thereof. Also, DNA may be stably incorporated into cells or may be transiently expressed using methods known in the art.

The recombinant cells can be used in a wide variety of cell-based assay methods, such as those methods described for cells expressing wild type or modified *A. victoria* GFPs or GFP fusion proteins (e.g., see U.S. Patent No. 5,625,048; International patent application Publication Nos. WO 95/21191; WO 96/23810; WO 96/27675; WO 97/26333; WO 97/28261; WO 97/41228; and WO 98/02571).

## F. COMPOSITIONS AND CONJUGATES

Compositions and conjugates and methods of use are described with reference to *Renilla* proteins and nucleic acids. The same compositions and methods for preparation and use thereof are intended for use with other luciferases, such as *Pleuromamma* and *Ptilosarcus* proteins and nucleic acids.

### *1. Renilla* GFP compositions

Compositions containing a *Renilla* GFP or GFP peptide are provided. The compositions can take any of a number of forms, depending on the intended method of use therefor. In certain embodiments, for example, the compositions contain a *Renilla* GFP or GFP peptide, preferably *Renilla mulleri* GFP or *Renilla reniformis* GFP peptide, formulated for use in luminescent novelty items, immunoassays, FRET and FET assays. The compositions may also be used in conjunction with multi-well assay devices containing integrated photodetectors, such as those described herein.

Compositions that contain a *Renilla mulleri* GFP or GFP peptide and at least one component of a bioluminescence-generating system, preferably a luciferase, luciferin or a luciferase and a luciferin, are provided. In preferred embodiments, the luciferase/luciferin bioluminescence- generating system is selected from those isolated from: an insect system, a coelenterate system, a ctenophore system, a bacterial system, a mollusk system, a crustacea system, a fish system, an annelid system, and an earthworm system. Presently preferred bioluminescence- generating systems are those isolated from *Renilla, Aequorea*, and *Vargula*.

In more preferred embodiments, the bioluminescence-generating system component is a *Renilla mulleri* luciferase having the amino acid sequence set forth in SEQ ID No. 18 or a *Renilla reniformis* luciferase. These compositions can be used in a variety of methods and systems, such as included in conjunction with diagnostic systems for the *in vivo* detection of neoplastic tissues and other tissues, such as those methods described in detail below.

These methods and products include any known to those of skill in the art in which luciferase is used, including, but not limited to U.S. application Serial No. 08/757,046, 08/597,274 and 08/990,103, U.S. Patent No. 5,625,048; International patent application Publication Nos. WO 95/21191; WO 96/23810; WO 96/27675; WO 97/26333; WO 97/28261; WO 97/41228; and WO 98/02571).

### *2. Renilla* luciferase compositions

DNA encoding the *Renilla mulleri* luciferase or *Renilla reniformis* luciferase is used to produce the encoded luciferase, which has diagnostic applications as well as use as a component of the bioluminescence generating systems as described herein, such as in beverages, and methods of diagnosis of neoplasia and in the diagnostic chips described herein. These methods and products include any known to those of skill in the art in which luciferase is used, including, but not limited to, U.S. application Serial No. 08/757,046, 08/597,274 and 08/990,103, U.S. Patent No. 5,625,048; International patent application Publication Nos. WO 95/21191; WO 96/23810; WO 96/27675; WO 97/26333; WO 97/28261; WO 97/41228; and WO 98/02571).

In other embodiments, the *Renilla* luciferase and the remaining components may be packaged as separate compositions, that, upon mixing, glow. For example, a composition containing *Renilla* luciferase may be provided separately from, and for use with, an a separate composition containing a bioluminescence substrate and bioluminescence activator. In another instance, luciferase and luciferin compositions may be separately provided and the bioluminescence activator may be added after, or simultaneously with, mixing of the other two compositions.

### 3. Conjugates

Conjugates are provided herein for a variety of uses. Among them arer for targeting to tumors for visualization of the tumors, particularly *in situ* during surgery. A general description of these conjugates and the uses thereof is described in allowed U.S. application Serial No. 08/908,909. In practice, prior to a surgical procedure, the conjugate is administered via any suitable route, whereby the targeting agent binds to the targeted tissue by virtue of its specific

interaction with a tissue-specific cell surface protein. During surgery the tissue is contacted, with the remaining component (s), typically by spraying the area or local injection, and any tissue to which conjugate is bound will glow. The glow should be sufficient to see under dim light or, if necessary, in the dark.

The conjugates that are provided herein contain a targeting agent, such as a tissue specific or tumor specific monoclonal antibody or fragment thereof linked either directly or via a linker to a targeted agent, a *Renilla* GFP, *Renilla* or *Gaussia* luciferase and other luciferases (including photoproteins or luciferase enzymes) or a luciferin. The targeted agent may be coupled to a microcarrier. The linking is effected either chemically, by recombinant expression of a fusion protein in instances when the targeted agent is a protein, and by combinations of chemical and recombinant expression. The targeting agent is one that will preferentially bind to a selected tissue or cell type, such as a tumor cell surface antigen or other tissue specific antigen.

Methods for preparing conjugates are known to those of skill in the art. For example, aequorin that is designed for conjugation and conjugates containing such aequorin have been produced (see, e.g., International PCT application No. WO 94/18342; see, also Smith *et al.* (1995) in *American Biotechnology Laboratory).* Aequorin has been conjugated to an antibody molecule by means of a sulfhydryl-reacting binding agent (Stultz et al. (1992) Use of Recombinant Biotinylated Apoaequorin from *Escherichia coli.* Biochemistry 31, 1433-1442). Such methods may be adapted for use herein to produce the luciferase coupled to protein or other such molecules, which are useful as targeting agents. *Vargula* luciferase has also been linked to other molecules (see, *e.g.*, Japanese application No. JP 5064583, March 19, 1993). Such methods may be adapted for use herein to produce luciferase coupled to molecules that are useful as targeting agents.

The conjugates can be employed to detect the presence of or quantitate a particular antigen in a biological sample by direct correlation to the light emitted from the bioluminescent reaction.

As an alternative, a component of the bioluminescence generating system may be modified for linkage, such as by addition of amino acid residues that are particularly suitable for linkage to the selected substrate. This can be readily effected by modifying the DNA and expressing such modified DNA to produce luciferase with additional residues at the N- or C-terminus.

Methods for preparing conjugates are known to those of skill in the art. For example, aequorin that is designed for conjugation and conjugates containing such aequorin have been produced (see, *e.g.*, International PCT application No. WO 94/18342; see, also Smith *et al.* (1995) in *American Biotechnology Laboratory).* Aequorin has been conjugated to an antibody molecule by means of a sulfhydryl-reacting binding agent (Stultz et al. (1992) Use of Recombinant Biotinylated Apoaequorin from *Escherichia coli.* Biochemistry 31, 1433-1442). Such methods may be adapted for use herein to produce aequorin coupled to protein or other such molecules, which are useful as targeting agents. *Vargula* luciferase has also been linked to other molecules (see, *e.g.,* Japanese application No. JP 5064583, March 19, 1993). Such methods may be adapted for use herein to produce aequorin coupled to protein or other such molecules, which are useful as targeting agents. The bioluminescence generating reactions are used with the *Renilla reniformis* GFP provided herein.

### a. Linkers

Any linker known to those of skill in the art may be used herein. Other linkers are suitable for incorporation into chemically produced conjugates. Linkers that are suitable for chemically linked conjugates include disulfide bonds, thioether bonds, hindered disulfide bonds, and covalent bonds between free reactive groups, such as amine and thiol groups. These bonds are produced using heterobifunctional reagents to produce reactive thiol groups on one or both of the polypeptides and then reacting the thiol groups on one polypeptide with reactive thiol groups or amine groups to which reactive maleimido groups or thiol groups can be attached on the other. Other linkers include, acid cleavable linkers, such as bismaleimideothoxy propane, acid labile-transferrin conjugates and adipic acid diihydrazide, that would be cleaved in more acidic intracellular compartments; cross linkers that are cleaved upon exposure to UV or visible light and linkers, such as the various domains, such as $C_H1$, $C_H2$, and $C_H3$, from the constant region of human $IgG_1$ (see, Batra *et al.* (1993) *Molecular Immunol.* 30:379-386). In some embodiments, several linkers may be included in order to take advantage of desired properties of each linker.

Chemical linkers and peptide linkers may be inserted by covalently coupling the linker to the TA and the targeted agent. The heterobifunctional agents, described below, may be used to effect such covalent coupling. Peptide linkers may also be linked by expressing DNA encoding the linker and TA, linker and targeted agent, or linker, targeted agent and TA as a fusion protein.

Flexible linkers and linkers that increase solubility of the conjugates are contemplated for use, either alone or with other linkers are contemplated herein.

Numerous heterobifunctional cross-linking reagents that are used to form covalent bonds between amino groups and thiol groups and to introduce thiol groups into proteins, are known to those of skill in this art (see, *e.g.*, the PIERCE CATALOG, ImmunoTechnology Catalog & Handbook, 1992-1993, which describes the preparation of and use

of such reagents and provides a commercial source for such reagents; see, also, *e.g.,* Cumber *et al.* (1992) *Bioconjugate Chem. 3*:397-401; Thorpe *et al.* (1987) *Cancer Res. 47*:5924-5931; Gordon *et al.* (1987) *Proc. Natl. Acad Sci. 84*: 308-312; Walden *et al.* (1986) *J. Mol. Cell Immunol. 2*:191-197; Carlsson *et al.* (1978) *Biochem. J. 173:* 723-737; Mahan *et al.* (1987) *Anal. Biochem. 162*:163-170; Wawryznaczak *et al.* (1992) *Br. J. Cancer 66*:361-366; Fattom *et al.* (1992) *Infection & Immun. 60*:584-589). These reagents may be used to form covalent bonds between the TA and targeted agent. These reagents include, but are not limited to: N-succinimidyl-3-(2-pyridyldithio)propionate (SPDP; disulfide linker); sulfosuccinimidyl 6-(3-(2-pyridyldithio)propionamido)hexanoate (sulfo-LC-SPDP); succinimidyloxycarbonyl-α-methyl benzyl thiosulfate (SMBT, hindered disulfate linker); succinimidyl 6-(3-(2-pyridyldithio) propionamido)hexanoate (LC-SPDP); sulfosuccinimidyl 4-(N-maleimidomethyl)cyclohexane-1-carboxylate (sulfo-SMCC); succinimidyl 3-(2-pyridyldithio) butyrate (SPDB; hindered disulfide bond linker); sulfosuccinimidyl 2-(7-azido-4-methylcoumarin-3-acetamide) ethyl-1,3'-dithiopropionate (SAED); sulfo-succinimidyl 7-azido-4-methytcoumarin-3-acetate (SAMCA); sulfosuccinimidyl 6-(alpha-methyl-alpha-(2-pyridyidithio)toluamido)hexanoate (sulfo-LC-SMPT); 1,4-di-(3'-(2'-pyridyldithio)propionamido)butane (DPDPB); 4-succinimidyloxycarbonyl-α-methyl-α-(2-pyridylthio)toluene (SMPT, hindered disulfate linker); sulfosuccinimidyl6(α-methyl-α-(2-pyridyldithio)toluamido)hexanoate (sulfo-LC-SMPT); *m*-maleimidobenzoyl-N-hydroxysuccinimide ester (MBS); m-maleimidobenzoyl-N-hydroxysulfosuccinimide ester (sulfo-MBS); N-succinimidyl(4-iodoacetyl)aminobenzoate (SIAB; thioether linker); sulfosuccinimidyl(4-iodoacetyl)amino benzoate (sulfo-SIAB); succinimidyl4(*p*-maleimidophenyl)butyrate (SMPB); sulfosuccinimidyl4-(*p*-maleimidophenyl)butyrate (sulfo-SMPB); azidobenzoyl hydrazide (ABH).

Acid cleavable linkers, photocleavable and heat sensitive linkers may also be used, particularly where it may be necessary to cleave the targeted agent to permit it to be more readily accessible to reaction. Acid cleavable linkers include, but are not limited to, bismaleimideothoxy propane; and adipic acid dihydrazide linkers (see, *e.g.,* Fattom *et al.* (1992) *Infection & Immun. 60*:584-589) and acid labile transferrin conjugates that contain a sufficient portion of transferrin to permit entry into the intracellular transferrin cycling pathway (see, *e.g.,* Welhöner *et al.* (1991) *J. Biol. Chem. 266*: 4309-4314).

Photocleavable linkers are linkers that are cleaved upon exposure to light (see, *e.g.*, Goldmacher *et al.* (1992) *Bioconj. Chem. 3*:104-107, which linkers are herein incorporated by reference), thereby releasing the targeted agent upon exposure to light. Photocleavable linkers that are cleaved upon exposure to light are known (see, *e.g.*, Hazum *et al.* (1981) in *Pept., Proc. Eur. Pept. Symp.,* 16th, Brunfeldt, K (Ed), pp. 105-110, which describes the use of a nitrobetizyl group as a photocleavable protective group for cysteine; Yen *et al.* (1989) *Makromol. Chem 190*:69-82, which describes water soluble photocleavable copolymers, including hydroxypropylmethacrylamide copolymer, glycine copolymer, fluorescein copolymer and methylrhodamine copolymer; Goldmacher *et al.* (1992) *Bioconj. Chem. 3*:104-107, which describes a crosslinker and reagent that undergoes photolytic degradation upon exposure to near UV light (350 nm); and Senter *et al.* (1985) *Photochem. Photobiol 42*:231-237, which describes nitrobenzyloxycarbonyl chloride cross linking reagents that produce photocleavable linkages), thereby releasing the targeted agent upon exposure to light. Such linkers would have particular use in treating dermatological or ophthalmic conditions that can be exposed to light using fiber optics. After administration of the conjugate, the eye or skin or other body part can be exposed to light, resulting in release of the targeted moiety from the conjugate. Such photocleavable linkers are useful in connection with diagnostic protocols in which it is desirable to remove the targeting agent to permit rapid clearance from the body of the animal.

### b. Targeting Agents

Targeting agents include any agent that will interact with and localize the targeted agent cells in a tumor or specialized tissue (targeted tissue). Such agents include any agent that specifically interacts with a cell surface protein or receptor that is present at sufficiently higher concentrations or amounts on the targeted tissue, whereby, when contacted with an appropriate bioluminescence generating reagent and activators produces light. These agents include, but are not limited to, growth factors, preferentially modified to not internalize, methotrexate, and antibodies, particularly, antibodies raised against tumor specific antigens. A plethora of tumor-specific antigens have been identified from a number of human neoplasms.

### c. Anti-tumor Antigen Antibodies

Polyclonal and monoclonal antibodies may be produced against selected antigens. Alternatively, many such antibodies are presently available. An exemplary list of antibodies and the tumor antigen for which each has been directed against is provided in U.S. application Serial No., which is incorporated by reference in its entirety. It is contemplated that any of the antibodies listed may be conjugated with a bioluminescence generating component following the methods provided herein.

Among the preferred antibodies for use in the methods herein are those of human origin or, more preferably, are humanized monoclonal antibodies. These are preferred for diagnosis of humans.

**d. Preparation of the conjugates**

The methods for preparation of the conjugates for use in the tumor diagnostic methods can be used for preparation of the fusion proteins and conjugated proteins for use in the BRET system desribed below. Any method for linking proteins may be used. For example, methods for linking a luciferase to an antibody is described in U.S. Patent No. 5,486,455. As noted above, the targeting agent and luciferin or luciferase may be linked directly, such as through covalent bonds, *i.e.*, sulfhyryl bonds or other suitable bonds, or they may be linked through a linker. There may be more than one luciferase or luciferin per targeting agent, or more than one targeting agent per luciferase or luciferin.

Alternatively, an antibody, or $F(Ab)_2$ antigen-binding fragment thereof or other protein targeting agent may be fused (directly or via a linking peptide) to the luciferase using recombinant DNA technology. For example, the DNA encoding any of the anti-tumor antibodies of Table 3 may be ligated in the same translational reading frame to DNA encoding any of the above-described luciferases, *e.g.*, SEQ ID NOs. 1-14 and inserted into an expression vector. The DNA encoding the recombinant antibody-luciferase fusion may be introduced into an appropriate host, such as bacteria or yeast, for expression.

**4. Formulation of the compositions for use in the diagnostic systems**

In most embodiments, the *Renilla* GFPS and components of the diagnostic systems provided herein, such as *Renilla* luciferase, are formulated into two compositions: a first composition containing the conjugate; and a second composition containing the remaining components of the bioluminescence generating system. The compositions are formulated in any manner suitable for administration to an animal, particularly a mammal, and more particularly a human. Such formulations include those suitable for topical, local, enteric, parenteral, intracystal, intracutaneous, intravitreal, subcutaneous, intramuscular, or
intravenous administration.

For example, the conjugates, which in preferred embodiments, are a targeting agent linked to a luciferase (or photoprotein) are formulated for systemic or local administration. The remaining components are formulated in a separate second composition for topical or local application. The second composition will typically contain any other agents, such as spectral shifters that will be included in the reaction. It is preferred that the components of the second composition are formulated in a time release manner or in some other manner that prevents degradation and/or interaction with blood components.

**a. The first composition: formulation of the conjugates**

As noted above, the conjugates either contain a luciferase or luciferin and a targeting agents. The preferred conjugates are formed between a targeting agent and a luciferase, particularly the *Gaussia, Renilla mulleri* or *Pleuromamma* luciferase. The conjugates may be formulated into pharmaceutical compositions suitable for topical, local, intravenous and systemic application. Effective concentrations of one or more of the conjugates are mixed with a suitable pharmaceutical carrier or vehicle. The concentrations or amounts of the conjugates that are effective requires delivery of an amount, upon administration, that results in a sufficient amount of targeted moiety linked to the targeted cells or tissue whereby the cells or tissue can be visualized during the surgical procedure. Typically, the compositions are formulated for single dosage administration. Effective concentrations and amounts may be determined empirically by testing the conjugates in known *in vitro* and *in vivo* systems, such as those described here; dosages for humans or other animals may then be extrapolated therefrom.

Upon mixing or addition of the conjugate(s) with the vehicle, the resulting mixture may be a solution, suspension, emulsion or the like. The form of the resulting mixture depends upon a number of factors, including the intended mode of administration and the solubility of the conjugate in the selected carrier or vehicle. The effective concentration is sufficient for targeting a sufficient amount of targeted agent to the site of interest, whereby when combined with the remaining reagents during a surgical procedure the site will glow. Such concentration or amount may be determined based upon *in vitro* and/or *in vivo* data, such as the data from the mouse xenograft model for tumors or rabbit ophthalmic model. If necessary, pharmaceutically acceptable salts or other derivatives of the conjugates may be prepared.

Pharmaceutical carriers or vehicles suitable for administration of the conjugates provided herein include any such carriers known to those skilled in the art to be suitable for the particular mode of administration. In addition, the conjugates may be formulated as the sole pharmaceutically ingredient in the composition or may be combined with other active ingredients.

The conjugates can be administered by any appropriate route, for example, orally, parenterally, intravenously, intradermally, subcutaneously, or topically, in liquid, semi-liquid or solid form and are formulated in a manner suitable for each route of administration. Intravenous or local administration is presently preferred. Tumors and vascular proliferative disorders, will typically be visualized by systemic, intradermal or intramuscular, modes of administration.

The conjugate is included in the pharmaceutically acceptable carrier in an amount sufficient to produce detectable tissue and to not result in undesirable side effects on the patient or animal. It is understood that number and degree of side effects depends upon the condition for which the conjugates are administered. For example, certain toxic and undesirable side effects are tolerated when trying to diagnose life-threatening illnesses, such as tumors, that would not be tolerated when diagnosing disorders of lesser consequence.

The concentration of conjugate in the composition will depend on absorption, inactivation and excretion rates thereof, the dosage schedule, and amount administered as well as other factors known to those of skill in the art. Typically an effective dosage should produce a serum concentration of active ingredient of from about 0.1 ng/ml to about 50-1000 μg/ml, preferably 50-100 μg/ml. The pharmaceutical compositions typically should provide a dosage of from about 0.01 mg to about 100 - 2000 mg of conjugate, depending upon the conjugate selected, per kilogram of body weight per day. Typically, for intravenous administration a dosage of about between 0.05 and 1 mg/kg should be sufficient. Local application for, such as visualization of ophthalmic tissues or local injection into joints, should provide about 1 ng up to 1000 μg, preferably about 1 μg to about 100 μg, per single dosage administration. It is understood that the amount to administer will be a function of the conjugate selected, the indication, and possibly the side effects that will be tolerated. Dosages can be empirically determined using recognized models.

The active ingredient may be administered at once, or may be divided into a number of smaller doses to be administered at intervals of time. It is understood that the precise dosage and duration of administration is a function of the disease condition being diagnosed and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Solutions or suspensions used for parenteral, intradermal, subcutaneous, or topical application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parental preparations can be enclosed in ampules, disposable syringes or multiple dose vials made of glass, plastic or other suitable material.

If administered intravenously, suitable carriers include physiological saline or phosphate buffered saline (PBS), and solutions containing thickening and solubilizing agents, such as glucose, polyethylene glycol, and polypropylene glycol and mixtures thereof. Liposomal suspensions may also be suitable as pharmaceutically acceptable carriers. These may be prepared according to methods known to those skilled in the art.

The conjugates may be prepared with carriers that protect them against rapid elimination from the body, such as time release formulations or coatings. Such carriers include controlled release formulations, such as, but not limited to, implants and microencapsulated delivery systems, and biodegradable, biocompatible polymers, such as ethylene vinyl acetate, polyanhydrides, polyglycolic acid, polyorthoesters, polylacetic acid and others.

The conjugates may be formulated for local or topical application, such as for topical application to the skin and mucous membranes, such as in the eye, in the form of gels, creams, and lotions and for application to the eye or for intracisternal or intraspinal application. Such solutions, particularly those intended for ophthalmic use, may be formulated as 0.01 % -10% isotonic solutions, pH about 5-7, with appropriate salts. The ophthalmic compositions may also include additional components, such as hyaluronic acid. The conjugates may be formulated as aerosols for topical application (see, e.g., U.S. Patent Nos. 4,044,126, 4,414,209, and 4,364,923).

Also, the compositions for activation of the conjugate *in vivo* during surgical procedures may be formulated as an aerosol. These compositions contain the activators and also the remaining bioluminescence generating agent, such as luciferin, where the conjugate targets a luciferase, or a luciferase, where the conjugate targets a luciferin, such as coelenterazine.

If oral administration is desired, the conjugate should be provided in a composition that protects it from the acidic environment of the stomach. For example, the composition can be formulated in an enteric coating that maintains its integrity in the stomach and releases the active compound in the intestine. Oral compositions will generally include an inert diluent or an edible carrier and may be compressed into tablets or enclosed in gelatin capsules. For the purpose of oral administration, the active compound or compounds can be incorporated with excipients and used in the form of tablets, capsules or troches. Pharmaceutically compatible binding agents and adjuvant materials can be included as part of the composition.

Tablets, pills, capsules, troches and the like can contain any of the following ingredients, or compounds of a similar nature: a binder, such as microcrystalline cellulose, gum tragacanth and gelatin; an excipient such as starch and lactose, a disintegrating agent such as, but not limited to, alginic acid and corn starch; a lubricant such as, but not limited

to, magnesium stearate; a glidant, such as, but not limited to, colloidal silicon dioxide; a sweetening agent such as sucrose or saccharin; and a flavoring agent such as peppermint, methyl salicylate, and fruit flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The conjugates can also be administered as a component of an elixir, suspension, syrup, wafer, chewing gum or the like. A syrup may contain, in addition to the active compounds, sucrose as a sweetening agent and certain preservatives, dyes and colorings and flavors.

The active materials can also be mixed with other active materials that do not impair the desired action, or with materials that supplement the desired action, such as cis-platin for treatment of tumors.

Finally, the compounds may be packaged as articles of manufacture containing packaging material, one or more conjugates or compositions as provided herein within the packaging material, and a label that indicates the indication for which the conjugate is provided.

**b. The second composition**

The second composition will include the remaining components of the bioluminescence generating reaction. In preferred embodiments in which these components are administered systemically, the remaining components include the luciferin or substrate, and optionally additional agents, such as spectral shifters, particularly the GFPs provided herein. These components, such as the luciferin, can be formulated as described above for the conjugates. In some embodiments, the luciferin or luciferase in this composition will be linked to a protein carrier or other carrier to prevent degradation or dissolution into blood cells or other cellular components.

For embodiments, in which the second composition is applied locally or topically, they can be formulated in a spray or aerosol or other suitable means for local or topical application.

In certain embodiments described herein, all components, except an activator are formulated together, such as by encapsulation in a time release formulation that is targeted to the tissue. Upon release the composition will have been localized to the desired site, and will begin to glow.

In practice, the two compositions can be administered simultaneously or sequentially. Typically, the first composition, which contains the conjugate is administered first, generally an hour or two before the surgery, and the second composition is then administered, either pre-operatively or during surgery.

The conjugates that are provided herein contain a targeting agent, such as a tissue specific or tumor specific monoclonal antibody or fragment thereof linked either directly or via a linker to a targeted agent, a luciferase (including photoproteins or luciferase enzymes) or a luciferin. The targeted agent may be coupled to a microcarrier. The linking is effected either chemically, by recombinant expression of a fusion protein in instances when the targeted agent is a protein, and by combinations of chemical and recombinant expression. The targeting agent is one that will preferentially bind to a selected tissue or cell type, such as a tumor cell surface antigen or other tissue specific antigen.

Methods for preparing conjugates are known to those of skill in the art. For example, aequorin that is designed for conjugation and conjugates containing such aequorin have been produced (see, *e.g.,* International PCT application No. WO 94/18342; see, also Smith *et al.* (1995) in *American Biotechnology Laboratory).* Aequorin has been conjugated to an antibody molecule by means of a sulfhydryl-reacting binding agent (Stultz et al. (1992) Use of Recombinant Biotinylated Apoaequorin from *Escherichia coli (Biochemistry 31*:1433-1442). Such methods may be adapted for use herein to produce aequorin coupled to protein or other such molecules, which are useful as targeting agents. *Vargula* luciferase has also been linked to other molecules (see, *e.g.*, Japanese application No. JP 5064583, March 19, 1993). Such methods may be adapted for use herein to produce aequorin coupled to protein or other such molecules, which are useful as targeting agents.

Aequorin-antibody conjugates have been employed to detect the presence of or quantitate a particular antigen in a biological sample by direct correlation to the light emitted from the bioluminescent reaction.

As an alternative, the *Renilla* GFP or *Renilla mulleri* or *Gaussia* luciferase or a component of the bioluminescence generating system may be modified for linkage, such as by addition of amino acid residues that are particularly suitable for linkage to the selected substrate. This can be readily effected by modifying the DNA and expressing such modified DNA to produce luciferase with additional residues at the N- or C-terminus.

Selection of the system depends upon factors such as the desired color and duration of the bioluminescence desired as well as the particular item. Selection of the targeting agent primarily depends upon the type and characteristics of neoplasia or tissue to be visualized and the setting in which visualization will be performed.

The *Renilla reniformis* GFP is added to one or both compositions to act as a spectral shifter.

**c. Practice of the reactions in combination with targeting agents**

The particular manner in which each bioluminescence system will be combined with a selected targeting agent

will be a function of the agent and the neoplasia or tissue to be visualized. In general, however, a luciferin, *Renilla* GFP, *Renilla mulleri, Pleuromamma* or *Gaussia* luciferase or other luciferase, of the reaction will be conjugated to the targeting agent, administered to an animal prior to surgery. During the surgery, the tissues of interest are contacted with the remaining component(s) of a bioluminescence generating system. Any tissue to which or with which the targeting agent reacts will glow.

Any color of visible light produced by a bioluminescence generating system is contemplated for use in the methods herein. Preferably the visible light is a combination of blue, green and/or red light of varying intensities and wavelengths. For visualizing neoplasia or specialty tissues through mammalian tissues or tumors deeply embedded in tissue, longer wavelengths of visible light, *i.e.*, red and near infrared light, is preferred because wavelengths of near infrared light of about 700-1300 nm are known to penetrate soft tissue and bone (*e.g.,* see U.S. Patent No. 4,281,645).

In other embodiments, the conjugate can be applied to the tissues during surgery, such as by spraying a sterile solution over the tissues, followed by application of the remaining components. Tissues that express the targeted antigen will glow.

The reagents may be provided in compositions, such as suspensions, as powders, as pastes or any in other suitable sterile form. They may be provided as sprays, aerosols, or in any suitable form. The reagents may be linked to a matrix, particularly microbeads suitable for *in vivo* use and of size that they pass through capillaries. Typically all but one or more, though preferably all but one, of the components necessary for the reaction will be mixed and provided together; reaction will be triggered contacting the mixed component(s) with the remaining component(s), such as by adding $Ca^{2+}$, FMN with reductase, $FMNH_2$, ATP, air or oxygen.

In preferred embodiments the luciferase or luciferase/luciferin will be provided in combination with the targeting agent before administration to the patient. The targeting agent conjugate will then be contacted *in vivo* with the remaining components. As will become apparent herein, there are a multitude of ways in which each system may be combined with a selected targeting agent.

## G. COMBINATIONS

*Renilla reniformis* GFP can be used in combination with articles of manufacture to produce novelty items. The *Renilla reniformis* GFP can be used with a bioluminescence generating system. Such items and methods for preparation are described in detail in U.S. Patent Nos. 5,876,995, 6,152,358 and 6,113,886) These novelty items, which are articles of manufacture, are designed for entertainment, recreation and amusement, and include, but are not limited to: toys, particularly squirt guns, toy cigarettes, toy "Halloween" eggs, footbags and board/card games; finger paints and other paints, slimy play material; textiles, particularly clothing, such as shirts, hats and sports gear suits, threads and yarns; bubbles in bubble making toys and other toys that produce bubbles; balloons; figurines; personal items, such as bath powders, body lotions, gels, powders and creams, nail polishes, cosmetics including make-up, toothpastes and other dentifrices, soaps, body paints, and bubble bath; items such as fishing lures, inks, paper; foods, such as gelatins, icings and frostings; fish food containing luciferins and transgenic fish, particularly transgenic fish that express a luciferase; plant food containing a luciferin or luciferase, preferably a luciferin for use with transgenic plants that express luciferase; and beverages, such as beer, wine, champagne, soft drinks, and ice cubes and ice in other configurations; fountains, including liquid "fireworks" and other such jets or sprays or aerosols of compositions that are solutions, mixtures, suspensions, powders, pastes, particles or other suitable form.

Any article of manufacture that can be combined with a bioluminescence-generating system as provided herein and thereby provide entertainment, recreation and/or amusement, including use of the items for recreation or to attract attention, such as for advertising goods and/or services that are associated with a logo or trademark is contemplated herein. Such uses may be in addition to or in conjunction with or in place of the ordinary or normal use of such items. As a result of the combination, the items glow or produce, such as in the case of squirt guns and fountains, a glowing fluid or spray of liquid or particles.

## H. Exemplary uses of *Renilla reniformis* GFPs and encoding nucleic acid molecules

### 1. Methods for diagnosis of neoplasms and other tissues

Methods for diagnosis and visualization of tissues *in vivo* or *in situ,* preferably neoplastic tissue, using compositions containing a *Renilla mulleri* or *Ptilosarcus* GFP and/or a *Renilla mulleri, Pleuromamma* or *Gaussia* luciferase are provided. For example, the *Renilla mulleri* GFP protein can be used in conjunction with diagnostic systems that rely on bioluminescence for visualizing tissues *in situ*, such as those described in co-pending application Serial No. 08/908,909. The systems are particularly useful for visualizing and detecting neoplastic tissue and specialty tissue, such as during non-invasive and invasive procedures. The systems include compositions containing conjugates that include a tissue specific, particularly a tumor-specific, targeting agent linked to a targeted agent, such as a *Renilla reniformis*

GFP, a luciferase or luciferin. The systems also include a second composition that contains the remaining components of a bioluminescence generating reaction and/or the GFP. In some embodiments, all components, except for activators, which are provided *in situ* or are present in the body or tissue, are included in a single composition.

In particular, the diagnostic systems include two compositions. A first composition that contains conjugates that, in preferred embodiments, include antibodies directed against tumor antigens conjugated to a component of the bioluminescence generating reaction, a luciferase or luciferin, preferably a luciferase are provided. In certain embodiments, conjugates containing tumor-specific targeting agents are linked to luciferases or luciferins. In other embodiments, tumor-specific targeting agents are linked to microcarriers that are coupled with, preferably more than one of the bioluminescence generating components, preferably more than one luciferase molecule.

The second composition contains the remaining components of a bioluminescence generating system, typically the luciferin or luciferase substrate. In some embodiments, these components, particularly the luciferin are linked to a protein, such as a serum albumin, or other protein carrier. The carrier and time release formulations, permit systemically administered components to travel to the targeted tissue without interaction with blood cell components, such as hemoglobin that deactivates the luciferin or luciferase.

### 2. Methods of diagnosing diseases

Methods for diagnosing diseases, particularly infectious diseases, using chip methodology, a luciferase/luciferin bioluminescence-generating system, including a *Gaussia, Pleuromamma* or *Renilla mulleri* luciferase plus a *Renilla reniformis* GFP, are provided. In particular, the chip includes an integrated photodetector that detects the photons emitted by the bioluminescence-generating system as shifted by the GFP. This chip device, which is described in copending U.S. application Serial No. 08/990,103, which is published as International PCT application No. WO 98/26277, includes an integrated photodetector that detects the photons emitted by the bioluminescence generating system. The method may be practiced with any suitable chip device, including self-addressable and non-self addressable formats, that is modified as described herein for detection of generated photons by the bioluminescence generating systems. The chip device provided herein is adaptable for use in an array format for the detection and identification of infectious agents in biological specimens.

To prepare the chip, a suitable matrix for chip production is selected, the chip is fabricated by suitably derivatizing the matrix for linkage of macromolecules, and including linkage of photodiodes, photomultipliers CCD (charge coupled device) or other suitable detector, for measuring light production; attaching an appropriate macromolecule, such as a biological molecule or anti-ligand, *e.g.,* a receptor, such as an antibody, to the chip, preferably to an assigned location thereon. Photodiodes are presently among the preferred detectors, and specified herein. It is understood, however, that other suitable detectors may be substituted therefor.

In one embodiment, the chip is made using an integrated circuit with an array, such as an X-Y array, of photodetectors, such as that described in copending U.S. application Serial No. 08/990,103 The surface of circuit is treated to render it inert to conditions of the diagnostic assays for which the chip is intended, and is adapted, such as by derivatization for linking molecules, such as antibodies. A selected antibody or panel of antibodies, such as an antibody specific for particularly bacterial antigen, is affixed to the surface of the chip above each photodetector. After contacting the chip with a test sample, the chip is contacted with a second antibody linked to the GFP, such as the *Renilla* GFP, to form a chimeric antibody- GFP fusion protein or an antibody linked to a component of a bioluminescence generating system, such as a *Pleuromamma*, *Gaussia* or *R. mulleri* luciferase. The antibody is specific for the antigen. The remaining components of the bioluminescence generating reaction are added, and, if any of the antibodies linked to a component of a bioluminescence generating system are present on the chip, light will be generated and detected by the adjacent photodetector. The photodetector is operatively linked to a computer, which is programmed with information identifying the linked antibodies, records the event, and thereby identifies antigens present in the test sample.

### 3. Methods for generating *Renilla mulleri* luciferase*,Pleuromamma* luciferase and *Gaussia* luciferase fusion proteins with *Renilla reniformis* GFP.

Methods for generating GFP and luciferase fusion proteins are provided. The methods include linking DNA encoding a gene of interest, or portion thereof, to DNA encoding a *Renilla reniformis* GFP and a luciferase in the same translational reading frame. The encoded-protein of interest may be linked in-frame to the amino- or carboxyl-terminus of the GFP or luciferase. The DNA encoding the chimeric protein is then linked in operable association with a promoter element of a suitable expression vector. Alternatively, the promoter element can be obtained directly from the targeted gene of interest and the promoter-containing fragment linked upstream from the GFP or luciferase coding sequence to produce chimeric GFP proteins.

For example, a chimeric fusion containing the luciferase, preferably a *Renilla* luciferase, more preferably a *Renilla reniformis* luciferase, and *Renilla reniformis* GFP encoding DNA linked to the N-terminal portion of a cellulose

binding domain is provided.

### 4. Cell-based assays for identifying compounds

Methods for identifying compounds using recombinant cells that express heterologous DNA encoding a *Renilla reniformis* GFP under the control of a promoter element of a gene of interest are provided. The recombinant cells can be used to identify compounds or ligands that modulate the level of transcription from the promoter of interest by measuring GFP-mediated fluorescence. Recombinant cells expressing chimeric GFPs may also be used for monitoring gene expression or protein trafficking, or determining the cellular localization of the target protein by identifying localized regions of GFP-mediated fluorescence within the recombinant cell.

## I. KITS

Kits may be prepared containing the *Renilla reniformis* GFP or the encoding nucleic acid moleucles (see, SEQ ID Nos. 23-26) with or without components of a bioluminescence generating system for use in diagnostic and immunoassay methods and with the novelty items, including those described herein.

In one embodiment, the kits contain appropriate reagents and an article of manufacture for generating bioluminescence in combination with the article. These kits, for example, can be used with a bubble-blowing or producing toy or with a squirt gun. These kits can also include a reloading or charging cartridge.

In another embodiment, the kits are used for detecting and visualizing neoplastic tissue and other tissues and include a first composition that contains the *Renilla reniformis* GFP and a selected luciferase, such as a *Renilla mulleri, Renilla reniformis* or *Gaussia* luciferase, and a second that contains the activating composition, which contains the remaining components of the bioluminescence generating system and any necessary activating agents.

In other embodiments, the kits are used for detecting and identifying diseases, particularly infectious diseases, using multi-well assay devices and include a multi-well assay device containing a plurality of wells, each having an integrated photodetector, to which an antibody or panel of antibodies specific for one or more infectious agents are attached, and composition containing a secondary antibody, such as an antibody specific for the infectious agent that is linked, for example, to a *Renilla reniformis* GFP protein, a chimeric antibody-*Renilla reniformis* GFP fusion protein, F $(Ab)_2$ antibody fragment-*Renilla reniformis* GFP fusion protein or to such conjugates containing the, for example, *Gaussia* or *Renilla mulleri or reniformis,* luciferase. A second composition containing the remaining components of a bioluminescence generating system, such as system that emits a wavelength of light within the excitation range of the GFP, such as species of *Renilla* or *Aequorea,* for exciting the *Renilla* luciferase, which produces green light that is detected by the photodetector of the device to indicate the presence of the agent.

In further embodiments, the kits contain the components of the diagnostic systems. The kits comprise compositions containing the conjugates, preferably *Renilla* GFP and a *Gaussia,* or *Pleuromamma* or *Renilla mulleri* luciferase and remaining bioluminescence generating system components. The first composition in the kit typically contains the targeting agent conjugated to a GFP or luciferase. The second composition, contains at least the luciferin (substrate) and/or luciferase. Both compositions are formulated for systemic, local or topical application to a mammal. In alternative embodiments, the first composition contains the luciferin linked to a targeting agent, and the second composition contains the luciferase or the luciferase and a GFP.

In general, the packaging is non-reactive with the compositions contained therein and where needed should exclude water and or air to the degree those substances are required for the luminescent reaction to proceed.

Diagnostic applications may require specific packaging. The bioluminescence generating reagents may be provided in pellets, encapsulated as micro or macro-capsules, linked to matrices, preferably biocompatible, more preferably biodegradable matrices, and included in or on articles of manufacture, or as mixtures in chambers within an article of manufacture or in some other configuration. For example, a composition containing luciferase conjugate will be provided separately from, and for use with, a separate composition containing a bioluminescence substrate and bioluminescence activator.

Similarly, the *Renilla reniformis* GFP and selected luciferase and/or luciferin, such as a *Pleuromamma, Renilla mulleri* or *Gaussia* luciferase or luciferin, may be provided in a composition that is a mixture, suspension, solution, powder, paste or other suitable composition separately from or in combination with the remaining components, but in the absence of an activating component. Upon contacting the conjugate, which has been targeted to a selected tissue, with this composition the reaction commences and the tissue glows. In preferred embodiments, the tissue glows green emitting light near 510 nm. The luciferase, GFP and bioluminescence substrate, for example, are packaged to exclude water and/or air, the bioluminescence activator. Upon administration and release at the targeted site, the reaction with salts or other components at the site, including air in the case of surgical procedures, will activate the components. In some embodiments, it is desirable to provide at least the GFPs or one component of the bioluminescence generating system linked to a matrix substrate, which can then be locally or systemically administered.

Suitable dispensing and packaging apparatus and matrix materials are known to those of skill in the art, and preferably include all such apparatus described in U.S. Patent Nos. see U.S. Patent Nos. 5,876,995, 6,152,358 and 6,113,886.

### J. Muteins

Muteins of the *Renilla reniformis* GFP are provided herein. Muteins in which conservative amino acid changes that do not alter its ability to act as an acceptor of energy generated by a *Renilla* luciferase/substrate reaction are provided. Also provided are muteins with altered properties, including muteins with altered spectral properties, muteins with altered surface properties that reduce multimerization, including dimerization.

#### 1. Mutation of GFP surfaces to disrupt multimerization

Figure 5 depicts the three anthozoan fluorescent protein for which a crystal structure exists, another available commercially from Clontech as dsRed (also known as drFP583, as in this alignment) (Wall *et al.* (2000); *Nature Struct. Biol. 7*:1133-1138; Yarbrough *et al.* (2001) *Proc. Natl. Acad. Sci. U.S.A. 98:* 462-467). A dark gray background depicts amino acid conservation, and a light gray background depicts shared physiochemical properties. These crystal structures and biochemical characterization (Baird *et al.* (2000) *Proc. Natl. Acad. Sci. U.S.A. 97:* 11984-11989) show that dsRed exists as an obligate tetramer *in vitro.* Evidence also exists that dsRed multimerizes in living cells (Baird *et al.* (2000) *Proc. Natl. Acad. Sci. U.S.A. 97*: 11984-11989). Sedimentation and native gel electrophoresis studies indicate that *Ptilosarcus* and *Renilla mullerei* GFPs also form tetramers *in vitro* and multimerize *in vivo. Ptilosarcus* and *Renilla mullerei* GFPs diverge strongly in amino acid sequence from dsRed (39% and 38% identical, cespectively). Computational polypeptide threading algorithms predict that these GFPs fold into essentially the same structure as dsRed, and also the much more sequence divergent *Aequorea victoria* GFP. *Renilla reniformis* GFP is similarly related in sequence to dsRed, *Ptilosarcus* and *Renilla mullerei* GFPs (37%, 51% and 53% identical, respectively), and thus is extremely likely to form similar multimers. Multimerization is undesirable for many applications that use GFP as the reporting moiety in chimeric protein fusion. Hence muteins in which the capacity to multimerize is reduced are provided. Thus provided are mutations of *Renilla reniformis* GFP that disrupt the formation of GFP multimers. Such mutations may also be effected in the *Ptilosarcus* and *Renilla mullerei* and other GFPs (see Figure 6).

Two interaction surfaces within the dsRed tetramer, one primarily hydrophobic (residues marked by X) and one primarily hydrophilic (residues marked by O) have been described (see, Wall *et al.* (2000); *Nature Struct. Biol. 7*: 1133-1138). In general, the corresponding residues vary considerably between the 4 GFPs in a complex way, although the physicochemical properties of the amino acids are often conserved. There are a few clusters of conserved residues, especially between *Ptilosarcus* and *Renilla mullerei* GFPs, in keeping with their 77% overall identity.

The scheme provided herein for disruption focuses on altering surface amino acid side chains so that the surfaces acquire or retain a hydrophilic character, and are also altered in their stereo-chemistry (the sizes of the side chains are altered). These GFP surface regions roughly map to the β-sheet secondary structures that comprise the GFP β-barrel tertiary structure. It is thus essential that the secondary structure in any surface mutants be retained, so that the choice of amino acid side chain substitutions is governed by this consideration.

It is also desirable to introduce mutations that alter charge. For example, such mutations are those in which R, H and K residues have been replaced with D, such that the hydrophobic and hydrophilic surfaces now each contain 3 mutated residues (SEQ ID No. 33; Lys to Asp at amino acids 108, 127 and 226, Arg to Asp at amino acids 131 and 199; His to Asp at amino acid 172.

Site directed mutagenesis techniques are used to introduce amino acid side chains that amenable to aqueous solvation, and at that significantly alter surface sterochemistry. Disruption of interacting surfaces involves loss-of-function mutagenesis. It is thus contemplated that altering only a few residues, perhaps even one, is sufficient.

#### 2. Use of advantageous GFP surfaces with substituted fluorophores

Other surfaces of GFPs may be key determinants of GFP usefulness as reporters in living systems. A GFP surface may adventitiously interact with vital cellular components, thereby contributing to GFP-induced cytoxicity. Anthozoan GFPs from bioluminescent luciferase-GFP systems serve fundamentally different biological functions than do anthozoan GFPs from coral and anemones. The *Renilla reniformis* GFP is present in low quantity and functions as a resonance energy acceptor in response to a dynamic neural network that enables a startled animal to emit light flashes. A coral GFP-like protein is present in large quantity and apparently is used primarily as a passive pigment; it may not have evolved to dynamically interact with sensitive cellular machinery. These two classes of anthozoan fluorescent proteins thus may have surfaces with markedly different biological properties.

Figure 4 exemplifies the site for substitution for inserting fluorophores into the background of *Ptilosarcus, Renilla*

*mullerei* and *Renilla reniformis* GFPs. In particular, the 20 amino acid region that lies between two highly conserved prolines with the corresponding 20 amino acid region from any other anthozoan GFP (the underlined regions corresponds to amino acids 56-75 of SEQ ID No. 27 *Renilla reniformis* GFP; amino acids 59-78 of SEQ ID No. 16 *Renilla mulleri* GFP; and amino acids 59-78 of SEQ ID No. 32 for *Ptilosarcus* GFP) is replaced or modified. These 20 residues comprise the bulk of a polypeptide region that threads along the interior of the β-barrel structure that is characteristic of anthozoan GFPs (Wall *et al*. (2000) *Nature Struct. Biol. 7*:1133-7138; Yarbrough *et al*. (2001) *Proc. Natl. Acad. Sci. U.S.A. 98*: 462-467); replacement or modification alters spectral properties.

**K. Transgenic plants and animals**

As discussed above, transgenic animals and plants that contain the nucleic acid encoding the *Renilla reniformis* GFP are provided. Methods for producing transgenic plants and animals that express a GFP are known (see, *e.g.,* U.S. Patent No. 6,020,538).

Among the transgenic plants and animals provided are those that are novelty items, such as animals with eyes or fingernails or tusks or hair or that glows fluorescently. Transgenic food animals, such as chickens and cows and pigs are contemplated from which glowing meat and eggs (green eggs and ham) can be obtained; glowing worms can serve as fishing lures. In addition, the *Renilla reniformis* can serve as a reporter to detect that a heterologous gene linked to the GFP gene is incorporated into the animal's genome or becomes part of the genome in some or all cells. The *Renilla reniformis* can similarly be used as a reporter for gene therapy. The GFP can be introduced into plants to make transgenic ornamental plants that glow, such as orchids and roses and other flowering plants. Also the GFP can be used as a marker in plants, such as by linking it to a promoter, such as Fos that responds to secondary messages to assess signal transduction. The GFP can be linked to adenylcyclase causing the plants to emit different spectral frequencies as the levels of adenylcyclase change.

**L. Bioluminescence Resonance Energy Transfer (BRET) System**

In nature, coelenterazine-using luciferases emit broadband blue-green light (max. ~480 nm). Bioluminescence Resonance Energy Transfer (BRET) is a natural phenomenon first inferred from studies of the hydrozoan *Obelia* (Morin & Hastings (1971) *J. Cell Physiol. 77*:313-18), whereby the green bioluminescent emission observed *in vivo* was shown to be the result of the luciferase non-radiatively transferring energy to an accessory green fluorescent protein (GFP). BRET was soon thereafter observed in the hydrozoan *Aequorea victoria* and the anthozoan *Renilla reniforms.* Although energy transfer *in vitro* between purified luciferase and GFP has been demonstrated in *Aequorea* (Morise *et al.* (1974) *Biochemistry 13*:2656-62) and *Renilla* (Ward & Cormier (1976) *J. Phys. Chem. 80*:2289-91) systems, a key difference is that in solution efficient radiationless energy transfer occurs only in *Renilla,* apparently due to the pre-association of one luciferase molecule with one GFP homodimer (Ward & Cormier (1978) Photochem. Photobiol. 27:389-96). The blue (486 nm) luminescent emission of *Renilla* luciferase can be completely converted to narrow band green emission (508 nm) upon addition of proper amounts of *Renilla* GFP (Ward & Cormier (1976) *J. Phys. Chem. 80*:2289-91). GFPs accept energy from excited states of luciferase-substrate complexes and re-emit the light as narrow-band green light (~510 nm). By virtue of the non-radiative energy transfer, the quantum yield of the luciferase is increased.

Luciferases and fluorescent proteins have many well-developed and valuable uses as protein tags and transcriptional reporters; BRET increases the sensitivity and scope of these applications. A GFP increases the sensitivity of the luciferase reporter by raising the quantum yield. A single luciferase fused (or chemically linked) to several spectrally distinct GFPs provides for the simultaneous use of multiple luciferase reporters, activated by addition of a single luciferin. By preparing two fusion proteins (or chemical conjugates), each containing a GFP having a different emission wavelength fused to identical luciferases, two or more reporters can be used with a single substrate addition. Thus multiple events may be monitored or multiple assays run using a single reagent addition. Such a reporter system is self-ratioing if the distribution of luciferin is uniform or reproducible.

The ability to conveniently monitor several simultaneous macromolecular events within a cell is a major improvement over current bioluminescent technology. BRET also enables completely new modes of reporting by exploiting changes in association or orientation of the luciferase and fluorescent protein. By making fusion proteins, the luciferase-GFP acceptor pair may be made to respond to changes in association or conformation of the fused moieties and hence serves as a sensor.

Energy transfer between two fluorescent proteins (FRET) as a physiological reporter has been reported (Miyawaki *et al*. (1997) *Nature 388*:882-7), in which two different GFPs were fused to the carboxyl and amino termini of calmodulin. Changes in calcium ion concentration caused a sufficient conformational change in calmodulin to alter the level of energy transfer between the GFP moieties. The observed change in donor emission was ~10% while the change in ratio was ~1.8.

Figure 2, reproduced from allowed copending application U.S. application Serial No. 09/277,716, illustrates

the underlying principle of Bioluminescent Resonance Energy Transfer (BRET) using GFPs and luciferase, preferably cognate luciferase, and its use as sensor: A) in isolation, a luciferase, preferably an anthozoan luciferase, emits blue light from the coelenterazine-derived chromophore; B) in isolation, a GFP, preferably an anthozoan GFP that binds to the luciferase, that is excited with blue-green light emits green light from its integral peptide based fluorophore; C) when the luciferase and GFP associate as a complex *in vivo* or *in vitro,* the luciferase non-radiatively transfers its reaction energy to the GFP fluorophore, which then emits the green light; D) any molecular interaction that disrupts the luciferase-GFP complex can be quantitatively monitored by observing the spectral shift from green to blue light. Hence, the interaction or disruption thereof serves as a sensor.

The similar use of a luciferase-GFP pair in the presence of substrate luciferin has important advantages. First, there is no background and no excitation of the acceptor from the primary exciting light. Second, because the quantum yield of the luciferase is greatly enhanced by nonradiative transfer to GFP, background from donor emission is less, and the signal from the acceptor relatively greater. Third, the wavelength shift from the peak emission of luciferase (~480 nm) to that of the GFP (typically 508-510 nm) is large, minimizing signal overlap. All three factors combine to increase the signal-to-noise ratio. The concentration of the GFP acceptor can be independently ascertained by using fluorescence.

For some applications, *in vitro* crosslinked or otherwise *in vitro* modified versions of the native proteins is contemplated. The genetically encoded fusion proteins have many great advantages: A) In vivo use - unlike chemistry-based luminescence or radioactivity-based assays, fusion proteins can be genetically incorporated into living cells or whole organisms. This greatly increases the range of possible applications; B) Flexible and precise modification - many different response modifying elements can be reproducibly and quantitatively incorporated into a given luciferase-GFP pair; C) Simple purification - only one reagent would need to be purified, and its purification could be monitored via the fluorescent protein moiety. Ligand-binding motifs can be incorporated to facilitate affinity purification methods.

**1. Design of sensors based on BRET**

Resonance energy transfer between two chromophores is a quantum mechanical process that is exquisitely sensitive to the distance between the donor and acceptor chromophores and their relative orientation in space (Wu & Brand (1994) *Anal. Biochem. 218*:1-13). Efficiency of energy transfer is inversely proportional to the $6^{th}$ power of chromophore separation. In practice, the useful distance range is about 10 to 100 A, which has made resonance energy transfer a very useful technique for studying the interactions of biological macromolecules. A variety of fluorescence-based FRET biosensors have been constructed, initially employing chemical fluors conjugated to proteins or membrane components, and more recently, using pairs of spectrally distinct GFP mutants (Giuliano & Taylor (1998) *Trends Biotech. 16*:99-146; Tsien (1998) *Annu. Rev. Biochem. 67*:509-44).

Although these genetically encoded GFP bioluminescence -based biosensors have advantages over less convenient and less precise chemical conjugate-based biosensors, all share a limitation in their design: it is generally difficult to construct a biosensor in which energy transfer is quantitative when the chromophores are in closest apposition. It is almost impossible to arbitrarily manipulate the complex stereochemistry of proteins so that conjugated or intrinsic chromophores are *stably positioned with minimal separation and optimal orientation.* The efficiency of such biosensors are also often limited by stoichiometric imbalances between resonance energy donor and acceptor; the donor and acceptor macromolecules must be quantitatively complexed to avoid background signal emanating from uncomplexed chromophores. These limitations in general design become important when biosensors must be robust, convenient and cheap. Developing technologies such as high throughput screening for candidate drugs (using high throughput screening (HTS) protocoals), biochips and environmental monitoring systems would benefit greatly from modular biosensors where the signal of a rare target "hit" (*e.g.*, complex formation between two polypeptides) is unambiguously (statistically) distinguishable from the huge excess of "non-hits"). Current genetically encoded FRET and bioluminescence-based biosensors display hit signals that very often are less than two-fold greater than non-hit signals, and are at best a few-fold greater (Xu *et al*. (1999) *Proc. Natl. Acad. Sci USA 96:* 151-156; Miyawaki *et al*. (1997) *Nature 388*:882-7).

To solve these problems, the anthozoan GFPs, particularly the *Renilla* GFPs, provided herein can be used in combination with its cognate luciferases. Anthozoan luciferases-GFP complexes provide a "scaffold" upon which protein domains that confer the biological properties specific to a given biosensor can be linked. Although one can construct many useful two component biosensors based on this scaffold, in a biosensor contemplated herein, independent protein domains that potentially complex with one another are respectively fused to the luciferase and the GFP.

There are many possible variations on this theme. For example, in a three component system either the luciferase or GFP can be fused to a ligand-binding domain from a protein of interest or other target peptide or other moiety of interest. If the design of the fusion protein is correct, binding of a small molecule or protein ligand then prevents the luciferase-GFP association. The resulting combination of elements is a BRET-based biosensor; the change in spectral properties in the presence and absence of the ligand serves as sensor. More complex protein fusions can be designed to create two component and even single component BRET biosensors for a multitude of uses.

The nucleic acids, and the constructs and plasmids herein, permit preparation of a variety of configurations of

fusion proteins that include an anthozoan GFP, in this case *Renilla reniformis,* preferably with a *Renilla* luciferase, more preferably with the *Renilla reniformis* luciferase. The nucleic acid encoding the GFP can be fused adjacent to the nucleic acid encoding the luciferase or separated therefrom by insertion of nucleic acid encoding, for example, a ligand-binding domain of a protein of interest. The GFP and luciferase will be bound. Upon interaction of the ligand-binding domain with the a test compound or other moiety, the interaction of the GFP and luciferase will be altered thereby changing the emission signal of the complex. If necessary the GFP and luciferase can be modified to fine tune the interaction to make it more sensitive to conformational changes or to temperature or other parameters.

### 2. BRET Sensor Architectures

Figure 3 depicts some exemplary BRET sensor architectures. The upper left panel depicts the luciferase-GFP scaffold, the basis for the representative BRET sensor architectures shown here. The depicted single polypeptide fusion constructs place the luciferase and GFP at the polypeptide termini, bracketing interacting protein domains of choice. The luciferase and GFP can alternatively be placed centrally within the polypeptide, between interacting protein domains (not shown). This alternative arrangement is advantageous for one step protein interaction-based cloning schemes, where cDNA fragments encoding potential protein targets can be ligated onto one end of the construct.

Single polypeptide sensors that detect conformational changes within protein targets or the association-dissociation of protein targets are well-suited for the detection of physiological signals, such as those mediated by phosphorylation or other modification of targets, or by binding of regulatory ligands, such as hormones, to targets. Sensors based on interference are best suited to assaying the presence of small molecules or proteins independent of any regulatory context. Quantitative assays of metabolites, such as a sugar and allergens, are among those contemplated.

Since *in vivo* and *in vitro* luciferase-to-GFP energy transfer can be nearly 100% efficient, binding interactions between the luciferase and GFP must be sufficient to establish an optimal spatial relationship between donor and acceptor chromophores. Optimization of the luciferase-GFP energy transfer module is important in building effective BRET sensors. In a single polypeptide sensor it is crucial that the luciferase-GFP interaction be weak relative to interactions between target domains, thus the need for an optimized energy transfer module. In practice, either the luciferase or GFP surface can be randomly mutagenized, and an optimized luciferase-GFP scaffold then selected by screening for either blue or green emission at two near physiological temperatures (thermal endpoint-selection) using current robotic systems. This disruption of BRET is readily achievable because loss-of-function mutants (weakened luciferase-GFP binding) are orders of magnitude more frequent than gain-of-function mutants.

With an optimized energy transfer scaffold in hand, thermal endpoint-selection can then be used, if necessary, to optimize the interactions between the target domains incorporated into a sensor. This second round of thermal endpoint-selection may be especially important for the construction of interference sensors because it is essential that such sensors be able to "open and close" at near physiological temperatures to sense interference. Thermal endpoint-selection can also be used to weaken the binding affinity of the analyte to the interference sensor, making it possible to thermally wash off the analyte and reuse the sensor, a great advantage for biochip-based applications.

### 3. Advantages of BRET sensors

There are many advantages to the BRET sensors provided herein. For example, BRET sensors are self-ratioing. The reporter and target are integrated into single polypeptide. This ensures 1:1:1 stoichiometry among luciferase, GFP and target (or a 1:N:1 stoichiometry if more than one, typically a homodimer, GFP can be bound to a luciferase). GFP fluorescence allows absolute quantitation of sensor. The null state gives signal that verifies sensor functionality. Quantifiable null state facilitates disruption-of-BRET sensors (DBRET). BRET sensors have better signal-to-noise ratio than GFP FRET sensors because there is no cellular autofluorescence, no excitation of the acceptor from the primary exciting light, the quantum yield of luciferase greatly enhanced by non-radiative energy transfer to GFP, and there is minimal signal overlap between emission of the luciferase and emission of the GFP. Also, anthozoan GFPs have 6-fold higher extinction coefficients than Aequorea GFP.

The BRET sensors can for used for hit identification and downstream evaluation in *in vitro* screening assays in *in vitro* or *in vivo* or *in situ,* including in cultured cells and tissues and animals. The BRET sensors can be created by thermal endpoint-selection, which is suited to DBRET (Disruption-of-BRET) and reduces need for knowledge of target 3D structure and functional dynamics. Existing screening robotics to optimize biosensors. BRET sensors benefit from vast genetic diversity anthozoans have evolved efficient luciferase-GFP energy transfer systems and the components can be mixed and matched. Highly efficient heterologous luciferases may be substituted for less active luciferases. For example, a copepod luciferase active site can be fused to an anthozoan luciferase GFP-binding domain. There are many diverse coelenterazine-using luciferases.

BRET sensors are modular so that an optimized sensor scaffold may be used with different targets. Also the BRET acceptor may be varied to give shifted emissions, facilitating multiple simultaneous readouts. The anthozoan

GFPs can be mutated, GPFs or other proteins can be modified with different chemical fluors, high throughput screening (HTS) fluor-modified FRET acceptors can be adapted, the BRET donor (luciferase) may be varied, such as by using an Aequorin (Ca + + activated) photoprotein, or a firefly luciferse (requires ATP and a firefly luciferin) to give conditional activation. The sensor scaffold can be incorporated into a variety of immobilization motifs, including free format plates, which can reduce reagent volumes, reusable microtiter plates, miniature columns and biochips. Finally, BRET sensors are inexpensive and reproducible reagents because they can be produced by standardized protein production and can incorporate purification tags. Genetically encoded reporters more reproducible than chemically modified reporters. Linear translation of BRET modules ensures sensor integrity.

The following example is included for illustrative purposes only and is not intended to limit the scope of the invention.

## EXAMPLE

Specimens of the sea pansy *Renilla reniformis* were collected from inshore waters off the coast of Georgia. To prepare the sea pansies for isolation of mRNA, about 25 or so at time were placed on a large bed of dry ice. They were flipped with a spatula to flip them over to prevent them from freezing. Oddly, the entire animal illuminated when it came in contact with the dry ice.

The brightest and greenest were culled, placed in a bag and back into sea water at about 65-70° C for two hours. This process of dry ice, culling and sea water treatment was repeated three time over a 6 hour period. In addition, the process was performed at night. After they were exhausted were the last chilling, the culled animals were frozen solid. A cDNA library was prepared from the frozen animals.

The animals that were selected this way were frozen in liquid nitrogen, and shipped to Stratagene, Inc. (La Jolla, Ca). a commercial vendor whose business includes the construction of custom cDNA libraries under contract to prepare the library. Purified polyA-mRNA was prepared and a cDNA synthesis reaction was performed, appending a 3' *XhoI* site and a 5' *EcoR*I restriction site to the cDNA. The cDNA was inserted by ligation between the *EcoR*I and *Xho*I sites of the Uni-ZAP Lambda phage cDNA cloning vector.

The resulting unamplified library contained approximately 1.6 x $10^8$ primary plaques, which after amplification gave a titer of 3.5-7.5 pfb (plaque forming units)/ml. Insert sizes ranged from 0.9 to 3.0 kb, with an average size around 1.5 kb. Two mass excisions were performed to give pBluescript phagemid containing the cDNA inserts; each excision from about 8 x 10 plaques gave rise to about 4.8 x $10^9$ cfu (colony forming units)/ml. Phagemids were transfected into the SOLR strain of *E. coli.*

Screening was performed by plating (using an artist's airbrush) approximately 200,000 colonies to each of 40 cafeteria trays containing LB agar medium incorporating 0.4% carbon black to absorb background fluorescence. After 24 hours growth at 30° C in a humidified incubator, GFP expressing colonies were identified by illuminating the plates using a 250 Watt quartz halogen fiber optics light (Cuda Products Corp) with an EGFP bandpass excitation filter (Chroma), and viewing colonies through a GFP bandpass emission filter. Approximately 10 fluorescent colonies were picked, DNA isolated from minipreps, and the DNA transformed into the XL-10 Gold *E. coli* strain (Stratagene). Analysis by restriction digestion resolved three distinguishable sizes of insert. DNA was prepared from a clone of each size class and sent to SeqWright LLB (Houston, TX) for sequencing. Sequencing data were reported to Prolume on 1-25-99.

Three independent cDNA clones of *Renilla reniformis* GFP were isolated (SEQ ID Nos 23-25). Each cDNA is full length as judged by identical 5' termini and each encodes an identical protein of 233 amino acids (see SEQ ID No. 27). Compared to the primary clone (Clone 1), the coding sequence of Clone 2 differs by 4 silent mutations. Clones 2 and 3 also contain small differences in the 5' and 3' untranslated regions of the cDNA. This nucleic acid has been inserted into expression vector, and the encoded protein produced.

Since modifications may be apparent to those of skill in the art, it is intended that the invention be limited only by the appended claims.

## Claims

1. An isolated nucleic acid molecule encoding a *Renilla reniformis* green fluorescent protein, comprising a sequence of nucleotides that encodes the protein of SEQ ID No. 27 or a green fluorescent protein encoded by a *Renilla reniformis* having at least 80% sequence identity thereto.

2. An isolated nucleic acid molecule of claim 1 that encodes a protein having at least 90% sequence identity to the protein of SEQ ID No. 27.

3. The isolated nudeic acid molecule of claim 1, comprising a sequence of nucleotides selected from the group consisting

of:

(a) the coding portion of the sequence of nucleotides set forth in any of SEQ ID Nos. 23-25;
(b) a sequence of nucleotides that hybridizes under high stringency to the sequence of nuaeotides of (a): and
(c) a sequence of nucleotides comprising degenerate codons of (a) or (b).

**4.** The isolated nucleic acid molecule of claim 1, wherein the nucleic acid is DNA.

**5.** The isolated nucleic acid molecule of claim 1, wherein the nucleic acid is RNA.

**6.** The isolated nucleic acid molecule of claim 1 or claim 2, comprising the sequence of nucleotides set forth in SEQ ID No. 26 which comprises codons optirnised for human expression.

**7.** A nucleic acid probe or primer, comprising at least 14 contiguous nucleotides selected from the sequence of nucleotides set of claim 1.

**8.** The probe or primer of claim 7, comprising at least 16 contiguous nucleotides selected from the sequence of nucleotides in claim 1.

**9.** The probe or primer of claim 8, comprising at lest 30 contiguous nucleotides.

**10.** A plasmid, comprising the sequence of nucleotides of claim 1 or claim 6.

**11.** The plasmid of claim 10 that is an expression vector, comprising:

a promoter element;
a cloning site for the introduction of nucleic acid; and
a selectable marker;

wherein the nucleic acid encoding the cloning site is positioned between nucleic acids encoding the promoter element and the green fluorescent protein and wherein the nucleic acid encoding the green fluorescent protein is operatively linked to the promoter element.

**12.** The plasmid of claim 11, further comprising a sequence of nucleotides encoding a luciferase.

**13.** A recombinant host cell, comprising the plasmid of claim 10.

**14.** The cell of claim 13, wherein the cell is selected from the group consisting of a bacterial cell, a yeast cell, a fungal cell, a plant cell, an insect cell and an animal cell.

**15.** An isolated purified *Renilla reniformis* green fluorescent protein (GFP) encoded by the nucleic acid molecule of claim 1.

**16.** A mutein of the GFP of claim 15 that exhibits altered spectral properties.

**17.** A composition, comprising the green fluorescent protein of claim 15 and at least one component of a bioluminescence generating system.

**18.** The composition of claim 17, wherein the bioluminescence generating system is selected from those isolated from: an insect system, a coelenterate system, a ctenophore system, a bacterial system, a mollusk system, a crustacean system, a fish system, an annelid system, and an earthworm system.

**19.** The composition of claim 17, wherein the bioluminescence generating system is selected from those isolated from: fireflies, *Mnemiopsis, Beroe ovata, Aequorea, Obelia, Vargula, Pelagia, Renilla, Pholas Aristostomias, Pachystomias, Porichthys, Cypridina, Aristostomias, Pachystomias, Malacosteus, Gonadostomias, Gaussia, Watensia, Halisturia,* Vampire squid, *Glyphus,* Mucotophids, *Vinciguerria, Howella, Florenciella, Chaudiodus, Malanocostus,* Sea Pens, *Chiroteuthis, Eucleoteuthis, Onychoteuthis, Watasenia,* cuttlefish, *Sepiolina, Oplophorus, Acanthophyra, Sergestes, Gnathophausia, Argyropelecus, Yarella, Diaphus, Gonadostomias* and *Neoscopelus.*

**20.** A mutein of claim 15, comprising substitution in amino acids at amino acids 56-75 of SEQ ID No. 27, whereby spectral properties are altered.

**21.** The composition of claim 20, wherein the bioluminescence generating system is selected from those isolated from *Aequorea, Obelia, Vargula* and *Renilla*.

**22.** A reporter gene construct, comprising the nucleic acid of claim 1.

**23.** A combination, comprising:

an article of manufacture; and
a *Renilla reniformis* green fluorescent protein (GFP) encoded by a nucleic acid molecule of claim 1.

**24.** The combination of claim 23, further comprising
at least one component of a bioluminescence generating system, whereby the combination is a novelty item.

**25.** The combination of claim 24, wherein the component of the bioluminescence generating system comprises a luciferase.

**26.** The combination of claim 24, wherein the component of the bioluminescence generating system comprises a luciferin.

**27.** The combination of claim 23, wherein the article of manufacture is selected from among toys, fountains, personal care items, fairy dust, foods, textile and paper products.

**28.** The combination of claim 27, wherein the article of manufacture is selected from among toy guns, pellet guns, greeting cards, fingerpaints, foot bags, slimy play material, clothing, bubble making toys and bubbles therefor, balloons, bath powders, body lotions, gels, body powders, body creams, toothpastes, mouthwashes, soaps, body paints, bubble bath, board game toys, fishing lures, egg-shaped toys, toy cigarettes, dolls, sparklers, magic wand toys, wrapping paper, gelatins, icings, frostings, fairy dust, beer, ornamental transgenic plants, wine, champagne, milk, soft drinks, ice cubes, ice, dry ice, soaps, body paints and bubble bath.

**29.** The combination of claim 28 that is a transgenic ornamental plant.

**30.** The combination of claim 28 that is a toy.

**31.** The combination of claim 28 that is a food.

**32.** The combination of claim 28 that is a cosmetic.

**33.** The combination of claim 28 that is a beverage.

**34.** The combination of claim 24, wherein the article of manufacture is selected from among toys, fountains, personal care items, fairy dust, foods, textile, transgenic ornamental plant and paper products.

**35.** The combination of claim 34, wherein the article of manufacture is selected from among toy guns, pellet guns, greeting cards, fingerpaints, foot bags, slimy play material, clothing, bubble making toys and bubbles therefor, balloons, bath powders, body lotions, gels, body powders, body creams, toothpastes, mouthwashes, soaps, body paints, bubble bath, board game toys, fishing lures, egg-shaped toys, toy cigarettes, dolls, sparklers, magic wand toys, wrapping paper, gelatins, icings, frostings, fairy dust, beer, wine, champagne, soft drinks, ice cubes, ice, dry ice, soaps, body paints and bubble bath.

**36.** An antibody that specifically binds to *Renilla reniformis* or a molecule or derivative of the antibody containing the binding domain thereof.

**37.** The antibody of claim 36 that is a monoclonal antibody.

**38.** A nucleic acid construct, comprising a nucleotide sequence encoding a luciferase and a sequence of nucleotides of claim 1 that encodes a *Renilla reniformis* fluorescent protein (GFP).

**39.** The nucleic acid construct of claim 38, wherein the luciferase is a *Renilla mulleri* luciferase, a *Gaussia* luciferase or a *Pleuromamine* luciferase.

**40.** The nucleic acid construct of claim 39, wherein the *Gaussia* luciferase is a *Gaussia princeps* luciferase.

**41.** The nucleic acid construct of claim 38, wherein the luciferase is encoded by:

a sequence of nucleotides set forth in SEQ ID No. 17, SEQ ID No. 19, or SEQ ID No. 28;
a sequence of nucleotides encoding the amino acid sequence set forth in SEQ ID No. 18, SEQ ID No. 20 or SEQ ID No. 29; and
a sequence of nucleotides that hybridizes under high stringency to the sequence of nucleotides set forth in SEQ ID No. 17, SEQ ID No. 19 or SEQ ID No. 28.

**42.** The nucleic acid construct of claim 38 that is DNA.

**43.** The nucleic acid construct of claim 38 that is RNA.

**44.** A plasmid, comprising the nucleic acid construct of claim 38.

**45.** The plasmid of claim 44, further comprising a sequence of nucleotides encoding:

a promoter element;
a selectable marker;

wherein, the sequence of nucleotides encoding the luciferase and GFP is operatively linked to the promoter element, whereby the luciferase and GFP are expressed.

**46.** The construct of claim 38, wherein the luciferase and the GFP are encoded by a polycistronic message.

**47.** The construct of claim 38. wherein the encoded luciferase and fluorescent protein comprise a fusion protein.

**48.** The construct of claim 38, wherein the luciferase is *Renilla reniformis* luciferase.

**49.** A recombinant host cell, comprising the plasmid of claim 44.

**50.** The cell of claim 49, wherein the cell is selected from the group consisting of a bacterial cell, a yeast cell, a fungal cell, a plant cell, an insect cell and an animal cell.

**51.** An isolated purified luciferase and GFP fusion protein, wherein the GFP is a *Renilla reniformis* GFP and the fusion protein is encoded by the nucleic acid construct of claim 47.

**52.** The fusion protein of claim 51, wherein the luciferase is a *Renilla* luciferase.

**53.** The fusion protein of claim 51, wherein the luciferase is a *Renilla reniformis* luciferase.

**54.** A composition, comprising the fusion protein of claim 48.

**55.** The composition of claim 54, further comprising at least one component of a bioluminescence generating system.

**56.** The composition of claim 55, wherein the component of the bioluminescence generating system is a luciferin.

**57.** The nucleic acid construct of claim 47, wherein the sequence of nucleotides encoding the luciferase and GFP are not contiguous.

**58.** The nucleic acid construct of claim 57, comprising a sequence of nucleotides that encodes a ligand binding domain of a target protein.

**59.** A biosensor, comprising a GFP protein encoded by the nucleic acid molecule of claim 1 and a luciferase.

**60.** The biosensor of claim 59, wherein the luciferase is a *Renilla* luciferase.

**61.** A biosensor of claim 59, further comprising a modulator.

**62.** A biosensor, comprising a fusion protein of claim 51.

**63.** The biosensor of claim 62, wherein the GFP and luciferase in the fusion protein are not contiguous.

**64.** A bioluminescence resonance energy transfer (BRET) system, comprising:

(a) a GFP encoded by the nucleic molecule of claim 1;
(b) a luciferase from which the GFP can accept energy when the GFP and luciferase associate; and
(c) a luciferin or other substrate of the luciferase.

**65.** The BRET system of claim 64, further comprising one or more modulators.

**66.** The BRET system of claim 65, wherein the GFP and luciferase are each attached to a different modulator, or each are attached to the same modulator.

**67.** The BRET system of claim 65, wherein a conformational change in a modulator causes an increase in the proximity of the luciferase and GFP.

**68.** The BRET system of claim 65, wherein a conformational change in a modulator causes a decrease in the proximity of the luciferase and GFP.

**69.** The BRET system of claim 65, wherein the luciferase is *Renilla reniformis* luciferase.

**70.** A microelectronic device, comprising:

a substrate;
a plurality of micro-locations defined on the substrate, wherein each micro-location is for linking a macromolecule;
an independent photodetector integrated at or adjacent to each micro-location and optically coupled to each micro-location, each photodetector being configured to generate a sensed signal responsive to the photons of light emitted at the corresponding micro-location when a light-emitting chemical reaction occurs at that micro-location, each photodetector being independent from the photodetectors optically coupled to the other micro-locations; and
an electronic circuit coupled to each photodetector and configured to read the sensed signal generated by each photodetector and to generate output data signals therefrom that are indicative of the light emitted at each micro-location by the light-emitting chemical reactions, whereby the device detects photons of light emitted by light-emitting chemical reactions, wherein:

each micro-location is defined by a portion of the surface; and
the micro-locations defined on the substrate each comprise a component of.a bioluminescence generating system and a green fluorescent protein of claim 1, whereby photons of light are emitted when a reaction takes place at that micro-location.

**71.** The device of claim 70, wherein the micro-locations are provided as an array.

**72.** The device of claim 70, wherein the bioluminescence generating system comprises a *Renilla* luciferase.

**73.** The device of claim 71, wherein the bioluminescence generating system comprises a *Renilla reniformis* luciferase.

**74.** A method of detecting and identifying analytes in a biological sample, comprising:

providing the microelectronic device of claims 70;
attaching a macromolecule or plurality of different macromolecules to the surface at each micro-location on the device, wherein macromolecule is specific for binding to selected analyte that may be present in the biological sample;

contacting the sample with the surface of the microelectronic device, whereby any of the selected analytes that are present in the sample bind to the macromolecule attached to the surface at each micro-location;
exposing the surface of the microelectronic device to a second macromolecule or plurality thereof bound to the selected analyte already bound to the first macromolecule at each micro-location, wherein the second macromolecule comprises a component of a bioluminescence generating reaction;
initiating the bioluminescence generating reaction by contacting the surface of the device with the remaining components of the bioluminescence generating reaction, wherein the wavelength of the resulting light is shifted by the *Renilla reniformis* GFP; and
detecting photons of light emitted by the GFP using a photodetector optically coupled to each micro-location, each photodetector generating a sensed signal representative of the bioluminescence generation at the respective micro-location.

**75.** A transgenic animal or plant that expresses the *Renilla reniformis* nucleic acid of claim 1.

**76.** The transgenic animal or plant of claim 75, selected from among fish, worms, monkeys, rodents, goats, pigs, cows, sheep, horses, flowering plants, ornamental plants.

**77.** The transgenic animal or plant of claim 75 that is an orchid.

## Patentansprüche

**1.** Isoliertes Nukleinsäuremolekül, das für ein grünes Fluoreszenzprotein aus *Renilla reniformis* kodiert, umfassend eine Nukleotidsequenz, die für das Protein gemäß SEQ ID Nr. 27 oder für ein von *Renilla reniformis* kodiertes grünes Fluoreszenzprotein mit einer mindestens 80 %igen Sequenzidentität dazu kodiert.

**2.** Isoliertes Nukleinsäuremolekül nach Anspruch 1, das für ein Protein kodiert, das eine mindestens 90 %ige Sequenzidentität zu dem Protein gemäß SEQ ID Nr. 27 aufweist.

**3.** Isoliertes Nukleinsäuremolekül nach Anspruch 1, umfassend eine Nukleotidsequenz ausgewählt aus der Gruppe bestehend aus:

(a) dem kodierenden Teil der Nukleotidsequenz, dargestellt in einer beliebigen SEQ ID Nr. 23 - 25;
(b) eine Nukleotidsequenz, die unter hoher Stringenz an die Nukleotidsequenz aus (a) hybridisiert; und
(c) eine Nukleotidsequenz, die degenerierte Codons aus (a) oder (b) umfasst.

**4.** Isoliertes Nukleinsäuremolekül nach Anspruch 1, wobei die Nukleinsäure DNA ist.

**5.** Isoliertes Nukleinsäuremolekül nach Anspruch 1, wobei die Nukleinsäure RNA ist.

**6.** Isoliertes Nukleinsäuremolekül nach Anspruch 1 oder 2, umfassend die Nukleotidsequenz, die in SEQ ID Nr. 26 dargestellt ist, welche Codons umfasst, die für die Humanexpression optimiert sind.

**7.** Nukleinsäuresonde oder -primer, umfassend mindestens 14 aufeinander folgende Nukleotide, die ausgewählt sind aus dem Satz an Nukleotidsequenzen nach Anspruch 1.

**8.** Sonde oder Primer nach Anspruch 7, umfassend mindestens 16 aufeinander folgende Nukleotide, die ausgewählt sind aus der Nukleotidsequenz nach Anspruch 1.

**9.** Sonde oder Primer nach Anspruch 8, umfassend mindestens 30 aufeinander folgende Nukleotide.

**10.** Plasmid, umfassend die Nukleotidsequenz nach Anspruch 1 oder Anspruch 6.

**11.** Plasmid nach Anspruch 10, das ein Expressionsvektor ist, umfassend:

ein Promotorelement;
eine Klonierstelle für das Einbringen von Nukleinsäure; und
ein auswählbarer Marker,

wobei die Nukleinsäure, die für die Klonierstelle kodiert, zwischen Nukleinsäuren, die für das Promotorelement und das grüne Fluoreszenzprotein kodieren, positioniert ist und wobei die Nukleinsäure, die für das grüne Fluoreszenzprotein kodiert, operativ mit dem Promotorelement verknüpft ist.

**12.** Plasmid nach Anspruch 11, weiterhin umfassend eine Nukleotidsequenz, die für eine Luciferase kodiert.

**13.** Rekombinante Wirtszelle, umfassend das Plasmid nach Anspruch 10.

**14.** Zelle nach Anspruch 13, wobei die Zelle ausgewählt ist aus der Gruppe bestehend aus einer Bakterienzelle, einer Hefezelle, einer Pilzzelle, einer Pflanzenzelle, einer Insektenzelle und einer Tierzelle.

**15.** Isoliertes gereinigtes grünes Fluoreszenzprotein (GFP) aus *Renilla reniformis,* das von dem Nukleinsäuremolekül nach Anspruch1 kodiert ist.

**16.** Mutiertes Protein von GFP nach Anspruch 15, das veränderte Spektraleigenschaften aufweist.

**17.** Zusammensetzung, umfassend das grüne Fluoreszenzprotein nach Anspruch 15 und mindestens einen Bestandteil eines Biolumineszenz-erzeugenden Systems.

**18.** Zusammensetzung nach Anspruch 17, wobei das Biolumineszenz-erzeugende System ausgewählt aus solchen, die isoliert sind aus: einem Insektensystem, einem Hohltiersystem, einem Rippenquallensystem, einem Bakteriensystem, einem Weichtiersystem, einem Krustentiersystem, einem Fischsystem, einem Ringelwurmsystem und einem Regenwurmsystem.

**19.** Zusammensetzung nach Anspruch 17, wobei das Biolumineszenz-erzeugende System ausgewählt ist aus solchen, die isoliert sind aus: Leuchtkäfern, *Mnemiopsis, Beroe ovata, Aequorea, Obelia, Vargula, Pelagia, Renilla, Pholas Aristostomias, Pachystomias, Porichthys, Cypridina, Aristostomias, Pachystomias, Malacosteus, Gonadostomias, Gaussia, Watensia, Halisturia,* Vampyroteuthis infernalis, *Glyphus, Mucotophids, Vinciguerria, Howella, Florenciella, Chaudiodus, Malanocostus,* Seefedern, *Chiroteuthis, Eucleoteuthis, Onychoteuthis, Watasenia,* Tintenfisch, *Sepiolina, Oplophorus, Acanthophyra, Sergestes, Gnathophausia, Argyropelecus, Yarella, Diaphus, Gonadostomias* und *Neoscopelus.*

**20.** Mutiertes Protein nach Anspruch 15, umfassend eine Aminosäuresubstitution an den Aminosäuren 56 - 75 gemäß SEQ ID Nr. 27, wodurch Spektraleigenschaften verändert werden.

**21.** Zusammensetzung nach Anspruch 20, wobei das Biolumineszenz-erzeugende System ausgewählt ist aus solchen, die aus *Aequorea, Obelia, Vargula* und *Renilla* isoliert sind.

**22.** Reportergenkonstrukt, umfassend die Nukleinsäure nach Anspruch 1.

**23.** Kombination, umfassend:

einen Herstellungsartikel; und
ein grünes Fluoreszenzprotein (GFP) aus *Renilla reniformis,* das von einem Nukleinsäuremolekül nach Anspruch 1 kodiert ist.

**24.** Kombination nach Anspruch 23, weiterhin umfassend
mindestens einen Bestanteil eines Biolumineszenz-erzeugenden Systems, wobei die Kombination ein neuer Gegenstand ist.

**25.** Kombination nach Anspruch 24, wobei der Bestandteil des Biolumineszenz-erzeugenden Systems eine Luciferase umfasst.

**26.** Kombination nach Anspruch 24, wobei der Bestandteil des Biolumineszenz-erzeugenden Systems ein Luciferin umfasst.

**27.** Kombination nach Anspruch 23, wobei der Herstellungsartikel ausgewählt ist aus Spielzeug, Wasserspielen, Körperpflegegegenständen, Zauberstaub, Nahrungsmitteln, Textilien und Papierprodukten.

**28.** Kombination nach Anspruch 27, wobei der Herstellungsartikel ausgewählt ist aus Spielzeugpistolen, Farbkugelpistolen, Grußkarten, Fingerfarben, Fußsäcken, glibberigem Spielmaterial, Bekleidung, blasenbildendem Spielzeug und Blasen dafür, Ballons, Badepulvern, Körperlotionen, Gelen, Körperpulvern, Körpercremes, Zahnpasten, Mundwässern, Seifen, Körperfarben, Schaumbad, Brettspielspielzeug, Köder für das Fischen, eierförmigem Spielzeug, Spielzeugzigaretten, Puppen, Wunderkerzen, Zauberstabspielzeug, Einwickelpapier, Gelatine, Kuchenglasur, Zukkerguss, Zauberstaub, Bier, transgenen Zierpflanzen, Wein, Champagner, Milch, alkoholfreien Getränken, Eiswürfeln, Eis, Trockeneis, Seifen, Körperfarben und Schaumbad.

**29.** Kombination nach Anspruch 28, die eine transgene Zierpflanze darstellt.

**30.** Kombination nach Anspruch 28, die ein Spielzeug darstellt.

**31.** Kombination nach Anspruch 28, die ein Nahrungsmittel darstellt.

**32.** Kombination nach Anspruch 28, die ein Kosmetikum darstellt.

**33.** Kombination nach Anspruch 28, die ein Getränk darstellt.

**34.** Kombination nach Anspruch 24, wobei der Herstellungsartikel ausgewählt ist aus Spielzeug, Wasserspielen, Körperpflegegegenständen, Zauberstaub, Nahrungsmitteln, Textilien, transgener Zierpflanze und Papierprodukten.

**35.** Kombination nach Anspruch 24, wobei der Herstellungsartikel ausgewählt ist aus Spielzeugpistolen, Farbkugelpistolen, Grußkarten, Fingerfarben, Fußsäcken, glibberigem Spielmaterial, Bekleidung, blasenerzeugendem Spielzeug und Blasen dafür, Ballons, Badepulver, Körperlotionen, Gelen, Körperpudern, Körpercremes, Zahnpasten, Mundwässern, Seifen, Körperfarben, Schaumbad, Brettspielspielzeug, Köder für das Fischen, eierförmiges Spielzeug, Spielzeugzigaretten, Puppen, Wunderkerzen, Zauberstabspielzeug, Einwickelpapier, Gelatine, Kuchenglasur, Zuckerguss, Zauberstaub, Bier, Wein, Champagner, alkoholfreie Getränke, Eiswürfel, Eis, Trockeneis, Seifen, Körperfarben und Schaumbad.

**36.** Antikörper, der spezifisch an *Renilla reniformis* oder ein Molekül oder Derivat des Antikörpers bindet, der die Bindedomäne davon enthält.

**37.** Antikörper nach Anspruch 36, der ein monoklonaler Antikörper ist.

**38.** Nukleinsäurekonstrukt, umfassend eine Nukleotidsequenz, die für eine Luciferase kodiert und eine Nukleotidsequenz nach Anspruch 1, die für ein Fluoreszenzprotein (GFP) aus *Renilla reniformis* kodiert.

**39.** Nukleinsäurekonstrukt nach Anspruch 38, wobei die Luciferase eine Luciferase aus *Renilla mulleri,* eine Luciferase aus *Gaussia* oder eine Luciferase aus *Pleuromamma* darstellt.

**40.** Nukleinsäurekonstrukt nach Anspruch 39, wobei die Luciferase aus *Gaussia* eine Luciferase aus *Gaussia princeps* ist.

**41.** Nukleinsäurekonstrukt nach Anspruch 38, wobei die Luciferase kodiert wird von:

einer Nukleotidsequenz, die in SEQ ID Nr. 17, SEQ ID Nr. 19 oder SEQ ID Nr. 28 dargestellt ist;
einer Nukleotidsequenz, die für die Aminosäuresequenz kodiert, die in SEQ ID Nr. 18, SEQ ID Nr. 20 oder SEQ ID Nr. 29 dargestellt ist; und
eine Nukleotidsequenz, die unter hoher Stringenz an die Nukleotidsequenz, die in SEQ ID Nr. 17, SEQ ID Nr. 19 oder SEQ ID Nr. 28 dargestellt ist, hybridisiert.

**42.** Nukleinsäurekonstrukt nach Anspruch 38, das eine DNA darstellt.

**43.** Nukleinsäurekonstrukt nach Anspruch 38, das eine RNA darstellt.

**44.** Plasmid, umfassend das Nukleinsäurekonstrukt nach Anspruch 38.

**45.** Plasmid nach Anspruch 44, weiterhin umfassend eine Nukleotidsequenz, die kodiert für:

ein Promotorelement;
einen auswählbaren Marker;

wobei die Nukleotidsequenz, die für die Luciferase und GFP kodiert, operativ mit dem Promotorelement verknüpft ist, wobei die Luciferase und GFP exprimiert werden.

**46.** Konstrukt nach Anspruch 38, wobei die Luciferase und das GFP durch eine polycistronische Information kodiert sind.

**47.** Konstrukt nach Anspruch 38, wobei die kodierte Luciferase und das kodierte Fluoreszenzprotein ein Fusionsprotein umfassen.

**48.** Konstrukt nach Anspruch 38, wobei die Luciferase Luciferase aus *Renilla reniformis* ist.

**49.** Rekombinante Wirtszelle, die das Plasmid nach Anspruch 44 umfasst.

**50.** Zelle nach Anspruch 49, wobei die Zelle ausgewählt ist aus der Gruppe bestehend aus einer Bakterienzelle, einer Hefezelle, einer Pilzzelle, einer Pflanzenzelle, einer Insektenzelle und einer Tierzelle.

**51.** Isoliertes gereinigtes Luciferase- und GFP-Fusionsprotein, wobei das GFP ein GFP aus *Renilla reniformis* ist und das Fusionsprotein von dem Nukleinsäurekonstrukt nach Anspruch 47 kodiert ist.

**52.** Fusionsprotein nach Anspruch 51, wobei die Luciferase eine Luciferase aus *Renilla* ist.

**53.** Fusionsprotein nach Anspruch 51, wobei die Luciferase eine Luciferase aus *Renilla reniformis* ist.

**54.** Zusammensetzung, die das Fusionsprotein nach Anspruch 48 umfasst.

**55.** Zusammensetzung nach Anspruch 54, weiterhin umfassend mindestens einen Bestandteil eines Biolumineszenz-erzeugenden Systems.

**56.** Zusammensetzung nach Anspruch 55, wobei der Bestandteil des Biolumineszenz-erzeugenden Systems ein Luciferin ist.

**57.** Nukleinsäurekonstrukt nach Anspruch 47, wobei die Nukleotidsequenz, die für die Luciferase und GFP kodiert, nicht aneinander grenzt.

**58.** Nukleinsäurekonstrukt nach Anspruch 57, umfassend eine Nukleotidsequenz, die für eine Ligandenbindungsdomäne eines Zielproteins kodiert.

**59.** Biosensor, umfassend ein GFP-Protein, das von dem Nukleinsäuremolekül nach Anspruch 1 kodiert ist, und eine Luciferase.

**60.** Biosensor nach Anspruch 59, wobei die Luciferase eine Luciferase aus *Renilla* ist.

**61.** Biosensor nach Anspruch 59, weiterhin umfassend einen Modulator.

**62.** Biosensor, umfassend ein Fusionsprotein nach Anspruch 51.

**63.** Biosensor nach Anspruch 62, wobei das GFP und Luciferase in dem Fusionsprotein nicht aneinander grenzen.

**64.** Biolumineszenz-Resonanz-Energietransfer (BRET)-System, umfassend:

(a) ein GFP, das von dem Nukleinsäuremolekül nach Anspruch 1 kodiert ist;
(b) eine Luciferase, aus welcher das GFP Energie aufnehmen kann, wenn sich GFP und Luciferase miteinander verbinden; und
(c) ein Luciferin oder ein anderes Substrat der Luciferase.

**65.** Das BRET-System nach Anspruch 64, weiterhin umfassend einen oder mehrere Modulatoren.

**66.** Das BRET-System nach Anspruch 65, wobei das GFP und Luciferase jeweils an einem unterschiedlichen Modulator angebracht sind, oder jeweils an demselben Modulator angebracht sind.

**67.** BRET-System nach Anspruch 65, wobei eine Konformationsänderung in einem Modulator zu einer Zunahme der Nähe der Luciferase zu dem GFP führt.

**68.** BRET-System nach Anspruch 65, wobei eine Konformationsänderung in einem Modulator zu einer Abnahme der Nähe der Luciferase zu dem GFP führt.

**69.** BRET-System nach Anspruch 65, wobei die Luciferase eine Luciferase aus *Renilla reniformis* ist.

**70.** Mikroelektronische Vorrichtung, umfassend:

einen Träger;
eine Vielzahl von Mikrostellen, die auf einem Träger festgelegt sind, wobei jede Mikrostelle zur Verknüpfung eines Makromoleküls vorgesehen ist;
ein unabhängiger Photodetektor, der an oder angrenzend an jede Mikrostelle integriert ist und gegebenenfalls optisch an jede Mikrostelle gekoppelt ist,
wobei jeder Photodetektor so gestaltet ist, dass er ein Lesesignal in Reaktion auf die Lichtphotonen erzeugt, die an der entsprechenden Mikrostelle ausgestrahlt werden, wenn eine lichtausstrahlende chemische Reaktion an der Mikrostelle auftritt, wobei jeder Photodetektor unabhängig von den Photodetektoren ist, die optisch an die anderen Mikrostellen gekoppelt sind; und
ein elektrischer Kreislauf, der an jeden Photodetektor gekoppelt ist und so gestaltet ist, dass er das Lesesignal liest, welches von jedem Photodetektor erzeugt wurde, und dass er Ausgangssignale daraus erzeugt, die das Licht anzeigen, welches an jeder Mikrostelle durch die lichtausstrahlenden chemischen Reaktionen ausgestrahlt wird, wobei die Vorrichtung Lichtphotonen nachweist, die von jeder lichtausstrahlenden chemischen Reaktion ausgestrahlt werden, wobei:
jede Mikrostelle durch einen Abschnitt der Oberfläche festgelegt ist; und
die Mikrostellen auf dem Träger jeweils einen Bestandteil eines Biolumineszenz-erzeugenden Systems und ein grünes Fluoreszenzprotein nach Anspruch 1 umfassen, wobei Lichtphotonen ausgestrahlt werden, wenn eine Reaktion an der Mikrostelle auftritt.

**71.** Vorrichtung nach Anspruch 70, wobei die Mikrostellen in einem Array bereitgestellt sind.

**72.** Vorrichtung nach Anspruch 70, wobei das Biolumineszenz-erzeugende System eine Luciferase aus *Renilla* umfasst.

**73.** Vorrichtung nach Anspruch 71, wobei das Biolumineszenz-erzeugende System eine Luciferase aus *Renilla reniformis* umfasst.

**74.** Verfahren zum Nachweisen und Identifizieren von Analyten in einer biologischen Probe, umfassend:

Bereitstellen der mikroelektronischen Vorrichtung nach Anspruch 70;
Anbringen eines Makromoleküls oder einer Vielzahl von unterschiedlichen Makromolekülen an der Oberfläche jeder Mikrostelle auf der Vorrichtung,
wobei das Makromolekül für die Bindung an den ausgewählten Analyten, der in der biologischen Probe vorliegen kann, spezifisch ist;
Inkontaktbringen der Probe mit der Oberfläche der mikroelektronischen Vorrichtung, wobei einer der ausgewählten Analyten, die in der Probe vorliegen, an das Makromolekül bindet, welches an der Oberfläche jeder Mikrostelle angebracht ist;
Exponieren der Oberfläche der mikroelektronischen Vorrichtung gegenüber einem zweiten Makromolekül oder einer Vielzahl davon, die an den ausgewählten Analyten gebunden sind, der bereits an das erste Makromolekül an jeder Mikrostelle gebunden ist, wobei das zweite Makromolekül einen Bestandteil einer Biolumineszenz-erzeugenden Reaktion umfasst;
Auslösen der Biolumineszenz-erzeugenden Reaktion durch Inkontaktbringen der Oberfläche der Vorrichtung mit den verbleibenden Bestandteilen der Biolumineszenz-erzeugenden Reaktion, wobei die Wellenlänge des sich ergebenden Lichts durch das GFP aus *Renilla reniformis* verschoben wird; und
Nachweisen von Lichtphotonen, die von dem GFP ausgestrahlt werden, unter Verwendung eines Photodetektors, der optisch an jede Mikrostelle gekoppelt ist, wobei jeder Photodetektor ein Lesesignal erzeugt, das für

die Biolumineszenzerzeugung an der entsprechenden Mikrostelle charakteristisch ist.

**75.** Transgenes Tier oder transgene Pflanze, die die Nukleinsäure aus *Renilla reniformis* nach Anspruch 1 exprimiert.

**76.** Transgenes Tier oder transgene Pflanze nach Anspruch 75, ausgewählt aus Fisch, Würmern, Affen, Nagern, Ziegen, Schweinen, Kühen, Schafen, Pferden, Blütenpflanzen, Zierpflanzen.

**77.** Transgenes Tier oder transgene Pflanze nach Anspruch 75, die eine Orchidee ist.

## Revendications

**1.** Molécule d'acide nucléique isolée codant pour une protéine fluorescente verte de *Renilla reniformis*, comprenant une séquence de nucléotides qui code pour la protéine de la SEQ ID N° 27 ou une protéine fluorescente verte codée par une *Renilla reniformis* présentant une identité de séquence d'au moins 80 % avec celle-ci.

**2.** Molécule d'acide nucléique isolée suivant la revendication 1, qui code pour une protéine ayant une identité de séquence d'au moins 90 % avec la protéine de la SEQ ID N° 27.

**3.** Molécule d'acide nucléique isolée suivant la revendication 1, comprenant une séquence de nucléotides choisie dans le groupe consistant en :

(a) la partie codante de la séquence de nucléotides indiquée dans l'une quelconque des SEQ ID N° 23 à 25 ;
(b) une séquence de nucléotides qui s'hybride dans des conditions hautement drastiques à la séquence de nucléotides de (a) ; et
(c) une séquence de nucléotides comprenant des codons dégénérés de (a) ou (b).

**4.** Molécule d'acide nucléique isolée suivant la revendication 1, dans laquelle l'acide nucléique est un ADN.

**5.** Molécule d'acide nucléique isolée suivant la revendication 1, dans laquelle l'acide nucléique est un ARN.

**6.** Molécule d'acide nucléique isolée suivant la revendication 1 ou la revendication 2, comprenant la séquence de nucléotides indiquée dans la SEQ ID N° 26 qui comprend des codons optimisés pour l'expression chez l'homme.

**7.** Sonde ou amorce d'acide nucléique, comprenant au moins 14 nucléotides contigus choisis dans la séquence de nucléotides indiquée dans la revendication 1.

**8.** Sonde ou amorce suivant la revendication 7, comprenant au moins 16 nucléotides contigus choisis dans la séquence de nucléotides de la revendication 1.

**9.** Sonde ou amorce suivant la revendication 8, comprenant au moins 30 nucléotides contigus.

**10.** Plasmide comprenant la séquence de nucléotides de la revendication 1 ou de la revendication 6.

**11.** Plasmide suivant la revendication 10, qui est un vecteur d'expression, comprenant :

un élément servant de promoteur ;
un site de clonage pour l'introduction d'un acide nucléique ; et
un marqueur sélectionnable ;

dans lequel l'acide nucléique codant pour le site de clonage est positionné entre les acides nucléiques codant pour l'élément servant de promoteur et la protéine fluorescente verte, et dans lequel l'acide nucléique codant pour la protéine fluorescente verte est lié de manière fonctionnelle à l'élément servant de promoteur.

**12.** Plasmide suivant la revendication 11, comprenant en outre une séquence de nucléotides codant pour une luciférase.

**13.** Cellule hôte recombinante comprenant le plasmide de la revendication 10.

**14.** Cellule suivant la revendication 13, la cellule étant choisie dans le groupe consistant en une cellule bactérienne, une cellule de levure, une cellule fongique, une cellule végétale, une cellule d'insecte et une cellule animale.

**15.** Protéine fluorescente verte (GFP) de *Renilla reniformis* purifiée isolée codée par la molécule d'acide nucléique de la revendication 1.

**16.** Mutéine de la GFP de la revendication 15 qui présente des propriétés spectrales modifiées.

**17.** Composition comprenant la protéine fluorescente verte de la revendication 15 et au moins un constituant d'un système engendrant une bioluminescence.

**18.** Composition suivant la revendication 17, dans laquelle le système engendrant une bioluminescence est choisi parmi ceux isolés : d'un système d'insecte, d'un système de coelentérés, d'un système de cténophore, d'un système bactérien, d'un système de mollusque, d'un système de crustacé, d'un système de poisson, d'un système d'annélide et d'un système de lombric.

**19.** Composition suivant la revendication 17, dans laquelle le système engendrant une bioluminescence est choisi parmi ceux isolés : de lucioles, de *Mnemiopsis*, de *Beroe ovata,* d'*Aequorea*, d'*Obelia*, de *Vargula*, de *Pelagia*, de *Renilla*, de *Pholas aristotomias,* de *Pachystomias*, de *Porichthys*, de *Cypridina*, d'*Aristostomias*, de *Pachystomias,* de *Malacosteus*, de *Gonadostomias*, de *Gaussia,* de *Watensia,* d'*Halisturia*, de calmars vampires, de *Glyphus,* de Mucotophides, de *Vinciguerria, d'Howella,* de *Florenciella,* de *Chaudiodus*, de *Malanocostus*, de plumes marines, de *Chiroteuthis,* d'*Eucleoteuthis*, d'*Onychoteuthis*, de *Watasenia*, de seiche, de *Sepiolina,* d'*Oplophorus*, d'*Acanthophyra*, de *Sergestes*, de *Gnathophausia*, d'*Argyropelecus*, d'*Yarella*, de *Diaphus*, de *Gonadostomias* et de *Neoscopelus*.

**20.** Mutéine suivant la revendication 15, comprenant une substitution dans des aminoacides aux aminoacides 56 à 75 de la SEQ ID N° 27, ce qui provoque une modification des propriétés spectrales.

**21.** Composition suivant la revendication 20, dans laquelle le système engendrant une bioluminescence est choisi parmi ceux isolés d'*Aequorea*, d'*Obelia*, de *Vargula* et de *Renilla*.

**22.** Produit d'assemblage de gène rapporteur, comprenant l'acide nucléique de la revendication 1.

**23.** Association comprenant :

un article manufacturé ; et
une protéine fluorescente verte (GFP) de *Renilla reniformis* codée par une molécule d'acide nucléique de la revendication 1.

**24.** Association suivant la revendication 23, comprenant en outre
au moins un constituant d'un système engendrant une bioluminescence,
l'association étant ainsi un article nouveau.

**25.** Association suivant la revendication 24, dans laquelle le constituant du système engendrant une bioluminescence comprend une luciférase.

**26.** Association suivant la revendication 24, dans laquelle le constituant du système engendrant une bioluminescence comprend une luciférine.

**27.** Association suivant la revendication 23, dans laquelle l'article manufacturé est choisi entre des jouets, des stylos, des articles pour soins individuels, de la poudre scintillante, des denrées alimentaires, des produits textiles et des produits en papier.

**28.** Association suivant la revendication 27, dans laquelle l'article manufacturé est choisi entre des pistolets factices, des pistolets à bouchons, des cartes de voeux, des peintures à appliquer avec les doigts, des chaussons, une pâte gluante récréative, des vêtements, des jouets faisant des bulles et produits faisant des bulles pour ceux-ci, des ballons, des poudres pour le bain, des lotions corporelles, des gels, des poudres corporelles, des crèmes corporelles, des pâtes dentifrices, des bains de bouche, des savons, des peintures corporelles, des produits pour bain moussant,

des jeux de bureau, des leurres pour la pêche, des jouets ovoïdes, des cigarettes factices, des poupées, des allumettes japonaises, des jouets en forme de baguette magique, le papier d'emballage, des gélatines, des glaçages, des garnitures givrées, la poudre scintillante, la bière, des plantes transgéniques ornementales, le vin, le champagne, le lait, des boissons sucrées, des cubes de glace, la glace, la neige carbonique, des savons, des peintures corporelles et des produits pour bain moussant.

**29.** Association suivant la revendication 28, qui est une plante ornementale transgénique.

**30.** Association suivant la revendication 28, qui est un jouet.

**31.** Association suivant la revendication 28, qui est une denrée alimentaire.

**32.** Association suivant la revendication 28, qui est un produit cosmétique.

**33.** Association suivant la revendication 28, qui est une boisson.

**34.** Association suivant la revendication 24, dans laquelle l'article manufacturé est choisi entre des jouets, des stylos, des articles pour soins individuels, la poudre scintillante, des aliments, des matières textiles, des plantes ornementales transgéniques et des produits en papier.

**35.** Association suivant la revendication 34, dans laquelle l'article manufacturé est choisi entre des pistolets factices, des pistolets à bouchons, des cartes de voeux, des peintures à appliquer avec les doigts, des chaussons, une pâte gluante récréative, des vêtements, des jouets faisant des bulles et produits faisant des bulles pour ceux-ci, des ballons, des poudres pour le bain, des lotions corporelles, des gels, des poudres corporelles, des crèmes corporelles, des pâtes dentifrices, des bains de bouche, des savons, des peintures corporelles, des produits pour bain moussant, des jeux de bureau, des leurres pour la pêche, des jouets ovoïdes, des cigarettes factices, des poupées, des allumettes japonaises, des jouets en forme de baguette magique, le papier d'emballage, des gélatines, des glaçages, des garnitures givrées, la poudre scintillante, la bière, le vin, le champagne, des boissons sucrées, des cubes de glace, la glace, la neige carbonique, des savons, des peintures corporelles et des produits pour bain moussant.

**36.** Anticorps qui se lie spécifiquement à *Renilla reniformis* ou une molécule ou un dérivé de l'anticorps contenant son domaine de liaison.

**37.** Anticorps suivant la revendication 36, qui est un anticorps monoclonal.

**38.** Produit d'assemblage d'acides nucléiques, comprenant une séquence de nucléotides codant pour une luciférase et une séquence de nucléotides de la revendication 1 qui code pour une protéine fluorescente (GFP) de *Renilla reniformis*.

**39.** Produit d'assemblage d'acides nucléiques suivant la revendication 38, dans lequel la luciférase est une luciférase de *Renilla mulleri,* une luciférase de *Gaussia* ou une luciférase de *Pleuromamma*.

**40.** Produit d'assemblage d'acides nucléiques suivant la revendication 39, dans lequel la luciférase de *Gaussia* est une luciférase de *Gaussia princeps.*

**41.** Produit d'assemblage d'acides nucléiques suivant la revendication 38, dans lequel la luciférase est codée par :

une séquence de nucléotides indiquée dans la SEQ ID N° 17, la SEQ ID N° 19 ou la SEQ ID N° 28 ;
une séquence de nucléotides codant pour la séquence d'aminoacides indiquée dans la SEQ ID N° 18, la SEQ ID N° 20 ou la SEQ ID N° 29 ; et
une séquence de nucléotides qui s'hybride dans des conditions hautement drastiques à la séquence de nucléotides indiquée dans la SEQ ID N° 17, la SEQ ID N° 19 ou la SEQ ID N° 28.

**42.** Produit d'assemblage d'acides nucléiques suivant la revendication 38, qui est un ADN.

**43.** Produit d'assemblage d'acides nucléiques suivant la revendication 38, qui est un ARN.

**44.** Plasmide comprenant le produit d'assemblage d'acides nucléiques de la revendication 38.

**45.** Plasmide suivant la revendication 44, comprenant en outre une séquence de nucléotides codant pour :

un élément servant de promoteur ;
un marqueur sélectionnable ;
dans lequel la séquence de nucléotides codant pour la luciférase et la GFP est liée de manière fonctionnelle à l'élément servant de promoteur, ce qui provoque l'expression de la luciférase et de la GFP.

**46.** Produit d'assemblage suivant la revendication 38, dans lequel la luciférase et la GFP sont codées par un message polycistronique.

**47.** Produit d'assemblage suivant la revendication 38, dans lequel la luciférase et la protéine fluorescente codées constituent une protéine de fusion.

**48.** Produit d'assemblage suivant la revendication 38, dans lequel la luciférase est la luciférase de *Renilla reniformis.*

**49.** Cellule hôte recombinante comprenant le plasmide de la revendication 44.

**50.** Cellule suivant la revendication 49, la cellule étant choisie dans le groupe consistant en une cellule bactérienne, une cellule de levure, une cellule fongique, une cellule végétale, une cellule d'insecte et une cellule animale.

**51.** Protéine de fusion de luciférase et de GFP isolée purifiée, dans laquelle la GFP est la GFP de *Renilla reniformis* et la protéine de fusion est codée par le produit d'assemblage d'acides nucléiques de la revendication 47.

**52.** Protéine de fusion suivant la revendication 51, dans laquelle la luciférase est une luciférase de *Renilla*.

**53.** Protéine de fusion suivant la revendication 51, dans laquelle la luciférase est une luciférase de *Renilla reniformis*.

**54.** Composition comprenant la protéine de fusion de la revendication 48.

**55.** Composition suivant la revendication 54, comprenant en outre au moins un constituant d'un système engendrant une bioluminescence.

**56.** Composition suivant la revendication 55, dans laquelle le constituant du système engendrant une bioluminescence est une luciférine.

**57.** Produit d'assemblage d'acides nucléiques suivant la revendication 47, dans lequel la séquence de nucléotides codant pour la luciférase et la séquence de nucléotides codant pour la GFP ne sont pas contiguës.

**58.** Produit d'assemblage d'acides nucléiques suivant la revendication 57, comprenant une séquence de nucléotides qui code pour un domaine de liaisons de ligands d'une protéine cible.

**59.** Biocapteur comprenant une protéine GFP codée par la molécule d'acide nucléique de la revendication 1 et une luciférase.

**60.** Biocapteur suivant la revendication 59, dans lequel la luciférase est une luciférase de *Renilla*.

**61.** Biocapteur suivant la revendication 59, comprenant en outre un modulateur.

**62.** Biocapteur comprenant une protéine de fusion de la revendication 51.

**63.** Biocapteur suivant la revendication 62, dans lequel la GFP et la luciférase dans la protéine de fusion ne sont pas contiguës.

**64.** Système de transfert d'énergie de résonance à bioluminescence (BRET), comprenant :

(a) une GFP codée par la molécule d'acide nucléique de la revendication 1 ;
(b) une luciférase dont la GFP peut accepter l'énergie lorsque la GFP et la luciférase s'associent ; et
(c) une luciférine ou un autre substrat de la luciférase.

**65.** Système BRET suivant la revendication 64, comprenant en outre un ou plusieurs modulateurs.

**66.** Système BRET suivant la revendication 65, dans lequel la GFP et la luciférase sont chacune fixées à un modulateur différent, ou sont chacune fixées au même modulateur.

**67.** Système BRET suivant la revendication 65, dans lequel un changement de conformation dans un modulateur provoque un accroissement à proximité de la luciférase et de la GFP.

**68.** Système BRET suivant la revendication 65, dans lequel un changement de conformation dans un modulateur provoque une diminution à proximité de la luciférase et de la GFP.

**69.** Système BRET suivant la revendication 65, dans lequel la luciférase est la luciférase de *Renilla reniformis*.

**70.** Dispositif microélectronique, comprenant :

un substrat ;
une pluralité de microemplacements définis sur le substrat, où chaque microemplacement est destiné à la liaison d'une macromolécule ;
un photodétecteur indépendant intégré ou adjacent à chaque microemplacement et couplé optiquement à chaque microemplacement, chaque photodétecteur étant configuré pour engendrer un signal capté sensible aux photons de lumière émis au microemplacement correspondant lorsqu'une réaction chimique photoémettrice se produit à ce microemplacement, chaque photodétecteur étant indépendant des photodétecteurs couplés optiquement aux autres microemplacements ; et
un circuit électronique couplé à chaque photodétecteur et configuré pour la lecture du signal capté engendré par chaque photodétecteur et pour engendrer des signaux de données de sortie à partir de celui-ci qui sont indicatifs de la lumière émise à chaque microemplacement par les réactions chimiques photoémettrices, ce qui fait que le dispositif détecte des photons de lumière émis par les réactions chimiques photoémettrices, dans lequel :

chaque microemplacement est défini par une partie de la surface ; et
les microemplacements définis sur le substrat comprennent chacun un constituant d'un système engendrant une bioluminescence et une protéine fluorescente verte de la revendication 1, ce qui fait que les photons de lumière sont émis lorsqu'une réaction s'effectue à ce microemplacement.

**71.** Dispositif suivant 1a revendication 70, dans lequel les microemplacements sont fournis sous forme d'un réseau.

**72.** Dispositif suivant la revendication 70, dans lequel le système engendrant une bioluminescence comprend une luciférase de *Renilla.*

**73.** Dispositif suivant la revendication 71, dans lequel le système engendrant une bioluminescence comprend une luciférase de *Renilla reniformis*.

**74.** Méthode pour détecter et identifier des analytes dans un échantillon biologique, comprenant les étapes consistant :

à fournir le dispositif microélectronique de la revendication 70 ;
à fixer une macromolécule ou une pluralité de macromolécules différentes à la surface à chaque microemplacement sur le dispositif, la macromolécule étant spécifique pour la liaison à un analyte choisi qui peut être présent dans l'échantillon biologique ;
à mettre en contact l'échantillon avec la surface du dispositif microélectronique, ce qui fait que n'importe lequel des analytes choisis qui sont présents dans l'échantillon se lie à la macromolécule fixée à la surface à chaque microemplacement ;
à exposer la surface du dispositif microélectronique à une seconde macromolécule ou à une pluralité de celles-ci liée à l'analyte choisi déjà lié à la première macromolécule à chaque microemplacement, la seconde macromolécule comprenant un constituant d'une réaction engendrant une bioluminescence ;
à déclencher la réaction engendrant une bioluminescence en mettant en contact la surface du dispositif avec les constituants restants de la réaction engendrant une bioluminescence, la longueur d'onde de la lumière résultante étant déplacée par la GFP de *Renilla reniformis ;* et
à détecter les photons de lumière émis par la GFP en utilisant un photodétecteur couplé optiquement à chaque

microemplacement, chaque photodétecteur engendrant un signal capté représentatif de la production de bioluminescence au microemplacement respectif.

**75.** Animal transgénique ou plante transgénique qui exprime l'acide nucléique de *Renilla reniformis* de la revendication 1.

**76.** Animal transgénique ou plante transgénique suivant la revendication 75, choisi entre des poissons, des vers, des singes, des rongeurs, des chèvres, des porcs, des vaches, des moutons, des chevaux, des plantes à fleurs et des plantes ornementales.

**77.** Animal transgénique ou plante transgénique suivant la revendication 75 qui est une orchidée.

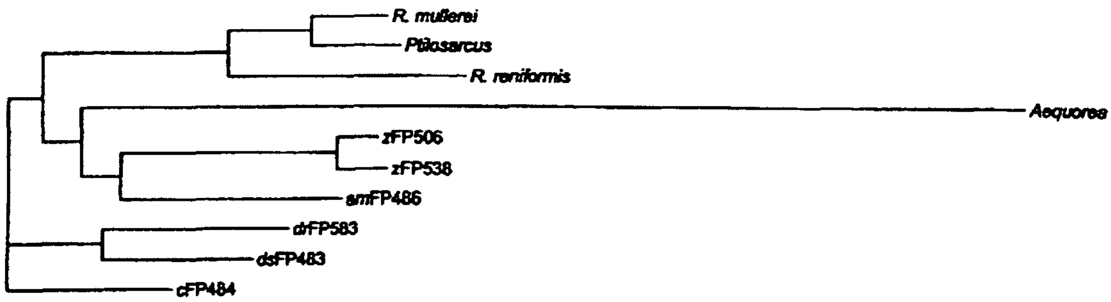
R. mulleri
Ptilosarcus
R. reniformis
Aequorea
zFP506
zFP538
amFP486
drFP583
dsFP483
cFP484

FIGURE 1

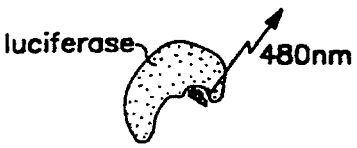


FIGURE 2A

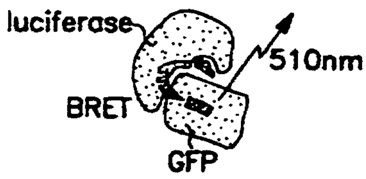


FIGURE 2C

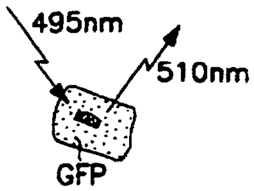


FIGURE 2B

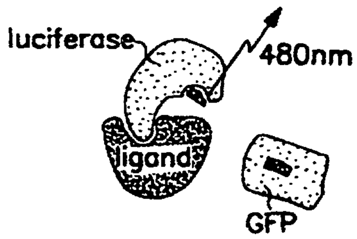


FIGURE 2D

FIGURE 2

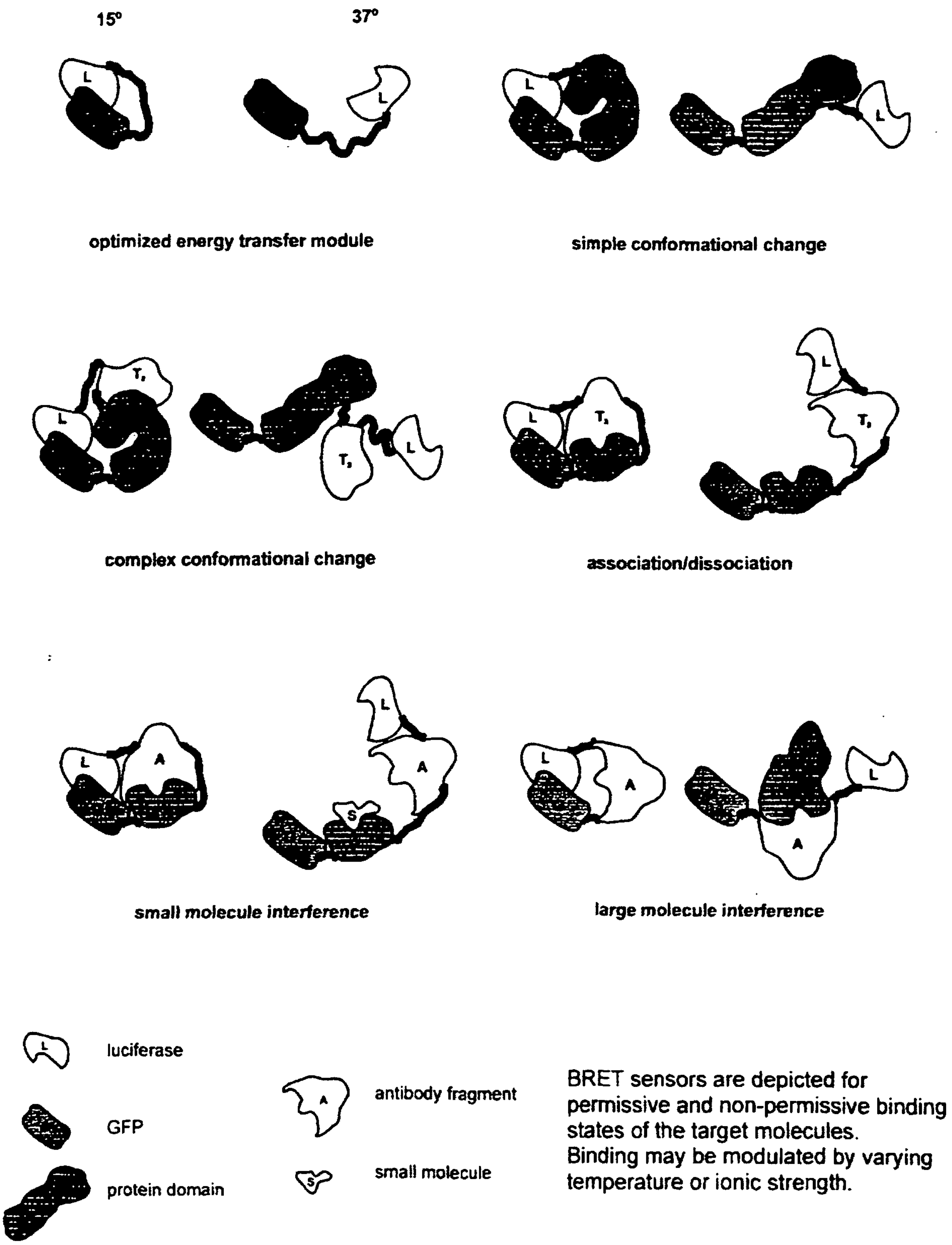
15°
37°
optimized energy transfer module
simple conformational change
complex conformational change
association/dissociation
small molecule interference
large molecule interference
luciferase
GFP
protein domain
antibody fragment
small molecule
BRET sensors are depicted for permissive and non-permissive binding states of the target molecules.
Binding may be modulated by varying temperature or ionic strength.

FIGURE 3

# Utilization of advantageous GFP surfaces with substituted fluorophores

```
                    60          *          80
RM-GFP   :  GAPLPFAFDIVSPAFQYGNRTFTKYPNDIS--  :  83
Pt-GFP   :  GGPLPFAFDIVSIAFQYGNRTFTKYPDDIA--  :  83
RR-GFP   :  GAPLPFAFDIVSVAFSYGNRAYTGYPEEIS--  :  80
cFP484   :  GAPLPFSYDILSNAFQYGNRALTKYPDDIA--  :  83
drFP583  :  GGPLPFAWDILSPQFQYGSKVYVKHPADIP--  :  80
asFP595  :  GGPLPFAFHILSTSCMYGSKTFIKYVSGIP--  :  77
dsFP483  :  GGPLPFGWHILCPQFQYGNKAFVHHPDNIH--  :  80
amFP486  :  GGPLAFSFDILSTVFKYGNRCFTAYPTSMP--  :  82
zFP506   :  GGPLPFAEDILSAAFNYGNRVFTEYPQDIV--  :  80
zFP538   :  GGPLPFSEDILSAGFKYGDRIFTEYPQDIV--  :  80
                    =================
```

FIGURE 4

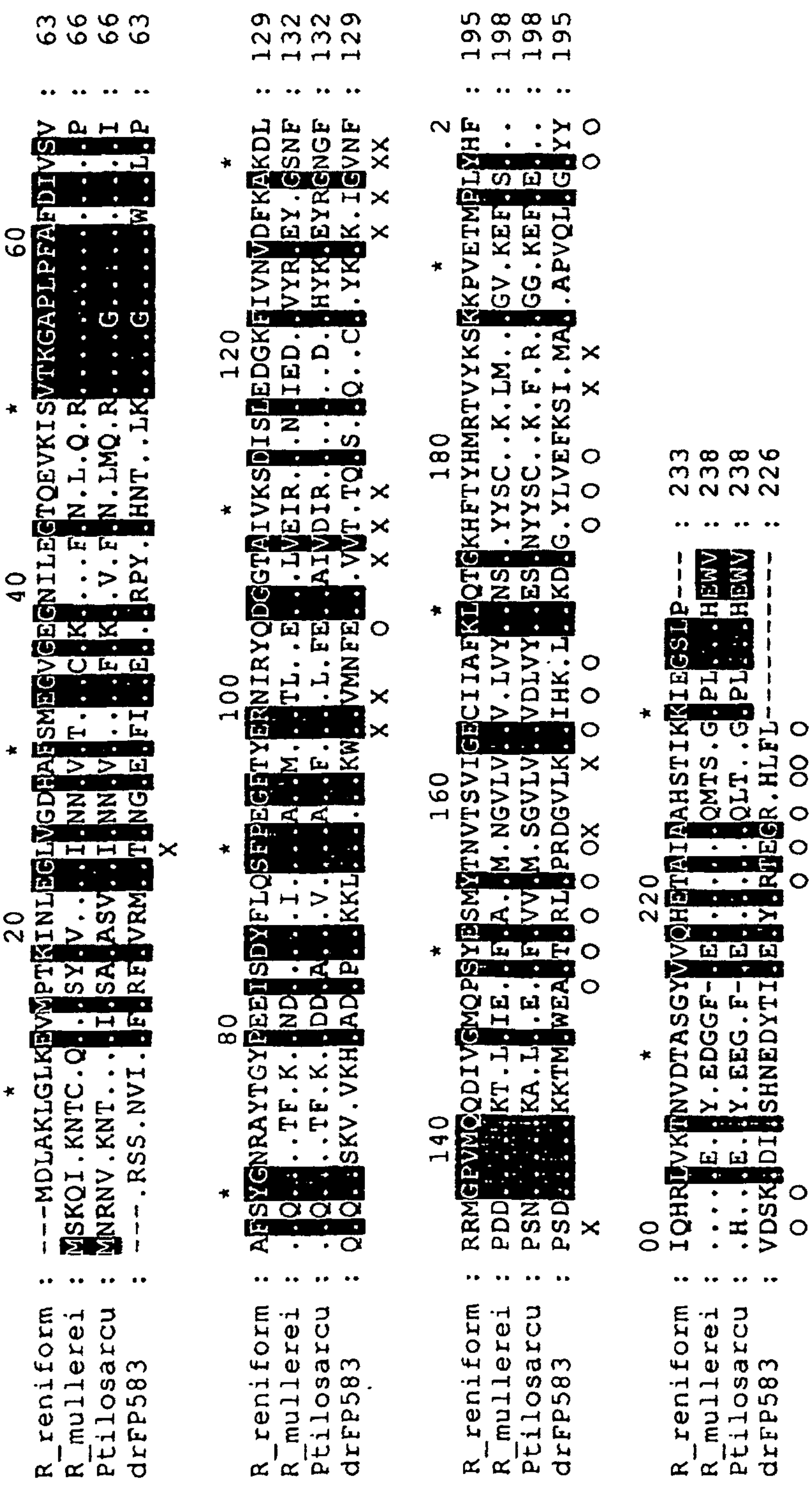


FIGURE 5

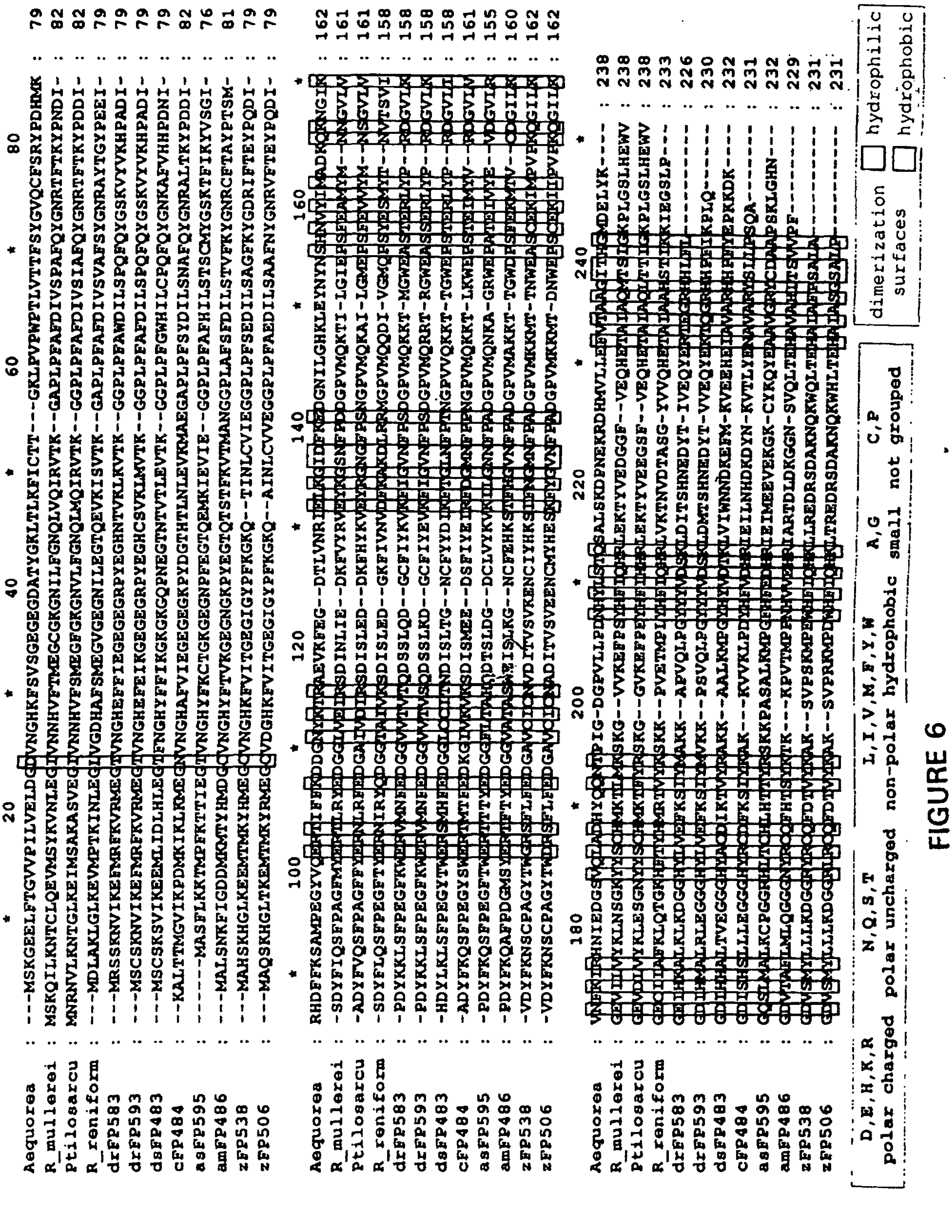
Aequorea
R_mullerei
Ptilosarcu
R_reniform
drFP583
drFP593
dsFP483
cFP484
asFP595
amFP486
zFP538
zFP506
D,E,H,K,R polar charged
N,Q,S,T polar uncharged
L,I,V,M,F,Y,W non-polar hydrophobic
A,G small
C,P not grouped
dimerization surfaces
hydrophilic
hydrophobic

FIGURE 6